# EUROPEAN PATENT APPLICATION

(11) **EP 4 056 703 A1**
(43) Date of publication of application: **14.09.2022**
(21) Application number: 22153020.7
(22) Date of filing: 03.03.2016
(51) Int. Cl.: C12N 15/11, A61K 31/712

(54) **METHODS FOR MODULATING MECP2 EXPRESSION**

(30) Priority: 03.03.2015 US 201562127693 P
(62) Divisional of application: 16759471.2
(71) Applicant: Ionis Pharmaceuticals, Inc., Carlsbad, CA 92010 (US); Baylor College of Medicine, Houston, TX 77030-3411 (US)
(72) Inventor: ZOGHBI, Huda, Y., Houston, 77005 (US); SZTAINBERG, Ezequiel, Houston, 77096 (US); FREIER, Susan, M., Carlsbad, 92010 (US)
(74) Representative: Carpmaels & Ransford LLP

(57) **Abstract**

Disclosed herein are methods for decreasing MECP2 mRNA and protein expression. Such methods are useful to treat, prevent, or ameliorate MECP2 associated disorders and syndromes. Such MECP2 associated disorders include MECP2 duplication syndrome.

SEQUENCE LISTING

<110>  Ionis Pharmaceuticals, Inc.
       Baylor College of Medicine

<120>  METHODS FOR MODULATING MECP2 EXPRESSION

<130>  BIOL0263WO

<150>  62/127,693
<151>  2015-03-03

<160>  16

<170>  PatentIn version 3.5

<210>  1
<211>  10241
<212>  DNA
<213>  Homo sapiens

<400>  1
ccggcgtcgg cggcgcgcgc gctccctcct ctcggagaga gggctgtggt aaaagccgtc      60

cggaaaatgg ccgccgccgc cgccgccgcg ccgagcggag gaggaggagg aggcgaggag     120

gagagactgc tccataaaaa tacagactca ccagttcctg ctttgatgtg acatgtgact     180

ccccagaata caccttgctt ctgtagacca gctccaacag gattccatgg tagctgggat     240

gttagggctc agggaagaaa agtcagaaga ccaggacctc cagggcctca aggacaaacc     300

cctcaagttt aaaaaggtga agaaagataa gaaagaagag aaagagggca agcatgagcc     360

cgtgcagcca tcagcccacc actctgctga gcccgcagag gcaggcaaag cagagacatc     420

agaagggtca ggctccgccc cggctgtgcc ggaagcttct gcctccccca aacagcggcg     480

ctccatcatc cgtgaccggg gacccatgta tgatgacccc accctgcctg aaggctggac     540

acggaagctt aagcaaagga aatctggccg ctctgctggg aagtatgatg tgtatttgat     600

caatccccag ggaaaagcct ttcgctctaa agtggagttg attgcgtact tcgaaaaggt     660

aggcgacaca tccctggacc ctaatgattt tgacttcacg gtaactggga gagggagccc     720

ctcccggcga gagcagaaac cacctaagaa gcccaaatct cccaaagctc caggaactgg     780

cagaggccgg ggacgcccca aagggagcgg caccacgaga cccaaggcgg ccacgtcaga     840

gggtgtgcag gtgaaaaggg tcctggagaa aagtcctggg aagctccttg tcaagatgcc     900

ttttcaaact tcgccagggg gcaaggctga gggggtgggg gccaccacat ccacccaggt     960

catggtgatc aaacgccccg gcaggaagcg aaaagctgag gccgaccctc aggccattcc    1020

caagaaacgg ggccgaaagc cggggagtgt ggtggcagcc gctgccgccg aggccaaaaa    1080

gaaagccgtg aaggagtctt ctatccgatc tgtgcaggag accgtactcc ccatcaagaa    1140

gcgcaagacc cgggagacgg tcagcatcga ggtcaaggaa gtggtgaagc ccctgctggt    1200

gtccaccctc ggtgagaaga gcgggaaagg actgaagacc tgtaagagcc ctgggcggaa    1260

aagcaaggag agcagcccca aggggcgcag cagcagcgcc tcctcacccc ccaagaagga 1320

gcaccaccac catcaccacc actcagagtc cccaaaggcc cccgtgccac tgctcccacc 1380

cctgccccca cctccacctg agcccgagag ctccgaggac cccaccagcc cccctgagcc 1440

ccaggacttg agcagcagcg tctgcaaaga ggagaagatg cccagaggag gctcactgga 1500

gagcgacggc tgccccaagg agccagctaa gactcagccc gcggttgcca ccgccgccac 1560

ggccgcagaa aagtacaaac accgagggga gggagagcgc aaagacattg tttcatcctc 1620

catgccaagg ccaaacagag aggagcctgt ggacagccgg acgcccgtga ccgagagagt 1680

tagctgactt tacacggagc ggattgcaaa gcaaaccaac aagaataaag gcagctgttg 1740

tctcttctcc ttatgggtag ggctctgaca aagcttcccg attaactgaa ataaaaaata 1800

tttttttttc tttcagtaaa cttagagttt cgtggcttca gggtgggagt agttggagca 1860

ttggggatgt ttttcttacc gacaagcaca gtcaggttga agacctaacc agggccagaa 1920

gtagctttgc acttttctaa actaggctcc ttcaacaagg cttgctgcag atactactga 1980

ccagacaagc tgttgaccag gcacctcccc tcccgcccaa acctttcccc catgtggtcg 2040

ttagagacag agcgacagag cagttgagag gacactcccg ttttcggtgc catcagtgcc 2100

ccgtctacag ctcccccagc tccccccacc tcccccactc ccaaccacgt tgggacaggg 2160

aggtgtgagg caggagagac agttggattc tttagagaag atggatatga ccagtggcta 2220

tggcctgtgc gatcccaccc gtggtggctc aagtctggcc ccacaccagc cccaatccaa 2280

aactggcaag gacgcttcac aggacaggaa agtggcacct gtctgctcca gctctggcat 2340

ggctaggagg ggggagtccc ttgaactact gggtgtagac tggcctgaac cacaggagag 2400

gatggcccag ggtgaggtgg catggtccat tctcaaggga cgtcctccaa cgggtggcgc 2460

tagaggccat ggaggcagta ggacaaggtg caggcaggct ggcctggggt caggccgggc 2520

agagcacagc ggggtgagag ggattcctaa tcactcagag cagtctgtga cttagtggac 2580

aggggagggg gcaaaggggg aggagaagaa aatgttcttc cagttacttt ccaattctcc 2640

tttagggaca gcttagaatt atttgcacta ttgagtcttc atgttcccac ttcaaaacaa 2700

acagatgctc tgagagcaaa ctggcttgaa ttggtgacat ttagtccctc aagccaccag 2760

atgtgacagt gttgagaact acctggattt gtatatatac ctgcgcttgt tttaaagtgg 2820

gctcagcaca tagggttccc acgaagctcc gaaactctaa gtgtttgctg caattttata 2880

aggacttcct gattggtttc tcttctcccc ttccatttct gccttttgtt catttcatcc 2940

tttcacttct ttcccttcct ccgtcctcct ccttcctagt tcatcccttc tcttccaggc 3000

agccgcggtg cccaaccaca cttgtcggct ccagtcccca gaactctgcc tgccctttgt 3060

cctcctgctg ccagtaccag ccccaccctg ttttgagccc tgaggaggcc ttgggctctg 3120

ctgagtccga cctggcctgt ctgtgaagag caagagagca gcaaggtctt gctctcctag 3180

gtagccccct cttccctggt aagaaaaagc aaaaggcatt tcccaccctg aacaacgagc 3240

cttttcaccc ttctactcta gagaagtgga ctggaggagc tgggcccgat ttggtagttg 3300

aggaaagcac agaggcctcc tgtggcctgc cagtcatcga gtggcccaac aggggctcca 3360

tgccagccga ccttgacctc actcagaagt ccagagtcta gcgtagtgca gcagggcagt 3420

agcggtacca atgcagaact cccaagaccc gagctgggac cagtacctgg gtccccagcc 3480

cttcctctgc tccccctttt ccctcggagt tcttcttgaa tggcaatgtt ttgcttttgc 3540

tcgatgcaga cagggggcca gaacaccaca catttcactg tctgtctggt ccatagctgt 3600

ggtgtagggg cttagaggca tgggcttgct gtgggttttt aattgatcag ttttcatgtg 3660

ggatcccatc tttttaacct ctgttcagga agtccttatc tagctgcata tcttcatcat 3720

attggtatat ccttttctgt gtttacagag atgtctctta tatctaaatc tgtccaactg 3780

agaagtacct tatcaaagta gcaaatgaga cagcagtctt atgcttccag aaacacccac 3840

aggcatgtcc catgtgagct gctgccatga actgtcaagt gtgtgttgtc ttgtgtattt 3900

cagttattgt ccctggcttc cttactatgg tgtaatcatg aaggagtgaa acatcataga 3960

aactgtctag cacttccttg ccagtcttta gtgatcagga accatagttg acagttccaa 4020

tcagtagctt aagaaaaaac cgtgtttgtc tcttctggaa tggttagaag tgagggagtt 4080

tgccccgttc tgtttgtaga gtctcatagt tggactttct agcatatatg tgtccatttc 4140

cttatgctgt aaaagcaagt cctgcaacca aactcccatc agcccaatcc ctgatccctg 4200

atcccttcca cctgctctgc tgatgacccc cccagcttca cttctgactc ttccccagga 4260

agggaagggg ggtcagaaga gagggtgagt cctccagaac tcttcctcca aggacagaag 4320

gctcctgccc ccatagtggc ctcgaactcc tggcactacc aaaggacact tatccacgag 4380

agcgcagcat ccgaccaggt tgtcactgag aagatgttta ttttggtcag ttgggttttt 4440

atgtattata cttagtcaaa tgtaatgtgg cttctggaat cattgtccag agctgcttcc 4500

ccgtcacctg ggcgtcatct ggtcctggta agaggagtgc gtggcccacc aggcccccct 4560

gtcacccatg acagttcatt cagggccgat ggggcagtcg tggttgggaa cacagcattt 4620

caagcgtcac tttatttcat tcgggcccca cctgcagctc cctcaaagag gcagttgccc 4680

agcctctttc ccttccagtt tattccagag ctgccagtgg ggcctgaggc tccttagggt 4740

tttctctcta tttccccctt tcttcctcat tccctcgtct ttcccaaagg catcacgagt 4800

cagtcgcctt tcagcaggca gccttggcgg tttatcgccc tggcaggcag gggccctgca 4860

gctctcatgc tgcccctgcc ttggggtcag gttgacagga ggttggaggg aaagccttaa 4920

gctgcaggat tctcaccagc tgtgtccggc ccagttttgg ggtgtgacct caatttcaat 4980

tttgtctgta cttgaacatt atgaagatgg gggcctcttt cagtgaattt gtgaacagca 5040

gaattgaccg acagctttcc agtacccatg gggctaggtc attaaggcca catccacagt 5100

ctcccccacc cttgttccag ttgttagtta ctacctcctc tcctgacaat actgtatgtc 5160

gtcgagctcc ccccaggtct acccctcccg gccctgcctg ctggtgggct tgtcatagcc 5220

agtgggattg ccggtcttga cagctcagtg agctggagat acttggtcac agccaggcgc 5280

tagcacagct cccttctgtt gatgctgtat tcccatatca aaagacacag gggacaccca 5340

gaaacgccac atcccccaat ccatcagtgc caaactagcc aacggcccca gcttctcagc 5400

tcgctggatg gcggaagctg ctactcgtga gcgccagtgc gggtgcagac aatcttctgt 5460

tgggtggcat cattccaggc ccgaagcatg aacagtgcac ctgggacagg gagcagcccc 5520

aaattgtcac ctgcttctct gcccagcttt tcattgctgt gacagtgatg gcgaaagagg 5580

gtaataacca gacacaaact gccaagttgg gtggagaaag gagtttcttt agctgacaga 5640

atctctgaat tttaaatcac ttagtaagcg gctcaagccc aggagggagc agagggatac 5700

gagcggagtc ccctgcgcgg gaccatctgg aattggttta gcccaagtgg agcctgacag 5760

ccagaactct gtgtcccccg tctaaccaca gctccttttc cagagcattc cagtcaggct 5820

ctctgggctg actgggccag gggaggttac aggtaccagt tctttaagaa gatctttggg 5880

catatacatt tttagcctgt gtcattgccc caaatggatt cctgtttcaa gttcacacct 5940

gcagattcta ggacctgtgt cctagacttc agggagtcag ctgtttctag agttcctacc 6000

atggagtggg tctggaggac ctgcccggtg ggggggcaga gccctgctcc ctccgggtct 6060

tcctactctt ctctctgctc tgacgggatt tgttgattct ctccattttg gtgtctttct 6120

cttttagata ttgtatcaat ctttagaaaa ggcatagtct acttgttata aatcgttagg 6180

atactgcctc ccccagggtc taaaattaca tattagaggg gaaaagctga acactgaagt 6240

cagttctcaa caatttagaa ggaaaaccta gaaaacattt ggcagaaaat tacatttcga 6300

tgtttttgaa tgaatacgag caagctttta caacagtgct gatctaaaaa tacttagcac 6360

ttggcctgag atgcctggtg agcattacag gcaaggggaa tctggaggta gccgacctga 6420

ggacatggct tctgaacctg tcttttggga gtggtatgga aggtggagcg ttcaccagtg 6480

acctggaagg cccagcacca ccctccttcc cactcttctc atcttgacag agcctgcccc 6540

agcgctgacg tgtcaggaaa acacccaggg aactaggaag gcacttctgc ctgaggggca 6600

gcctgccttg cccactcctg ctctgctcgc ctcggatcag ctgagccttc tgagctggcc 6660

tctcactgcc tccccaaggc cccctgcctg ccctgtcagg aggcagaagg aagcaggtgt 6720

gagggcagtg caaggaggga gcacaacccc cagctcccgc tccgggctcc gacttgtgca 6780

caggcagagc ccagaccctg gaggaaatcc tacctttgaa ttcaagaaca tttggggaat 6840

ttggaaatct ctttgccccc aaacccccat tctgtcctac ctttaatcag gtcctgctca 6900

gcagtgagag cagatgaggt gaaaaggcca agaggtttgg ctcctgccca ctgatagccc 6960

ctctccccgc agtgtttgtg tgtcaagtgg caaagctgtt cttcctggtg accctgatta 7020

tatccagtaa cacatagact gtgcgcatag gcctgctttg tctcctctat cctgggcttt 7080

tgttttgctt tttagttttg cttttagttt ttctgtccct tttatttaac gcaccgacta 7140

gacacacaaa gcagttgaat ttttatatat atatctgtat attgcacaat tataaactca 7200

ttttgcttgt ggctccacac acacaaaaaa agacctgtta aaattatacc tgttgcttaa 7260

ttacaatatt tctgataacc atagcatagg acaagggaaa ataaaaaaag aaaaaaaaga 7320

aaaaaaaacg acaaatctgt ctgctggtca cttcttctgt ccaagcagat tcgtggtctt 7380

ttcctcgctt ctttcaaggg ctttcctgtg ccaggtgaag gaggctccag gcagcaccca 7440

ggttttgcac tcttgtttct cccgtgcttg tgaaagaggt cccaaggttc tgggtgcagg 7500

agcgctccct tgacctgctg aagtccggaa cgtagtcggc acagcctggt cgccttccac 7560

ctctgggagc tggagtccac tggggtggcc tgactccccc agtccccttc ccgtgacctg 7620

gtcagggtga gcccatgtgg agtcagcctc gcaggcctcc ctgccagtag ggtccgagtg 7680

tgtttcatcc ttcccactct gtcgagcctg ggggctggag cggagacggg aggcctggcc 7740

tgtctcggaa cctgtgagct gcaccaggta gaacgccagg gaccccagaa tcatgtgcgt 7800

cagtccaagg ggtcccctcc aggagtagtg aagactccag aaatgtccct ttcttctccc 7860

ccatcctacg agtaattgca tttgcttttg taattcttaa tgagcaatat ctgctagaga 7920

gtttagctgt aacagttctt tttgatcatc tttttttaat aattagaaac accaaaaaaa 7980

tccagaaact tgttcttcca aagcagagag cattataatc accagggcca aaagcttccc 8040

tccctgctgt cattgcttct tctgaggcct gaatccaaaa gaaaaacagc cataggccct 8100

ttcagtggcc gggctacccg tgagcccttc ggaggaccag ggctggggca gcctctgggc 8160

ccacatccgg ggccagctcc ggcgtgtgtt cagtgttagc agtgggtcat gatgctcttt 8220

cccacccagc ctgggatagg ggcagaggag gcgaggaggc cgttgccgct gatgtttggc 8280

cgtgaacagg tgggtgtctg cgtgcgtcca cgtgcgtgtt ttctgactga catgaaatcg 8340

acgcccgagt tagcctcacc cggtgacctc tagccctgcc cggatggagc ggggcccacc 8400

cggttcagtg tttctgggga gctggacagt ggagtgcaaa aggcttgcag aacttgaagc 8460

ctgctccttc ccttgctacc acggcctcct ttccgtttga tttgtcactg cttcaatcaa 8520

taacagccgc tccagagtca gtagtcaatg aatatatgac caaatatcac caggactgtt 8580

actcaatgtg tgccgagccc ttgcccatgc tgggctcccg tgtatctgga cactgtaacg 8640

tgtgctgtgt ttgctcccct tccccttcct tctttgccct ttacttgtct ttctggggtt 8700

tttctgtttg ggtttggttt ggtttttatt tctccttttg tgttccaaac atgaggttct 8760

ctctactggt cctcttaact gtggtgttga ggcttatatt tgtgtaattt ttggtgggtg 8820

aaaggaattt tgctaagtaa atctcttctg tgtttgaact gaagtctgta ttgtaactat 8880

gtttaaagta attgttccag agacaaatat ttctagacac tttttcttta caaacaaaag 8940

cattcggagg gagggggatg gtgactgaga tgagagggga gagctgaaca gatgacccct 9000

gcccagatca gccagaagcc acccaaagca gtggagccca ggagtcccac tccaagccag 9060

caagccgaat agctgatgtg ttgccacttt ccaagtcact gcaaaaccag gttttgttcc 9120

gcccagtgga ttcttgtttt gcttcccctc cccccgagat tattaccacc atcccgtgct 9180

tttaaggaaa ggcaagattg atgtttcctt gaggggagcc aggaggggat gtgtgtgtgc 9240

agagctgaag agctggggag aatggggctg ggcccaccca agcaggaggc tgggacgctc 9300

tgctgtgggc acaggtcagg ctaatgttgg cagatgcagc tcttcctgga caggccaggt 9360

ggtgggcatt ctctctccaa ggtgtgcccc gtgggcatta ctgtttaaga cacttccgtc 9420

acatcccacc ccatcctcca gggctcaaca ctgtgacatc tctattcccc accctcccct 9480

tcccagggca ataaaatgac catggagggg gcttgcactc tcttggctgt cacccgatcg 9540

ccagcaaaac ttagatgtga gaaaacccct tcccattcca tggcgaaaac atctccttag 9600

aaaagccatt accctcatta ggcatggttt tgggctccca aaacacctga cagcccctcc 9660

ctcctctgag aggcggagag tgctgactgt agtgaccatt gcatgccggg tgcagcatct 9720

ggaagagcta ggcagggtgt ctgccccctc ctgagttgaa gtcatgctcc cctgtgccag 9780

cccagaggcc gagagctatg gacagcattg ccagtaacac aggccaccct gtgcagaagg 9840

gagctggctc cagcctggaa acctgtctga ggttgggaga ggtgcacttg gggcacaggg 9900

agaggccggg acacacttag ctggagatgt ctctaaaagc cctgtatcgt attcaccttc 9960

agtttttgtg ttttgggaca attactttag aaaataagta ggtcgtttta aaaacaaaaa 10020

ttattgattg cttttttgta gtgttcagaa aaaaggttct ttgtgtatag ccaaatgact 10080

gaaagcactg atatatttaa aaacaaaagg caatttatta aggaaatttg taccatttca 10140

gtaaacctgt ctgaatgtac ctgtatacgt ttcaaaaaca cccccccccc actgaatccc 10200

tgtaacctat ttattatata aagagtttgc cttataaatt t 10241

<210> 2
<211> 80001
<212> DNA
<213> Homo sapiens

<400> 2
gctaccatca actctttctc ctagactgtt ccagggcctt gcaactagcc ttgtgctgta 60

gttttgtttc atcacgtcca gttctccact ctacacctgc aacatagatc agacagctcc 120

tggctcaaaa tcctctgagg gcttctcatc ttagaataaa ctctcggttc tggccgggtg 180

cggtggctta cgcctgtagt cccagcactt tgggagtccg aggcgggcgg atcacttgaa 240

ctcaggagtt tgagaccagc ctgggcaaca tggtgaactc ccatctctat caaaaataca 300
aaaacttagc caggcgtggt ggttcgcatc tgtggtccca gctacttagg acgctgagga 360
gggaggatcg cttgagctca gggtggacgt tgcagtgagc caagattgcg ccactgcact 420
gcagcctggg tgacagaatg agaccccatc cccacccccc ccaaaaaaga atgaactccc 480
agttctcata gtggccccag ctgcctttcc aatcacattc cctaccactc tccagcaaca 540
ctgacttcct cgttagtccc caacatgcca ggcatagtct ctcctcatgt cctttgaact 600
tgcctggaat gttctttccc cagatattca tatgagggag taaaatgagg gtgaaaacca 660
gcagatatct aaatagcacc cccttcactt agtttatctt tctcaaagcc cttatcacta 720
tgtgaaatga tatattatac ttatttgtat gctagtatga atcttcccgg caagaatgtt 780
agtttgctgt ctgttcagta ccgtgcatcc agagcctgga agagtgcctg gcacatagca 840
ggtagtcaat aaatgaatgg gggcaagcag ccaaatcaga atcaggtttt cttgctaagc 900
atagaactaa cagaaggatc attgaatgga ttggataatg actggcatca gggtaaggtc 960
cccttaacaa acactcctgt cctgaacacc tggttagcta acagttttct catactctta 1020
ttttcccaaa acacaattgc tggatctcag ctccaaatca actcttctag gaaagtgaaa 1080
aattgctgga tctcagctcc caaatcatat cttccaggca gagctaacat tgccccttat 1140
tcacacctcc accaaaccat ctgatccaac agtgacaggt gtcacgaggc cttggcatgc 1200
actctcttcc cccgccagag ttctgcgaaa gccagggttg cgatttgttg tcagtttatt 1260
ccccgcctct atgagagtgt gagcactggg caggctcgga tgaaataatg cattgagtag 1320
gcctctgaaa ccaaggcccc tcagctgggg caacgtcagg ctccagggtg ggcaactttg 1380
ctgcttctgc cgaagatagt gatattgaga aaatgtgggt gcaatgaaac gcttattgca 1440
gcgcactcgg tgcatctgtg gacagagggt caatcgcccc tcagagcagc gcaaacaggc 1500
gtcccaagcc taggccttca cttgccccag catccgcaag ggtccattaa tccttaacat 1560
tcaaattccg cccactaaac cagtccctcc gcgcccaagc cgcctctttt ccccaaacga 1620
cggccgaaag cagccaatca acagctggag gggtccgccc ccttttccct ggccgaaatg 1680
gacaggaaat ctcgccaatt gacggcatcg ccgctgagac ctcccccctc ccccgtcctc 1740
cccgtcccag cccggccatc acagccaatg acgggcgggc tcgcagcggc gccgagggcg 1800
gggcgcgggc gcgcaggtgc agcagcgcgc gggccggcca agagggcggg gcgcgacgtc 1860
ggccgtgcgg ggtcccggcg tcggcggcgc gcgcgctccc tcctctcgga gagagggctg 1920
tggtaaaagc cgtccggaaa atggccgccg ccgccgccgc cgcgccgagc ggaggaggag 1980
gaggaggcga ggaggagaga ctgtgagtgg gaccgccgtg gccgcggcgg gggacccttg 2040
ccggggggcg ggggtcaggg gcgggacgtg gcgcgggagg ggcccgcggg gtcggacgac 2100

acggctggcg gatggcgtcc ctcctctcta ccctccccct cccgccgccg ccggtggcga 2160

ctctcccctc ggcccgtcac ccgtgctcgc gggtgaccgt cctcggcgcg gcctccctgg 2220

agccgccttc gcctgacgcc cctcttcctc ccgccctcga cgcgcatccc ggcccccggc 2280

cccgcgggcg cccctgtcgc cggggttcgc ctgtcggggc tgcgcgcgct cctgcccttc 2340

tcggggcttt gggccgcggc gccgtcgcgc gcccgcggcc ccggcctctc cctggatcgc 2400

gctctccccc tccctccctc gcgcgccccc tctcccgtta ctcggccccc ccaccggcgc 2460

gcgtgcgcac ttcgcctccc gtcgggagag tgcgccacaa gggctcctga gctctcaccc 2520

ccatctctgg gctttgcctc cccctccttc tcgcccattc catgaatttc tgccccccgc 2580

tacccccccg cgagcgagta ggtccaccgg ctcctttccc catctagcag gaacaagtag 2640

gtggggatta ttatccacaa aagggactag acattgtgtt ctgggtccca caactcatca 2700

taaagaggtg gttatagttc ccatcaggag ccgtgggtag gggactgtgc gtccagcagc 2760

acccgaggct cttcggcgcc agaggctcta aggtgcgagc gtgtccccag ggtgctcaga 2820

ggttctttgg agtgctgtgg cctcggaatg tgagcaccct cccatcctac cctccccttc 2880

gccggcgatc ttccagttac ataagtggag tgggacatag taggtaacgg gctctcatcg 2940

ccctggagcg ctcgggatca ccggtaccag atggggcaag ttcatcgcga cgctgtggct 3000

cctgttaatt gtgcctgaaa ggttatcctc tgttcagttt ggtcatgacc gaatcaccca 3060

aactgaaact cagatgactt ttatatggca gtggcaatgt ttctgtggtt ttgcactaaa 3120

gacttaaggt cattatgaag agtgtaaaag gattgctgtc gcaggggaga ccgtttatga 3180

cattgctaag tatggacccc gagggggaaa aagctcattc tggaattgct ctgtggcggt 3240

tgcttctgtt gcgagtgtgt tcgggtgtgg gttggtcaca gcagaaatgg gctccaccac 3300

aaaattgata ttcctgactc atggtcaggc agatgtgtgc ttctggttat ttttccagga 3360

acaggaatgg gctctgccga gcctttcaca cgttgtgtct ctgctgtgcg ggatcagtac 3420

cccagactgt gagataacag gatcgaattc aggggttggt tggtgatcca ggaaccatac 3480

ctacttggat gtgatactga gtggcctaga cgttgtagga ctctcataag ttttagacat 3540

tttgttatac tgtaaaaatg aagcatatac ggcctcagta gtaacattgc actaataggt 3600

aattattact ctagagaatt ttgggttcca tggctggaat gttcatcagt atctcagcaa 3660

tttcagttaa taaccagatt aagctgacct cttgagtctt ttgaaatctg ctgggtccct 3720

ataataagac agggtctgga ttttttaatc ttttgggaat ttccaccaag gatttaggga 3780

aataggcttt tatgggcttt tcaggatttc tatttcaaat cccaatgagg aagtctagga 3840

aacacttgct tttattttct tgcctgaata tgtcagaacc ttaaatcacc caatcaatct 3900

agaagtacta cagttgatta aagtaagtca ccacaggtca cccattcgtg gcaggtcagc 3960

taggtggctg atcactggaa tacagtcata tccccaggca tacacacgag cctcacttga 4020

cgatcccaaa gtttgtacat ctgagcttga gacgtgacaa gttgaggtct ctgctttgtt 4080

cttcatagtt ggcagtttgt ctcctcgggt gcccctccgg ttagctgttt atcatacgtc 4140

tagccctatc aacagtgcag gcagcttcca gagtatagtt ttctctgcaa agtgcctggt 4200

ggaggggtac ctccttttcc attgtcctaa tggctccact gggcttccat accagccctt 4260

tccagtgtct taacagctca aagaaaccca tctgtggctt tcttgttagc actggcctat 4320

cacaccttcc tattgcctgt taactaaaat tttgcctgga atgctttaag cttagagtac 4380

tgggtctttg tttcacccga ttgataggag agtgtcatgc agtcagccga actccgactt 4440

ctgtgctgtt taaatcctta ggtaggtctg gggcaaggca caaaccactg tgtattcaaa 4500

gggttatggt gcttgcattc tgtgtgtata tgtttgtaat ttccaaccag agtcgtttcc 4560

cgtgcctgcc ctgcctctgt gcaatctagt ctagtagggt agggaggcaa gctgtggagt 4620

ttgaaaaaaa aaaaaaaagc tagatgagtt taataggctg ttaccccctc cagaacattc 4680

ctcccatgtg gagatgaaac gagaagttca tttctttaac ctaaaataaa gaacaacaga 4740

gataaaattg ttagttcctt attggagcct ggtttccatt gctaatgtac ctgcttggga 4800

ctctgcatga taaatgacgt gtcctggaac ctgaaatctt ttataaaggt taatatcagc 4860

agaaatcttt taattagcta agagcagctt gttctcaagc tgcccgagtt atcttggagg 4920

agagaatcac aggaaatgtt tgatatttct agtaaccagt gttttcagtg cgtggggtaa 4980

agcacactcc tagactggcc agtcccaaag agtgtggcag gacacctgct tgttttccct 5040

gccctctgct atggtgacat ccttgcagac cagtcttgcg tggctggcag acgcaatctt 5100

tttttcttgg gtagagcatt atcaccgctg cgatggaagg accttgtcct cggtgcctcc 5160

ataccagccc tttccagtga aactactgct gtcccggcaa gcccctgtgc gtgtgcatca 5220

ttccgggtag gacaagatgt gaacaggtcc ctcttctttg ggcttaagta gaagttgtgc 5280

tcttctgctt ttacctgtgt gctcttctgc ttttacctgt tctcttctgc ctttctcttg 5340

caagaaacct gtcgaaagct tgctttgcat gccattgctt aaatactttg atggtatgta 5400

tggtatgtgg gataactgag tgggagccgg aattgggggg tgggcagggc atccccaccc 5460

ccacccccac atcaaagggg gaatgaccat ttcgttagag aataaagcct gagtcttata 5520

acttcttcaa gcacatgtat gctgggtctc ctggcgtgtg aatgtgttcc cgctgtgctg 5580

tgtggctgtt ttgcagttac tgtagacact gtagtctggg ctctcattat tgcctctgaa 5640

gttgacagga ccaagcctta gtaaggatgc acttgtcttc ctagcccaag agctaggggt 5700

gttttatata tatattaact ttctttttta gatttcataa actgctctca ttttctcttt 5760

tctttctttc tttttttttt tttttttttg agacagtctc actctgtcac ccatgctgga 5820

gtgcagtggc acgatctcag ctcactgcaa cctctgcctc ccgggctcaa gcagttctct 5880

gcctcagcct cccaagtagc tgggattaca ggcacctgcc accacgcctg gctaattttt 5940

gtattttttt tttttttttt tttttttttt tagtcgagac ggggtttcac catcttggcc 6000

acgctggtct tgaactcctg acctcgtgat ccacccgctt cagcctccca aagtgcaggg 6060

attacaggcg tgagccaccg cacccagcct ctggctcttt tctttttgac atgaaatctt 6120

aagttaatta tcttaaactt taagaactag aaggattctt agtccagcac catcagttta 6180

caaacaaaaa aaacttgtgt ccctgagaag ttgtgacttt tccagggcct cctagccagg 6240

ataccagttt ggtttttatt atatagctag ccgagtaata ctattaactt gacttctggg 6300

atttcaaagt attctatgac ttgactgttt aagggaatta tgaggcctca ctgaacctca 6360

aaagataatt ttaggtacca tcctggtagc tgtagagcag caacagacca gtaaaagact 6420

tggttggtgt tgcccttctt ctgggtttga ttacatgagt aattgtgtga atagtctcta 6480

agttgtatgc tctgagcttt cgtttttagc ttataaaagt gctactcttg ggccagcata 6540

gtatcagaaa ttagctaatt catatggggc agtttaggct ttaactagga aatgatggtt 6600

atcttaatga caaaaaaggt actgacaaaa gtcctttttt gaacatgtgt tcaaaagaaa 6660

aagagaaact atcaaactaa taactgagtt tgttgaacat cgagattgac tgatctgaga 6720

ggcttaaaac tgattgccct gaaatagact tgcatgtttt ggatgatggc tttgggtctc 6780

ccagagcact tggtttccct tggtgctgat tttttctctc aggattaaag cccctttatg 6840

tgatgttgtt acagcagctt attgcaactt cattcagctg cttgaaaaag aagggagact 6900

gcctctaggt tccatgtgtt ctttcaggga tggtattttg atgttgcgta ttctcttgaa 6960

catgtattct tccctgagaa tttctgtgtg ccgtggtaga agaaatactt gccagaaatc 7020

gccactcatg gtatgctttt gtagtgtcga agtgtcccct agaggtgaca aggcttgtga 7080

tagtgttgat tctaacaagc atgaatcttt cctttatttt agcactgtgt gttacgtgcc 7140

agtaatttgc agcttatcct ttgtttctag ctaggtaagc tgggaaatag cctagtactt 7200

tgtctatgtg tttatcttca aaatgtccca aatagccctg ggaaaaaggt cgtgcagctc 7260

aatgggggct ttcaacttac aattttcttt gttttaggct ccataaaaat acagactcac 7320

cagttcctgc tttgatgtga catgtgactc cccagaatac accttgcttc tgtagaccag 7380

ctccaacagg attccatggt agctgggatg ttagggctca ggtaagtaac cttccttttt 7440

ttttttttag tatatgtcct ggtttggcca tctgtttttt ttttttttaa aaaaaaaaaa 7500

aaaaggaaaa gaggaaaaaa atatactact cttggacagt ataaaagtac cccaaagact 7560

aaagacataa ctgtgccaaa ctgtgccata taataaaaaa aagtcacttc cctgagccct 7620

gaaaggtcag tgtgtgtagg gttacttggt cgccacagcg tgatctgggg gcgggcgtca 7680

gattagagcc ggaactggtg atctgcaact tcagttcacc ttgaagcagg tcagctgagc 7740

tgagagcgct tgcactgagc ccttttgcgc tgctgctgtt gccttagcgg tcttcagcat 7800

gtgtttgctt tggtttgggg taaatggctt agtggtcaac attagtgagc agtggtaatg 7860

cattttcaga tatgggaact ggtatgtggt tggttcccta gaaggacacc ctcctgaaag 7920

ctgtctcaga acaccggggg ccatggctaa tgtcatgtgc ttgctacact cctcccatgg 7980

taactaaaga gagtacccag atatacatct ggttcttggg actctagagg aggatgagta 8040

ctttgtaaaa acctgggggc cccagtcatt ctaggtctga cactcaggtg ctgtatcagc 8100

tgcagtttga actacttggc accattgtgt ggactttagt tttgtaaaaa tgaatggttc 8160

cttaatttct caaccttcag gctcaacatc tcaagctgtt tgttgttgtt gttgttgttg 8220

ttgttttgtt ttgtcttttt gattagtggt cactccgcat ttgatgtttt gacatgtgga 8280

tttcagaact tctgtgtggt aaagcagaat gttccaattg aattttccca ttttttttcc 8340

cctaagcaaa aatgtgagtt ttcacttatg cggccattgt gattccgact gagaccctaa 8400

gtcgttcttt gttgtctctc tagtggtttc tgacacctca gtgtgaagct gttgtagaca 8460

tccataagaa atagcctgct tagggattat gtgagggcaa ggtttggctg aaggagaata 8520

gaaggtgtgg aaggaagcgg aagaccagaa gagcatcaca ctgcctcaag tcccaaattt 8580

gattctgctc ctgatcctgt gactcaaatt gtcattctct tttactgctg tggggtgtgc 8640

tctgggccca gctgacagga cattctttgt actccacgta tttatgctgg tgggagttgt 8700

atggctagtg ctttgtgctt tgtctcagaa attgtgtgga ctaaatgtaa tatgggcagt 8760

gaatgggttt cttctggaag atgttaggcc tggagggtgg tttgggtttt tacgagtctt 8820

tttctcagat tacagattac gaatggatgt tataaaacag gcagtgttga caccatacag 8880

tttctcctaa gaaatgtaca gagagttcaa cctgaggaag ctaattgaaa ataaactttt 8940

aaaaagaaat gtagaaagag gaaatttaaa aagtatctta tgaatgggaa agatgtagcc 9000

atttgagctt caaagaactt ttgagggaat tctcaaagga cagtacccgt ctttagtaga 9060

taaatgcttg atatctattt ttcttctttt actttttaaa gaattactaa aataatacat 9120

ggatttatag aaaaatcaaa ccacttaaaa attgatcagg tggctgggca aggtggctca 9180

tgcctgtaat cacagcgctt tgggaggcct aggcgggtgg atcacctgaa gtcaggagtt 9240

cgagaccagc ctggctaaca cggcgaaacc ccatctctat taaaaataca aaaattagct 9300

ggccatggtg gcttgagcgt gtagtcccag ctactcggaa ggctgaggca ggagaatcac 9360

ttgaacctgg gaggcggagg ttgcagtgag ccaagatcac accactgcac tcctgcctgg 9420

gtgacagagt gagactcggt ctcaaaaaaa aaaaaaaaaa aaagtatcag gtaaaaaaat 9480

gaaagctccc cgcttaatct ctgctcccac tacccagaat aactgctgtt aatagcttaa 9540

tgtagatcct tctaagtctt ctaagactaa actgtgtact ttttgtgttg taattaacag 9600

gagggttttt gttttgtttt gtttttcttt tttttgagat ggagtcttgc tctgtcatcc 9660

aggctggagt acagtggcac aatcttggct cactgcaacc tccacctcct gggttcaagt 9720

gattctcctg cctcagcctc ctgagtagct gggattacag gcgcctgcca ccacacccag 9780

ctaatttttg tatttttagt agagatgggt ttcaccatgt tggccaggct ggtgtcgaac 9840

tcctgacctc aggcgatcca cctgccttgg cctcccaaag tgctgggatt acaggcgtga 9900

gccaccgtgc ctggcctcag gatggtgtta tatatacata ttctgcattt ttccatctca 9960

tatattcatc tctgcctcat ttcttttaaa tagcagtgta gtccagggct gacttaccgt 10020

catgcatttc gttgtctctc tatgttatta aaaatgctac aggattgaaa ttcctcatgt 10080

gccagtcatt ttgaagaatg gctgcctaga cttggaattt ctggtggata ccaccacatc 10140

actgtccaaa atggctatcc cagttcccac aacagtctga gaaagtgcca ttttcccata 10200

tcgtcccaga attggatata catcttttaa attggtgccg cttggattat tactgaggct 10260

aaacctcaat aatcattgtc atagctctta atgtattaaa gatgttaact ctttgtcatg 10320

cagatatttt cctgttttgt cttacgtctt gattcttgtc tttctgatat ataaaattta 10380

tgttatgatt tattcttttg ctttcatact taggaaatcc ttttttactc tttttttttt 10440

tttttttttt tttttttgag acggagtctc gctctgtcgc ccaggttgga gtgcaatggc 10500

gtgatcttgg ctcactgcaa gctccatctc ccgggttcat tccattctcc tgcctcagcc 10560

tcccgagtag ctgggactac aggcgcccgc caccacgcct ggctaatttt tttgtatttt 10620

tagtagagac agggtttcac catgttagcc aggctggtct cgatctcctg accttgtgat 10680

ccgcccgtct ctgcctccca aagtgctggg attacaggcg tgagccaccg cgccccgcca 10740

ggaaatcctt tcctactctt aacaacagat caatagtcat ctatattttc ttcctaaaaa 10800

acctgatgat gtattatatt catacatacg tatttaatac atacaaaagg gccagatgcg 10860

gtggctcatg cctgtaatcc cagcactttg ggaggctgag gcgggtggat cacctgaggt 10920

caagagttca agaccagcct ggccaagatg gtgaaatccc gtctctacta aaaatacaaa 10980

aattagccag gcgtggtggc gggcacctgt aatctcaact actggggtga ctgaggcagg 11040

agaatcgctt gaacccagga ggcagaagtt tcggtgagcc aagatcacgc cactgcactc 11100

ctgcctgggc gatagagtga ggctctgtct caaaaataaa aaataaaaat aataatttta 11160

tagttaagtt gccctgtgtt tttcctgtga ttaaaaaaaa aaatacctgt tttgtattta 11220

actttggggt gggataaggt atagcccact tagtcagttg tgtctaagcc acttgatgaa 11280

ccagtcaatc cagtcttcac ctttaacaaa tgctgatttc ttcaagtgag caaaatatct 11340

cttaaacttt ttcctatttt ctctataaag ttgctttttt ctgaattttt tctttatgag 11400

gcttgatggg cagccctatc agcagacaat cattcattca cccaacaaat atttgagtat 11460

ctgcttcaag ccaggggcag tgctaagccc tggaagtggt atcttctcct tatccgggag 11520

tggggctaca gtgttcattc cagaagactc aagaaaatag cagatcatca caaaagtagg 11580

ttttacataa atgaggagga atccacaggg ttgtgtatat gtgtgtgtat tttaaattct 11640

gacataagtt ggagctttag ggtagactgt gacataagag tagtttgttt tccaactcat 11700

gtaattgccg gctgattttt gtcttgaatt atgataatgg taacttctgt tgaaacaatt 11760

ctgtattcat cttagcttgg ttgggatgca ataaaagttt tgtactggta aaaatgataa 11820

ttttcttcca tgatgatttt gaagcttttc tgtaacacct gataaaaata gaggggccag 11880

gcatggtggc tcatgcctgt agtcccagct actcgggagg atgaggtgag aggatcccat 11940

gagcccagga gttttaggct acagtgagcc atcatcacac cactgtactc ctgtactcta 12000

gcctgggcag cctcgttctt tagagtaaga cactgtctct ttaaaaaaaa aaaaaaagaa 12060

aaaagaaaaa aagaaaaggc ttttagtgaa tatattttta gggtagcaag tgtaattttt 12120

ataaattgca ttatttgaat ctaataaata ccctcaaagc tgaagtcgaa ttctgatttt 12180

gattgaaaat attttttact taaaggaaga gggatagaat aaatgacacg attaaggaaa 12240

agacttcttg tataaggccg gggtttttta aatgattttt tgaatagtta gatggacaat 12300

acggaattaa aaaggtggtg taaaaagtta tacggggctg ggcacagtga ctcacgcctg 12360

caatcccagt actttgggag gccgaggtgg gtggatcacc tgaggtcagg agttcgagac 12420

cagcctagcc aacatgacga aacatcattt ctactaaaaa tacaaaaatt agctgggctt 12480

ggtggtatgc acctgtaatc cagctactcg ggaggctgag gcagaagaat cgtttgaacc 12540

caggaggcag aggttgcagt gagccaagat cgtgccattg cactccagcc tgggcgacag 12600

agtgagactc catctcagaa aaataataat agaaaaaagt tatatggggc tgggcatggt 12660

ggctcatgcc tgtaatccca gcagtttggg aggctgaggc agcttgatca cttgaggtca 12720

ggaattcgag actagcctgg ccaacatggt gaaactccgt ctccactaaa aatacaaaaa 12780

ttagccaagc gtggtggtgc gtgcctgtag tcccagctac ttgggaagct gaggcaggag 12840

aatcgcctga acctgggagg cagaggttgc agtgagccaa gatcatgccg ttgcactcca 12900

gcctggacga caagagcgga actccgtctc aaaaaaaaaa aaaaaagagt tatattgtaa 12960

agtatctctg ctaccctgcc ccaaacattc atttctttcc ctggaggcag ccactattac 13020

ctgtttcttt aaaaaaaaaa aaaaattgtg gccgggcacg gtggctcacg cctgtaatcc 13080

tagcactttg ggaggtggag gtgggtggat catgaggtca agagatcgag accatcttgg 13140

ccaacatggt aaaaccccat ctctactaaa aatacaaaaa attagccggg catggtggcg 13200

ggcacctgta gtcccagcta ctcgggaggc tgaggcagga gaatcgcttg aacctgggag 13260

gcagaggttg cagtgaactg agatcacgcc gctgcactcc agcctggcaa cagaatgaga 13320

ctccatctca aaaaaaaaag cgtgtgtgta cccgtgtgtt aaatttttat tataaaatat 13380

atataacagg ctgggcgtgg tggctcacgc ctgtaatccc agcactttgg gaggccgagg 13440

caggctgatc acgaggtcag gagatcgaga ccatcctggc gagcacggtg aaacctcgtc 13500

tttactaaaa atacaaaaaa ttagccgggc atggtggcgg gcgcctgtag tcccagctac 13560

tctgagagcc tgaggcagga gaatggcgtg aacccaggag gcggagcttg cagtgagccg 13620

agatcgcgtc actgcactcc agcctgggcg acagaccgag actccgtctc aaaaaatata 13680

tatcgatata tataacaatt tacagtttta atcatcttaa ctgtacagtt cagtggcact 13740

gagtacattc acattgttgt acagccatta ctaccatcca tctctacaac ctttcgtctt 13800

gcaaaactga aatttcatac ctattaaaca ctaactccca cttctcccct ttcccccacc 13860

ccaccctctg gcaactacta ttctactctt tgtctctatt ggacatattt ttgtgtgttt 13920

aggtttgtgt atatatttta catgtgtgta taaatacaca agggtatgtg ggcgttgtgt 13980

ttttacacat actataaaat attgtgcaca tttgtttatt ttaatgtatc ctgtagatta 14040

tttagaatcg gttcatacag agccgtatca ttccttttaa gggttgggtt taaatagtct 14100

gttatggatg taccacaatt tatttaacca gtttttaaat tttttactta ttttttttaa 14160

tgttttttta gagacaggat cccactccat cacccagact ggagtgcaac agcatgatca 14220

tagcttactg cagccttgaa ctccggggct gatgtgatcc tcccacctca ggctctcttg 14280

tagctgggac tacagggatg tgccgccatg cctggctaaa ttttttattt ttatttttat 14340

ttttattttt ggtagacacg gggtttcact gtgttgccca ggctggtctt gaactccttg 14400

ggcttgagta atcctcctgc cttggcctcc caaagtgccg agattacaac catgagccat 14460

ggagcccgac aagagtagcc ttttatttgc agaccattag agaaaaagtt gcgtgtaagt 14520

agataagcat acgaatttat cactgcaata ctgtttgtag agatcacaga gctgttaggc 14580

taggaagaag tttttagcag ttagcactag aaagcaaaac tttggaaact tccatttcat 14640

caaaagtgac ctcatctgag tagggcatgt gagggaagga cctttttact cgacaagggg 14700

aacttgccag ttcttgtttg tgaaccttga agaaagtgga gcatttttat tttgggacca 14760

ttctaggttg tggtagttcc ttggtactcg gggttggaga atctccctct gctgctggac 14820

cttctgcctc ctcatgctgt aatatgcata agaggtgtcc tagaagtatt caggaacagg 14880

ctgtggttgc tttgttgttc tgtgactgtt gaactcttta tatagcagta cttttttaag 14940

ctgtactaat tgagcattta ttatgtgtca gttactgtgc tagctgctgg aaactatcct 15000

accctcagca gtcttctggt ctaatgggag gtacagacag gcaaatgggc tatgacttta 15060

caagaggcta acttctgtga tggagagaag ttccagccac taagggagca cataaaaatc 15120

agaggagatc ctaccaagaa ttgggaggta ggtcagggag gagacttctt tgaaaaatgg 15180

catctaagac ctgaacagca aggaaacatt agctaggtca aggacaaagc atcagtgggg 15240

agagatgcat gagtcttttg gaaggaccag catgtgtaga ggctctgaca ggagagaatc 15300

tctcaggccc attcagagag tccactgtgt gaagacgagg catgaacaac cagccaggac 15360

atgtggagag gagcattttt ctttttttct tttttctttc tttttttttt tttttttttt 15420

ttgagacgtc tttctctgtc acccaggctg gagtgcaatg gcatgatctc ggctcactgc 15480

aacccccact tccagggttc aagcaattct tctgccttag cctcccaagc agctaggatt 15540

acaggcaccc gccaccatgc ccggttaatt tttgtatttt tagtagagac ggagtttcac 15600

catgttggcc tcaaactcct gaccttaggt gatccacccg tctcggcctc ccagagtgct 15660

gggattacag gtgagagccc ccacgcccgg ccaggctcgg ctttctttcc aggccccgtc 15720

acactcttag atgttccctg ggccccagaa ggcagcgtga ggtgctctgc ccatctatgc 15780

tgtccttttc caccccaccc tatccactgg gcccctgggg ctcccactcc actccaggtc 15840

tcttcatgga cccttctttc ccttcacttc tgtcccagga gacctgggag actcatggga 15900

acaggcatca tcttctccag cacagttcat tgtctaatgc acgaaagtgc tgggctgtga 15960

gaaggtcagg agcttacatg gaatgggagt cagctgtggt acaaagtacg ctgcttagtt 16020

ctactgattt tttatttttt atttttacat aagactttga tggtaaagga agcagtgtgt 16080

gagcaagctg actcctgtct tgtcaagtac caggtccact ctgatgagcc cctgtggaca 16140

gcactgctgt aactccccaa gcctgggctc aggaggtggg ccctaagcgc tgttcccagc 16200

tcttcctcca tgctgtcagc tcttccacca ttcttcctgg cgtcattcta aggtcctgtc 16260

ttcccccgcc ctcttccttc ctggcttcta cgctcagctc aagtagtagc tgtttgggta 16320

cttttcaccc agacttagga aatgttatca gttggtcgta ttcctgtcag ccctcttact 16380

atggaactca aataggacag attcagctga attcccagcc ctgtggttgc actctttgtc 16440

tgccctgctt ccccaaggta gcctgctccc taaaggtcac cgagatactc cccatgcaag 16500

gtttttgttg tattacacat gacaacatgg aataaagtgt tttgcaaatt tactggatga 16560

tatcctacta tgtggaacat cctacaactt actcttttca ctcaacattt gacttttgaa 16620

cctggtctgt attgctccgt gcagaccaat gttcattcat ttgatatgtc tccttgtgcc 16680

atagaaatag ttttacaata gaagcctgtc cagagccatg agggagaagg ggattgtaga 16740

gaaacaagga ttggcatctg gtttatgttt acatccttta gtcctgttaa ccgagaagaa 16800

gatattttta tttccatgtt tgcgaaagct ttccacaaaa aaaagcatcc attttctaga 16860

cttattaata gggcagtcca aaggttttat ttttagattt gaaagaaagc aaagtaggct 16920

tctgagatat gcacatttcc ctgtgtccct gtctcttttg caacattcca cattgctgct 16980

aaaacttgaa catgagcact ttcaagcatg tgcttacttt ctatgtatga ctcatttttt 17040

aaaacattaa agcattttcg gccgggtgtg gtggctcacg cctatattcc tagcacttta 17100

gaaggccgag gcgggtagat cacttgaggt caggagttca agaccagcct agccaatata 17160

gtgaaacccc atctccacta aaaatacaaa aattagccgg gcatggtggc gggcgcctgt 17220

aatcccagct actcgggagg ctgaggcagg agaattgctt gaacccagga ggtggaggtt 17280

gcagtgagca gagatcgagc cactgcactc tagcctaggt gacagagcga gactctgtct 17340

caaaaaaata aaaataaagc attaaagcac tttgtctggt ggtatgtaag ttttggcatt 17400

acttagataa gttgtatttt aagtctttct acacacaata acttattttt aaaatacaag 17460

tttctgtgta cttccgggtt ttgtgttttt gtaaacagca gaaggtatta gtccatatta 17520

agtactaatt tgttctaata ttttcagctg tttttggaat tacacttggc aaaaaaatag 17580

acaaatgtct gcagttgtga cagtttgttt ttttgttctt tttaactcca gaataaataa 17640

ttctagtgag tttttttctt ggcttgtaag aacatgggaa tgattagagt catcataaca 17700

cttaagtagt tatagaaggt tgaacatctg tagtcagttg tataaaattt cctagtcatc 17760

cttccaggtt gtttaagaca aacaaataca tagtatttat taagctctta taaattttaa 17820

ttgtaaaaca tcattgtaca aaactttgaa aatgcgtata ggtacaaaga taaaaaatta 17880

ggccaggcat gccatggctc acgcctgtaa tcccagcact ttgggaggcc aaggtgggag 17940

gatcttttga gcccaggagt tcaagaccag cctgagcaac atattgagac cctatctcta 18000

caaaaatatc ataaaacaaa atagaatcac tctggcgatt ccactatcca aaaaaaaaaa 18060

aaactgctgt taatatttgg tatgtatctt tctagaccaa ggcctccaac tttggttgag 18120

aattcagccg ggtctgtgac cttggatggg aaaaacattc actctttatt ttcactaatg 18180

tctctgaaaa cccagatttt tagctgggca tggtggctcg tgcctgtaat cccagtgctt 18240

tggaagcccg tggagggcag atcacttgag cccaggagtt tgagaccagc ctgggcaaca 18300

tagcaagacc ctgtctttta aaaaaaaaaa aaatccagcc gggtgtggtg gctcatgcct 18360

gtaatcccag cactttgaga ggctgaggca ggcagatcac ctgaggtcag gagtttgaga 18420

ccagcctgac caacatggtg aaatcctgtc tctactaaaa atacaaaaat cagccgggca 18480

tggtggtggg cgcctgtaat cccagctact ccggaggctg aggcaggaga atcacttgaa 18540

cccggaaggt agaggttgca gtgagccaag atcacaccat tgcactccag cctgggcgac 18600

agagcgagac tctgtctcaa aaaaaaaaaa aaaaatccac attttcttca atgacaattg 18660

taagcaacag atcatggtag tattagaata gcagtgactg tcaccagtag agatcaaaga 18720

tactttcata tcatattata gttgttacag atattttgaa atatatatgt ccatcactgc 18780

ttcaaactta aggtaattat tagacccatc tctagatctg gttatttaat gatttactga 18840

agaagcacat attatttgag aacgaactct tgaaatatgt tgttaatttc tgtgcatttt 18900

acacatttca aaacattctt ctgaggaggg gtccagaagg ccaaaagggt ccatggcaga 18960

aaaaggttaa gaacacctgt tctgtacttt gtaccatgtg gctacaaata taaaaacaga 19020

cgttaagccg ggtatggtga tgcacacctg tagttccacc tacttgggag gctgaggtgg 19080

gaaaatcact tgagcccagg agtttgaggc cagcctgggc aacagagcaa gaccccatct 19140

cttaaaaacc agtcagcact tgactagtca gttagtgggg gaaaacacag gtacagtaaa 19200

aggagtcaaa attacactta aacttaggtc atcttattat atcaaacatt tattaacatg 19260

tttagtgttt tgtgtatatt atgcaaagtg taatgcaact aaaactgttt aacacttctc 19320

gatatcacat aaatttgttg gtgcataata gcctaaaagt tacttttgct gttggcattt 19380

tattttatta cttacttatt tatttattta tttatttatt ttgagacaga gtcacactgt 19440

cgccaggctg gagtgcagtg gtgcgatctc agctcactgc aacctctgcc tccctggttc 19500

aagcgattct cccgcctcag cctcctgact agctgggact acaggtgcgc gccaccatgc 19560

ccagctaatt tttgtatttt tagtagagat agggtttcgc catgttggcc aggatggtct 19620

tcatctcttg accttgtgat ctgcccacct cggcctccca aagtgctagg attacaggca 19680

tgagccacca cgctgggcct gctgttggca ttttaaacat ccttctagag tctttcttat 19740

ttgcctgtaa gcacgtacac atgttgttgt tgttttgaga caaggtctgt cgcccaagct 19800

ggagcacagt ggtgcaatca cagctcacta cagcttcgac ctcttgggtg caagtgatcc 19860

tcccaatcag cctcccaagt aggtgggact acaggtgtcc gctaccatgc ccagctaatt 19920

tttgtatttt ttttgtagag acggagtttc accatgttgc ccagactggt ctcaaactcc 19980

tttgctcaaa cgatccttcc gcctcggcct cccaaagtgc cgggattaca agtgtgcacc 20040

tctgtgcctg gcccacatat tgttttagat gaatgagatc atatatgtac ttttgtaact 20100

tgctaccttt cctcaacaaa atgttgtaaa tatccatgtc cataaaaata gacgtatatc 20160

ttcacttttc cctaaaatga aaataactta acttgcattt tcttttttgt ttttgttttt 20220

gttttgagac aggctcttgc tctgttgctc aggctggagt gcagtgatgt cacagctcac 20280

tacagcctcc acctccagtt tcaagtattc cacctacctc agcctccaag atagctaaga 20340

ctacaggcac atgtcacgac gcctggctaa tttttaaaat attttttgca gaaatgggat 20400

ttcactatgt tgcccaggct ggcctcaaac tgctggcctc aagtgatctt cctgccttgg 20460

cctcccaaag tgctgggatt acagtcgtga gccactgtgc ccagcctgca ttttcttatt 20520

ataaaagtaa tacatgttca atggacaaaa aattttcaga aaatatgcaa agatgaaaag 20580

taaaaattgt ccataaatct gtcatctaca gataaagata acttctggat aatgtttttc 20640

taccatcatt tttagtaatc acataacatt tcgttgtatg tctatgcctt catttaatta 20700

agaggcattt ccattatttc tgcatgttca tgactctgaa tgatgatatg tctgcctgct 20760

gatggctaca accctgtttc tgcatttcaa acctctctct tgagctccag atttgatggc 20820

ctcgtcagca tttacttgag ttgctcataa gtgtctcaaa tttaacaagt cccagtcttg 20880

atttttttcc cccttcaaac ctattcctca tgttgtctct atttcagtaa acaatatcaa 20940

catccaccca gttactcagt ccaaaattct aggagtcatc cttgattctg ctctttcttt 21000

tttttttttt ttttgagacg gagtctcgct ctgtcgccca ggccggactg cggactgcag 21060

tggcgcgatc tcggctcact gcaagctccg cttcccgggt tcacgccatt ctcctgcctc 21120

agcctcccga gtagctggga ctacaggcgc ccgccaccgc gcccggctaa ttttttgtat 21180

ttttagtaga gacggggttt caccttgtta gccaggatgg tctcgatctc ctgacctcat 21240

gatccacccg cctcggcctc ccaaagtgct gggattacag gcgtgagcca ccgcgcccgg 21300

cctctttttt ttttttgata cggagtctca ttctcgtcat ccaggctgga gtgcagtggt 21360

gcaatcttgg ctcactgcaa cctccgcctc ccgggttcaa gcaattcttc tgccccaacc 21420

tcctgagtag ctgggcctgg ctaatttttt gtatttttag tagagatggg gtttcaccat 21480

gttggccagg ctggtcctga actcctgacc ttgtgatccg cccacctcag cctcccaaag 21540

tgctgggatt acaggcgtga gccactgcac ccgccctgat tctgctcttt ctctcatcat 21600

ccctgatctg tcagcaagtc ctgttaggcc aaccacttct cagcatctgc actgccagta 21660

tgctagtcca agccacaaaa cacctctcag aggtacgcaa caggactttg ttgttgttgt 21720

gtgtgtgtgt gttttttttt tttttacggt agaattattt tataacttga agtgtaggga 21780

tgaccttcct tttaaaattt tatttatttt cttaattttt tattcccata gattttaggg 21840

gaacaggtgg tatttggtga catgagtaag ttctttaatg gtgatttctg agattttggt 21900

gcacccatca cccgagcagt gtacactgaa cccaatttgt agtcttttat ccctcacccc 21960

ctcccaccct ttccctcaag tccccaaagt ccattgtatc attctgatgc ctttgcatcc 22020

tcatagctta gctcccactt atgagtgaga acatacggtg tttggttttc cattcctgag 22080

ttacttcact tagaataata gtctctagtt ccatccaggt tgctgtgaat gccattaagt 22140

cattcctttt tatggctgag tagtattcct ttgtatatat atgccacaat ttctctattc 22200

actttctgat tgatgggcat ttgggctggt tctatatttt tgtaattgtg aattgtgctg 22260

ctataagcat gcgtgtgcaa gtatcattat gcaacgggac ttcggatgac atattgaaca 22320

acttcctcac tgatatcctt gtgaaacgct acatatttca tgggtctgtt tgttatacca 22380

acactcaatt ctgaatgatg gaattatttc acatttagtc tctttttcat ctagccatta 22440

tagtttagtt taaacaccaa gttccattcc tgctgcatgc tgaaggattt gcagtacatc 22500

aggctatagt ccatcaattc attccacaat tgagtaagta aggtatctat agtacctcag 22560

gccctgtgtt aggcagggat ggggtaacct agacagattt ggtacctgtc ctcatggagc 22620

ttgactttag cggcagagac agatcttaga taattgtacc agtacttaag tgttcagaag 22680

gacaagtatg gaggtgggag ccactccagt cctctacagt cacgtgctgc acaacaacgt 22740

ttcaaccgac aacagagcac acatacagtg gtgttcccat aagattagaa tgccatattt 22800

ttactgtacc ttttctatgt ttagatatac aaatacttac cattgtgttc cagtgatcta 22860

cagtattcag tatagtacca tgctgtacag ttttatagcc caggagcaat aggctatata 22920

ccgtatagcc taggtgtgta gtatgctaca ccatctagat ttgtgtaaga acactctctg 22980

atgttagcac aaataaatcg cccaatgaca catttctcag aacatatccc tgttgttgag 23040

cgactcatga ctgtacaatg ggggaacagc tcagcctgcc ttccccaagg aggcaacgcc 23100

gatgcaggga accgagggtt gaacagaagt gggcaaggtg cagagaggaa aaaggctttc 23160

cagatggaga agagcatgtg tgaaggccct cagtctggca agagcttagt cctgtgtggc 23220

aggagctgaa cgaggggtta ggggagagtg gcaaagggtc cgggccatca gagaggtcaa 23280

caggggctgg atcatgcagg attttgaatg ttaacatcag agcgatagaa aacatgaagg 23340

gtaaggtggt ttgggttttg aatttttatc aaaatattgc aactactcgg caacaagtta 23400

gatataacaa aagaggtcat cgtaaaaaca gcagtctacc ccttcccctt cccttccctg 23460

actcagacat gacagctttt aactgtttct gattttagtt cctttgatgg ttacctccaa 23520

aattaaatca tatgcttata catctttctt gatttgccaa ctttagggaa ctgtgttaac 23580

tccctgaaat gaaagagaat gacttagctt acgttacctc cccatgttga aattcatatt 23640

cacatttctc tgtaattttt tttttgagat ggagtctcac tctgtcgccc aggctggagt 23700

gcagtgatgt gatctcggct cactgaaacc tctgcctccc gggttcaagc aattctcgtg 23760

cctcagcctc ctgagtagct gggactacag gcgcacacca gtgcactggg ctaatttttg 23820

tatttttagt agaggcgggg tttcaccatg ttggccaggc tggtcttaaa ctcctgacct 23880

caggtgatct gcctgccttg gcctcccaaa gtgctgggat tacaggtgtg agctaccatg 23940

ccaagcccac atttctctgt aaattttaaa cagtatttta gtccatatgt cagttcccct 24000

ctttttaaga taaggatatt aataacccta cttttccttc cactttccct ccttaacttc 24060

tgcgtcttaa aagctgtact tgaccatacc cattcttgca tttgttaaca tgtgtgttct 24120

gccctgcaac cacagccaag ttgtagctta attctaaagt cgaaaataac attatttaca 24180

ttattacaca tgtgagtatt gttcttggcg agaccaaata atgaatgtgc ccaggattac 24240

atttccttct ttacagatct ggaccactca aaggagaatg accaatcaag gtcaaatgga 24300

ttcccttttc ttagactctg tcacctgctt aaaatccttg ctaggattta atctacttca 24360

tattcaaacc ttggcttgct tatatagctc tttgttttgc ccaccatttc tagttgcctt 24420

tttattttgt ttactattcc tccttgatta tgttgtaaaa tgctattagc agtgctgttt 24480

atttcgtacg attcggcttt ttccccactg aagacctttc tcttggaagg cctctgccct 24540

tctgcaatct gggcaagtat ctgctctttg atttaaggtt gcatatacag ctgtacctgg 24600

accttctcct cactgggctc ttgggttgga gcccgtgtct taaaacccat atcttccttt 24660

cacatggttt actccctcgt tttctggact gcatccttaa ataatgtgct cagatagagt 24720

gcatggggag taaactgtag tggataccgt tgtcacctac cccatatcca tttcttcttt 24780

gcccattcct aacagaatcc tgatattatt cacctttcta ccttattccc aacttcagaa 24840

aggatcctta agtcattcca cacattgcat tcctttggca actgttactg gtcaaggatg 24900

gacatacgat ctaagttggt tcactcagac tgaagggaag gacttccatg gcacgttcag 24960

ggaagacttt ctcttttctt ctcactggag taagtgagaa aggtgttgct ggggctctt 25020

ctgtaacctc agactgaggg acaaagcctg aggataaagc ctgctcacca aggaaggcgg 25080

agcagagaaa cgggaagaac ctgggtcttg atgccgttgc ttagctggtg attgcactgt 25140

ggctggggcc tgtcctacct gagctaggga ctcagatagg atggactttc ttactgaatg 25200

agccagcttc agttttcttt tctgttactt gtaaccaaaa gcattgtaac agataggtct 25260

tctgagtcac tgcatgcctg aaataccttt cttctactct tacacttgat tggctgagta 25320

tagggttcca agttgaaaat catttttctt cctaactttg gaggcatctc ttctagtatg 25380

cagagtaaca aatgcaacaa atgcctgatg ccaatcttat tatggttctt ttgtaggtgc 25440

ccttcttttc tcttccttcc ctggaagttt ttaggatttc gtctccttat ttttagtgtc 25500

ctgaaatctc atgagaatat gtctagagtt gtgtcttgtt cactcgttgt actgtggtca 25560

ttcaatctga atacttttgt caactctttt ttttaaaaaa aaattactaa tttacatgct 25620

ttcattttct ctatggggac tactgtttaa ctttggacct cttatattgg tcttctgtct 25680

tctctcatat ttttcattta ttgggcggtg gggggtgctc tgaattttgg aagatctcct 25740

gcatttattt tctttttcct tttttctttt tttttgaggc agagtcttgc tctgtcaccc 25800

aggctggagt gcagtagcct gatctcggct cactgcaacc tctgccttcc aggttcaagc 25860

aattctgcct ctgcctccca cgtagctggg attacagacc tgcaccacca cgcccggcta 25920

atttttgtat ttttaataga gatggggttt caccatgttg ccaggctggt ctcgaactcc 25980

tgacctcaag tgatccgccc gcctcagcct cccaaagtgg tgtgagccac cgcacccagc 26040

ctcctgactt tattttctat cccttctgtt aaattcttta tttcagccaa accttttttt 26100

tttttttttt aacttactgt gaagcctgtg tgcctactgc ctagattaga caattgttaa 26160

cattttggcg tattttcctg gtctgtatag atagggtgca tttttatacc tttttgctca 26220

ccatttaaaa ggaactcaca gatgtcacga catttcaccc ctaagtactt cagtatacat 26280

ctcctaaaaa taaggacatt ttccaatata agcacaatac cccgatcaca tctttaaaag 26340

ttaacaataa ttctctaatg ttatcaggca cccactttaa attcagattt cctccaaatg 26400

tctcttatcg ctgttgaggg gctccaaagc aattaagatt tatacattgc atttgaatat 26460

gcttcctttg tctcttattt atttatttat tttattttga gacagagtct cgctgtctcc 26520

caggctggag tgcagtggtg tgatctcagc tcactgcaac ctccgccttc caggttcaag 26580

cgattctcct gcctcagcct cccaaatagc tgggattaca ggtacgcacc accacgccca 26640

gctaactttt ttgtattttt agtagagaca gggtttctac taaaccatgt tggccaggct 26700

ggttttgaac tcctgacctc aagtgatctg cccgcctcgg cctcccaaag ttctaggatt 26760

ataggcgtga gccaccatgc ccagcccctt tgtctctttt aacaagaaca gcctacccac 26820

ctctccttca cttgtgatac tgactttttg aagcagtcag cccaattgtc atctagaagg 26880

gcccacattt taagatacat atttttagag atggcgtgtc actatgttgc ctaggctggc 26940

cttgaactcc tggcctcaag taatcctcct gcctcagcct cccaggtagc tgggactaca 27000

ggcttgtgcc accacgacta gctggcccac attttagatg tctctctttt cttgtgatat 27060

aatttaactt gtttgtccat atatattact tgtagacaag aagttaggtc tacaggcatg 27120

ttcaggttaa acattttggg caagaatatt tcataggcag tactgtgtac ttcatatagc 27180

atcagctcac caggcactta atggttggtt accctgtggt ttgtgatgct acgttttaac 27240

acttgattaa agaaggaggt gactgccaaa tctccccatt gtagacatac agttttcctg 27300

tggtaattga tatataatct gtgatgtgat gctttggcac catacaaata tcctattgcc 27360

caacagccat ttgcataatt attttagcat tcattgatcc ttgcctgaaa ccattattac 27420

attgggggtt gcaaaatgat agttttttta attgtcacct cttctacatt tagtactgtc 27480

attcctctgt aatgaatttt ccctcatcaa ataaggaggc attatatttc ctcctaaaaa 27540

gacagtatag atgcttaatc tttgccttta ttacatttca gagtaaggag ttggtagaat 27600

aatcacctca aatggtggca aaatatttgc tgttgtgttt ccttttctct ttcatgtcaa 27660

tatggactca tagggattta tttattaaat gttttacaat tcaaattggg tcaacaagag 27720

tcccttcatg ttggttccta tattctcctg gcataccctc attaattagt ctttgagcaa 27780

tttcttgttt tctggcacaa gatattccat cccaatatgt gcttttcgtg tcctagaact 27840

gattccaagg agctctggtt ccttatagtg gcaacggtat ttagaatcca acatgtgggt 27900

actaaatgtg ctgattctta ctggggtaac attatatcta ggcactttca gaggacatcg 27960

ctagaaaata tattcagttt ttatttttta tttttaattt ttttgtagag acaaggtctc 28020

actatgttgc ccaagctggt ttcgaactga gctcaagcga tcctcccacc tcagcctccc 28080

aaagtgctgg gattacatct gtcagggcaa actgctggta aatattttca caaaagaaaa 28140

ataaaaaaac caaagtgctg ggattacaga tgtgagccac tgtgccctcc attttttttt 28200

taagtcataa atacataatg atgtcttcta tggcaattta acattacagg gttcttcctt 28260

aacttttttt tttttttttc ttttttttga ggcagggtct tgctctattg cccaggctgg 28320

agtggtgcag tggtacaatc ctaattcact gcagcttcaa atgcctaggc ttcaaacaat 28380

cctcctccct cagcctcccg agtagctaag actacaggtg catatcacca tgcccagcta 28440

atttttaaaa attttttgta gagatggggg gtctcgttgc gttgcccagg ctggtctcga 28500

actcctggcc tcaagtgatc ctcccatctc gatttcccaa agtgctggga ttacaggtgt 28560

gagccaccat gcctggccaa cttttatttg atctgtatat atcttttcct tacactgaaa 28620

atgttgggtc ttactaatgt taacatagtt acttatttgc tttatcttgt aatagacata 28680

aaattacaaa attataatac caatattacg ataaatagct gggcatggcg gcgctcgcct 28740

gtagtcccag ctactcagga agctgaggtg gagggactgc ttgaacctaa gagtttgaag 28800

ctgcagtgag ctgtggttgt gccactgtac tcctacccgg acaacagagt gagtgggggg 28860

aaaaaaaaga aataatggaa gatccagaga aagccccaaa tatttaaaaa ttaaacaata 28920

cactttgcag cttcctcacc tctctcagcc ttcatagaat tgaagagagt tagggccttg 28980

cactagatta ggctttgcct taaggaaatg ttgtgtttga tttgatcttt tatccagacc 29040

attaaaactt ccttaacgtt tattagtaat aaggctattt cactttcata tcatttgtgt 29100

gttcactgga gtagcacttt tagtttcctt caagaacttt gattcacaac ttggctaact 29160

ttggtacaag aggcctagct tttggcttct cggctttcaa catgcctctc actaagcctg 29220

atcatttggt ttttggttta aagagaaaga catatgactc ttccttttac ttgaatgctt 29280

agaggccatt gtagggttat taattggcct aatttcagta ctgtcttgtc caagggaata 29340

gggaggccca aggagagaga gagagatggg ggaacagctg attggtggag tagtcagaat 29400

atacacattt atcacttaag ttttgccatc ttttcttttt ctttttcttt tttttttttg 29460

agacagtctt gctctgtcac ccaggctggc atgcagtggc atgatctcag ttcactgcaa 29520

cctctgcctc ctgggctcaa gcaattctcc tgcctcaacc tcccgagtag ctggtattac 29580

aagcgtgcgc caccataccc agctaatttt tatatttttt agtagagaca gggtttgcca 29640

tgttggccag gctggtctcg aattcctgac ctcaagtgat ccacccgcct tggcctccca 29700

aagtgcgggg attacaggcg taagccactg ccgcctggcc aagtttgcca ccttttttat 29760

tttttttatt ttttgagatg gagtctcact ccatcaccca ggctggagtg cagtgatgca 29820

atctcggctc actgcagtct ctgcctcctg gattcaagcg attctcctgc ctcagcctcc 29880

cgagtagctg ggactacagg catgtgccag cacgcccagc ttgttttttg tatttttagt 29940

agagacgggg tttcaccatg ttggccaggc tggtctccaa ctcctgacct caggtgatct 30000

gcccggctca gcctcccaaa gtgctgggat tacaggcgtg agccaccccg cccggccaag 30060

tttgccatct tttatgggtg cagtttgtgg tgccgcaaaa tgatgatagt agtaacatca 30120

gagagcattg atcacagatc accataacaa atataataat gagaaatttg aaacattgtg 30180

aaaattacca aaatgtgaca gagacatgaa gtaagcacat gctattggaa aaatggcact 30240

ggtaaacttg cttgatgcag ggttgccaca caccttcaat ttgttaaaaa ctcagtatct 30300

gcgaagtaca gtaaagcaaa gcacaatcaa atgaggtgcg cctgcatatt acaaccaaga 30360

ggcttttcgc agaaacacga gtgtgcttca acattcaaaa atcagttggt gtaattcacc 30420

acattaagat aataaaggag gccgggtgcg gtggctcaca cctgtaatcc cagcactttc 30480

agaagccgag gtggacagat cacttgaggc caggagtttg agagcagcct ggccaacata 30540

gtgaaatcct gtctctacta aaaatagaaa aattagccgg gcagggtggc acgcaccttt 30600

agtcccagct attcgggagg ctgaggcagg tgaatccttg aaaccaagag gcggaggttg 30660

cagtaagctg agatcacgcc aatgcattct agcctgggca acagtgagac ccagtatcca 30720

aaaaaaaaaa aaaaaaaaaa aaagataata aaggagaaaa accatatagt ctattcaata 30780

aatacagaaa acgcatctga caaaattcag tgccctttca tgataaaaat tcagcagatt 30840

agaaatagag gaagtgcatg tatgaaagag ctacagatag attgtactta attgtgaaat 30900

attgaacatg ttaccccgaa aatcaggaaa aaggcaaaga tgtccactct aaccacttct 30960

attcaacatt atactagagg tcatagccag tataatgagg caagaaaaaa cataagaggc 31020

attaatattt aaaggaagta aaactgtttc tatttgtagg taatgtcgaa aatgtgaaag 31080

aaactatcag aacctgctag aactaaaaag tgaattcagc aaggtctcag ggtacgagat 31140

cattcaaaaa ctaatcagaa atgaaatttc aaatgcaata tcgttaggaa taatttaata 31200

agaaatgtac aagatttaca cgctgaaact gaaaatgttg ctgaaagaaa ttaaagacct 31260

aaataaatgg aatgttgtac catgttcatc agctgaaaga ctaagtgtct ttacagtgtc 31320

agttctccct tcattgaact gtagattcat aaccccagtc ataactgcag ggtttttttt 31380

gtagaaattg attgtaaaat gtatatggaa gctgggcacg gtggcttatg cctgtaaacc 31440

agcactttgg gaggctgagg cagacagatg acttgaggtc aggaatttga gaccagcctg 31500

gctaacatga cgaaaccccg tctctacaaa aaatacaaaa attagccaga catggtggca 31560

cacacctgta atcccagcta cttgggaggc tgaggcatga gaatcacttg aacccggagg 31620

tggaggttgc agtgagccaa gatcatgcca ctgcacgccc gcctgggcaa cagagtgaga 31680

ctttgcctca aataaataaa taaataaaac gtatatggaa atgcaaagga cctagaacat 31740

ccaaagtaaa cttgaaagag aacaaaatta aggggcttat gtgatgtgac tttatagtca 31800

tttgattttt aacagaggca ccagaacagt ccggtgcggg aaaggaaagt cttttagcac 31860

gtgccggatg atggcaaatg acatccatac cagacaaaaa tgaaccttga ccttacccta 31920

aatattagtt tcttagagtt gccacaaact aggtggctta aaaccacaga aatttggctg 31980

ggtgcggtgg ctgacgcctg taatcccggc ctttgggagg ccgagtcggg cagatcacct 32040

gaggtcagga gtgcaagact agcctggcca acgtggtgaa actttgtctc tactaaaaat 32100

acaaaaaact tagtcaggtg tagtggtgca cacatataat cccagctagc caggaggctg 32160

aggcaggaga attgcatgaa cctgggaggt tgcactgagc caagattgca ccactgcact 32220

ccagcctggg tgacagagtg agactccgtc tcaaaacaaa accacagaaa tttgctgtct 32280

cataattcca gagtctagaa gtccaaaatc aactgtcatc agggccatgg tctctgtgaa 32340

acctgtaggg gagtccttcc ttgcctcttc ctagcttcca gtggttggca acctttggca 32400

ttctttgatt tgcagctgca ttacttcaat atctgtcttc ctcatcacac agcattctcc 32460

atgtatgtct ctgtctttac atggcctcct tcttgtaaga acaacagtaa tattggattg 32520

gggcctgccc tactccagtg tgacctcacc ttaactaatt acacctgcaa caacgctgtt 32580

tccaactaag gtctcagtgt gaggtactga gggttaggac ttcaacatat ctttttgggg 32640

ggacatagtc caacccatga cactacttca caccatacat aaaaattaat tcatgagaga 32700

ttatagacct aaatgtttta aaagctaaaa atataaagct tctgcaagaa aacataggag 32760

aatatctgtg cagtccaaag aataaggttt tttttaaaca caatttcact ctgttgccca 32820

ggctggagtg cagtggtgcg atctcagctc actgcaacct ctgcctcccg agttcaagcg 32880

attcccatgc ctcagccttc caagtagctg ggattacagg tgtgcgccac catgcctggc 32940

taatttttgt atttttagta gaggcagggt tttgccatgt tggccaggct ggtctcaaac 33000

tcctgacctc aagtgatctg cctgcctcgg cctcccaaag tgctgggatt acaggtatga 33060

gccaccgtgc caggccaaaa aaagatttat taggatacat gaagcaatag ctattaaaag 33120

aaaaaataaa ttggacttca ttaaaattta aaactttggg tcctcaaaag ataccattaa 33180

gaaaatgaaa agacagagct gggcgtggtg aggcttgcct gtagtcacta ctgggtactt 33240

gagaggctga ggcaggagga tcacttgagc ccaggagttc taggccaaca taggcaatat 33300

agtgagacag tgtctcttaa aaaaaaaaag aaaaagaaaa aacaaaagaa aataggctga 33360

gagctgtggc tcatgcttgt aatcccagca ctttgggagt acaaggcaga tggattgctt 33420

gagctcagga gttcaagacc agcctgggca acagtgaaat cccatctcta caaaagatac 33480

aaaaaattag ccaaccatgg tggcgtgcac ccgtagtcca tagtcccagc tactcgggaa 33540

gcagaggcag gaggattgct tgatcccaag gaggtcaagg ctgcagtgag ctgaggtcgc 33600

gccaccgcac cccagcctgg gtgactgagt gagaccctgt ctcaaagaaa atgaaaagac 33660

aagtccacca agaaaacagt tgagaccagt catagaaaaa atagaaagtc atatctgaca 33720

aaggatttgt attcagaaga attcctataa ctcggtaata aaaagacaac ctgattttta 33780

aagggtgaaa atattttaat ggatactttt tttttttttt ttgagacaga gtctcactgt 33840

gtcacctagg ctggagtgca gtggtgccat catacatagc tcactgcaga ctcgaactcc 33900

tgggctcaag tggtcttcct gcctcagcct cctgcgtagc taggactaca agcacgtgcc 33960

actatgcctg gctaattttt aaatttttgg tagacatggg ctggtgtgtg tctgtgttgt 34020

ctaggctggt cttgaactcc tgggctcaag tgatcctccc acctctgccc cccaaagtgc 34080

tgggccacca cgcctggtgt ggtgtcacca gagatgagcc accatgccct gcctgatact 34140

tttcttttat catctgttta tttcttgttt atgggtgttt ctgttctgat tttatgacta 34200

ttctgtattc taagatttct ctgagaatat tatttgtttc ctctaaggac agacttggtc 34260

tctcttccat ggggctggtt cttgagccgc cagggatggg gggaggacct gggtagctgc 34320

tgcgctgccc cttctctgct gtggtggagt ggcccgttgc ctgcggggtc tgtctctgaa 34380

gggtggtttg gagaagttct gtggaggcga ggcaggggtc agctcttgcc ccatggaccc 34440

tcagagactg ggaaaaggta ggcatcacct gggctgctgc cagccacatc aagagccttt 34500

ggggtgggtg gggtgggggc cggggaaggg aaggttctag ctgttgattt agaaaagacc 34560

cattgatttc ttgcctgagg gaagaagtgg caccttgcca ctccaagttt gctctgctca 34620

ccagccccaa cagcatcagc ctcacctggg agcttattga aggtgccctg ccccaaagct 34680

tgtgggtcgg gatctggcag tcagcagccc ccaggtgttg cacacatgac agcactgctt 34740

gcccctcctc tcgtctggtc tggtctggtc tggtctggtc tggtctgaga gcttctttgg 34800

ggctctgaca ggtatacagg ctctcatctg ctctacagct ctcttgtagt acattcctct 34860

ctgttccatt agtcaacatg ttcttgccta cttccagcat ctagaaattt gctgagtgta 34920

tcttatccgt tcagaggtcc tctcctgcta ttgcctctgt tagaaatttc ttccctttca 34980

tatgtctgta cttcggtttc tttgaggttt gaagggtcaa gtggcaatta catgtaggca 35040

gttggccatc ctctggtgaa ggctttaaag cccaggaata acaagatcca ttatgtctaa 35100

aactagcaaa aaccctacct gtcacatggt gaactgaatt agaggtgatg gggacagaag 35160

ccagattgga gtgagtggaa ggtgagggag tggagattgg gtatagacaa ctctctttaa 35220

gaagttttgt tgtgatggtg accagaaaaa taggagtggg gtagaggggg atgagggtgt 35280

ttttcacgtg ggtgaatatg agagcatgtt tacatccttc tgggaatggc ccaggagaga 35340

ggggagcgtg agaaggagga aatggggacc tggaggagca gactccttgt gtggaggggg 35400

gaagcggcat ctagagctgt tgcaggaggt gggctttgag taaatgcttc ctctttggta 35460

aagacgggga acgggaacgg tagaagttgc acggaccctt tgttaaagct cagaagctga 35520

gcgagaatta gagcttaagt ttatggagta gtcagaataa agtaccccta actgtattct 35580

tgcacagtgc aggaagccag gggtctgaaa tcaagatgtt gcagggctcc attcccccca 35640

gagcctctag catggatcct tccttgcctc ttccagcttt tggtggctgc ctggcagtcc 35700

tagactttcc ttggcttgca gttgcattgc tccattctct gcttgtgtct tcacatggcc 35760

ttctctgtgt ccccgtattc ttctctggat gcttgtcact gtattaggcc caccctaaat 35820

ccaggatgat ctcatatcaa gatccttcac ttaattacag aagcaaagac cttccaaata 35880

aggtcagagc cacagggtct gggggttaag acatacacct aactattcgg ggctaccatc 35940

gaacccctgt agaagcctgt ccatttccag gatagcagag cccaaggaag ggccagaagt 36000

ccccccaaaa cagttgtttg cattcaccag attctaagct ataagcagat gggcagtggc 36060

agtcggtcct aatccatata ccattggcaa caatagttta gttcactgta gacataatga 36120

gatgcttatc ttctgctaag tagtcctcat ggtaacagcc atattactcc tggctttgag 36180

tgacagcgct gtgctcttgt ctggtgccca tatacttcag cagctggaaa caagacagtg 36240

ctcatgattc accggaaagt ttctgtagtt aaaatcaagt gatcccttga gtctattcta 36300

atatttgtct gtaccatgtt ctgtgacgag acatggaaac aaaacattaa gaagtgaaag 36360

tatctttgat tatgctcttg aagacaactt tttgtttgtt tgttttgttt gagacggagt 36420

ttcactcttg ttgcccaggc tggagtgcaa tggtgtgatc tcggctcact gcaacctccg 36480

cattcccggg ttcaagcgat tctcctgcct cagcctccca agtagccagg attacaggca 36540

tgcaccacca cgcctggcta atttttttta ttttagtaga gacggggttt caccatgcta 36600

gtcagactgg tctcaaactc ctgacctcag gtgatccgcc tgcctctgcc tcccaaagtg 36660

ccgggattac aggtgtgagc caccgtgcct ggccgaagac aactttttaa agatgcatat 36720

gctctgggct gggtgtggtg gctcacacct gtaatcccag cactttggga ggccaaggcg 36780

ggcagatcac ctgaggtcag gggttcaaga ccagcctggc caacatggtg aaacaccgtc 36840

tctactaaaa gtataaaaat tagccgggtg tggtggcggg tgcctgtaat cacagctact 36900

caggaggctg aggcaggaga atcgcttgaa ccccagaggc ggaggttgca gtgaattgag 36960

actgtgccac tgcactccag cctgggcgac agagtgagac ttggtctcaa taaataaaat 37020

aaaataaaga tgcatatatg ctctgaaggc tggctacatt ttattttttc tcaaacaact 37080

tttacagtgg atagtgctgt aaaagttgtt tagctctggt cattttatct tttttgtgat 37140

ctgcaaaagg agatcctttt ccttttcact gcctctaata cccaactcca atactccttt 37200

gacctcacag gcacttagaa tccatcagtg gctccatctt ttctcagact tcacctcctt 37260

cgtatcctta ttcctctcct ttgtagcttc agtgccagtc agacgtcgtt actccctggc 37320

acactacctc agcccttcgg cactttcttg ctttgttata ttcagtcgac aaagccccag 37380

cctcatgaaa ttcagtcatg tctgccccat gcctgctccg tcctgaagct gaatgtgact 37440

ccttcattga ttcccccttt aagttcatgt ctaccccagg taaacttctt gccgccaagc 37500

ccacctgcat actgcagttc cccggtctct cctagcccct cccaccctcc tcatcagctg 37560

tcacacctcc ttgcagctga tatcttagtt cccagagagc acagaagcaa gctcaagaga 37620

gtgtctacag gccagcgtct actccttcgc tctgcctccc tcatgctgtc acggagcgcc 37680

tgtcccaggc cctcactcct gttctaaggc atctctccag tcctctactg cactctcccc 37740

catcagagat tcctccctcg cctgccctag aaacttgttg ctacttctcc caccttgaca 37800

aaagctgctc ccaacccccc acctgtctag catctctgtt ttcctttacc agcaagactc 37860

cagtccctgt ctccacaccc tcttcaacct tctccagttg ttcctttgct gccacagctc 37920

tgccaagctg ctccatgagg tctccagtga tgaccttcat gttgttaaat ctgggagctg 37980

cttgtcactt cttttcccag agctgtcgtc gtcagttaac cccaataccc atacgagaca 38040

atcctttccc tcccttgagt ctctgcactg atgtgcacct tctcagtgca aaccacagct 38100

tttgtctctt tctagctgaa tacatgactg actgtgctta tcttagctgt cacccctgct 38160

ggagcagaag ctccacagga caggattgtc tccttgattc acagatgtag ctcaagctcc 38220

tagaacagtg cctggtgtct gtgggtcctc aggaagtcct aggtgagtgt gtgaatgtct 38280

gtcctcctcg gtcttttctg tttcgcatgt gatgttggaa ggcctcaggt tcaggctttt 38340

cttctccacc ctcactcccg aagtgattcc gtccagcctc atggctccca aatttatgtc 38400

tccaggctga ctgggtcacg ggaactcctt atcctcccta gaccttctct ttccctggtc 38460

ttctcctttc cagtaaacag caactccaac tcctgctcct caggccagaa acctggcgct 38520

ggttacctca tttgttcaca aatactgtta ctccacctgc aagatggata caaaatctca 38580

tgagttcccc tgcctccatg gcccccaccc tggtccaggt gtccagcttc cctgcctctg 38640

cccttgcccc ctggttcttt gtttagtcct gcaatattgt gctcttctgt tcagaaccct 38700

ccagtgggtc cctgcttgct cagagtcaca gcccaacagg atcacctctc ttgccactct 38760

cccaccactc atgcactgcg gcctcactgg cctcctggct cctccctgga ccctccaagt 38820

atgcccctgc ctcaaggcct tgacctctcc tctgccctct gtcccaggta tcacacatgt 38880

ctgctcaagt gtcaccttcc caatgtggcc ttccccgagc acacccatgt ctgtaagtca 38940

ggcagcctgc ctctgcccca ccagttcccc ctgcctagca taggacttgt caccatctgc 39000

cacaagctgt gttactgctt ggttttgttt gtgctctccc actcccttcc cctccctccc 39060

tctccccacc cctatagaat ctgagtccca tgaggacaga gatttttttt tttgcctctt 39120

cctcaatata tccccagtgc ctagaatcat gcatatttta atttattgag tgaattaaca 39180

aataaattct gcagtgggac attgctctaa ggttttgtct tataagaggg acagaagaac 39240

atgcagcctg ctctgctgtt ggctccttag cttctccatg gctaacagga acaatattgt 39300

ttatgagata atcaaagcca tagtcatgag ttagaaggta catataaagg caaatatgtg 39360

gagattgagt tactttggcg gggcggaggg ggtgtgtagt tatctgaaaa catggaacaa 39420

atcaatgaga gtctaaatag cacccacata tttgcccctc ttttaaaaca tacctccttt 39480

atgagtgtag aatttaacag cacagtttcc cagtgcagct ttgcgaaaca agagaaatga 39540

attttaaagg ctgtcaggtg cctttgcaat ctgtggctta gtatttgtgt aacaacattt 39600

gtgttttgcc aacagatgta ctgcttaaag tacccatgag aatggcctct gtggttttct 39660

actttctcgg atggaacctt aggaactgat tattgagaag aggctgatac tgcccacctc 39720

agctcccagg caggcttcag ggccttggtt actactcctt tgggccctca ctgacccttg 39780

cagacgtctt ttgcaacctc tgtagccaga agagacaact gctttctggc atgttgctct 39840

ttccctaatg gctttatgag cttcttgaac accaaagcat gtgtgacttc ctcacgcctt 39900

cccctgtata gtatagcacc cctaactgta tgtgtaagga gaaggagtga gcaattgatg 39960

gcgtccccct ggatctccaa gggaatggga aggggcaggg tttctggatt cagtcatgtg 40020

gaatctggtc gttggtgtct aaatgtgcct gttacttgtg cccaatgtcc aggttcacca 40080

ggaggcttag cctgctcatt tccctgctag tagctatatt ttgggagagc attttttatg 40140

ctctaattga actttgtggg tctcagcaat ttgaacaatg ttttgttttt cttctggtca 40200

ggttacaaag gaaagaaaag gaatctggtg ttgtaggagg tggtgtgttg tgtaattact 40260

tgtttttttt ttttaagttt ttttatattt ttgcaacaag gcaagttttc tttgaataaa 40320

taatgctggg agcttatcac atagaaggca tatgtgttgt actcagtaca ttctgaatta 40380

tctgggttta tttttatctc ttgaattcac ttataaacaa ttcaaaggct gcttttttag 40440

ctaacagaaa taggaagaga atctcagaat ctgttttgtc attttttcaa tgggttgagt 40500

tatagtgatt atgctaactc ggtggaaaag cagtgtagtg agtaaacccc acatacggca 40560

tatcatgaac attttgttgc ttttttggct tctgaccatc ttcatacctt tttttgtgtt 40620

aaaagtctcc ccattgtgta tatcttagta agaggcaggt ggaccctagt ttctacctaa 40680

gaaaacctga agatggggtt gggggcctgt ggtgtacttc cttctcatgc tgtccatgac 40740

cactgcagtg gctttgtaag gatccaggac ccagggcccc tcagtgggtg tcttccattt 40800

ctcaagattg cctcttggtt gtgagatgac ggttgctgct ctagtcattg catccactcc 40860

tccaggtagg aagaaagagg tcatagggga aaggcaaaat ggcacttgac atcttaactt 40920

ctaggcccat tcaatatttc catttccatc ttcacaccca tcatcttgtc ctagggaaca 40980

aacagcacca gcagctcagt agctataaca gcaattatta tttctcacac atgggtctgg 41040

gtcagctgca agatgggatt cagatctgtt ctcaagcctg tcgttccagg acccaggagg 41100

gagaagcagc tgccagggga agtctcttcg taggcggagg tcaggagtcc aagaggagtg 41160

agcagagtca cagaagcctc ttaaagcctc ttcttccccc atcccatcaa cacgtgagca 41220

agcccagagt cagtgggtgg gaggtgtgct ccgcccatag tcagtagaac aagagcaagc 41280

ttgaaagaga acgatgaaca ggagtgcact cggccactgt ctccttagcc ataacgtagt 41340

cccatggtct gctgggaagc ggggaataac tctccttagc tgagcgcagg gtctcattag 41400

taaggcagat ggagaacatg gatgttgggg gacaactggc agtgcctgcc acaggggaat 41460

ggatatattc cctccccttg ctctctggaa actaggggat ggcgatgaag cctaggtttg 41520

gccaaaccca ttctgccttc agggactttg actcttgagg gacagtgagg gattagtcat 41580

ggtggtggca gttgagtcac aaaaccagca gtggtggcta atgtccagtg atgacatgca 41640

acagtgccca gggtgggtgt cccaaccagc aggtccctgc agtataacct tggctgtgtt 41700

tgcagtggtg cagcctctat tcctgctgct tttctgagcc tagttctcta gttctctctc 41760

catttccaga tctgctgccc aagaactcct ttgtgattga atttaaccag agttgatttc 41820

tgttgcttga aactcaggac cttgatgcag tgtgcagggt ctgggcaagg taaaaacaca 41880

ctttcatgat ttcgcctcaa gtatagtaga tgagcttagg ctttttgagc caggtagcct 41940

tgggattaat tctcagctct tgcacctagc aggtgacctc agcaatcaag tcacttgccc 42000

tccttgagcc tcagtgttgc cctcagttaa acattgctct cgtgtgttgc tgagcattca 42060

gtgagattgt atataaagtg atcattgtga aggtattggg acataatttg ggaaagcgtc 42120

ctggtctaag cttttgattg gctctccatc atttgggtaa tgggatagtg atttaggagt 42180

atcatagcag ctcaaggggc ccttctggaa agggtatgag gaaggtcagg gacaagtctg 42240

aaatgaatca tcctaggctt aatgcctgtg actgggcagt gggtacaatt atctcaggct 42300

agtcagtagt catttattgt gagaatacat gggcagattt tttaatttcc ttctcagtct 42360

agacttgaat gcaaacagtt cttattgcta acttaccact agtcaccact aatgaacaaa 42420

gactatgaac aggaaattca taaaagaaga gatacagatg gccaatgaag ataggaaaag 42480

agttctgcct gctggtaatc aaagagatgc aaacgagaac aaaaatgatg ccttttcacc 42540

taccaaattt gtcaagatta aaaagaaagc aaagagccag cgtcagctga tgttcatacc 42600

tgcacctgct cggtagcttg ctaatgttct gcctgctcca cacgccaggc cagcctccac 42660

ggcgcagcca ggtgaggcct ttccttgtat acaggcacgt ttggattcac tgttgcataa 42720

gtgagacatg tgcatgcttc acaattcagg aagtgcaaac tccagagtga tgcaaggtct 42780

gagtgtccct gctgccctgg tctcccagct gcctaggcct ctcttcaggg aacggctgca 42840

tccagggtct tgtctgtgtc ttttgaggtg ttctatgtac acgcagacac atctaaacct 42900

atttttattt tccctgcacc tgcttgggct actgtaagct gttaaatttt tttttctagg 42960

aatttgtcca ttttgtctga ctagcataaa gttgttcatg ttatagttta tacatttgca 43020

acatctgtaa ttatgtctgc tttttcattc ctagtgtttg ttgtttactt gtaccttctc 43080

ccgtttttcc ttgtcttggt aaaagctcat taggttcatt ggtcttacaa gaagcagaca 43140

tgtaggcatt attgccctgt attactctgt gtctcttttc tagttcatta aattctgctt 43200

ttgcttttat ctttattatt ttttctatgg tcttagagta cattctctgt gttttgttgt 43260

tgttgttgtt gttgtttgtt ttttgttttg agagagagag tcttgctctg tcgcccaggc 43320

tggagtgcag tagtgcaatc tcagctcact gcaacctcca cctcctgggt tcaagtgatt 43380

ctcgtgcctc aacctcccga gtagctgaga tttacaggca tttgccccac acccggcaaa 43440

ttttttgtat ttttagtaga gatggggttt tggcatgttg accaggcggg tctcgaactg 43500

ctgacgccag gtgctccacc cgccttggcc tcccaaagtg ctgggattac aggcatgagc 43560

caccacgcct ggccctaact tcttaagtta taaatttggt tcattgattt ttctaccctt 43620

ttttcaaatg taaacattta aggctaaccc tctggaacta cttcaactgt atctagcaag 43680

ttctgatagt attgtcactt taagttagcc caaagatatt ttaaattttc tttgtttctt 43740

cctttgactc acatattatt ttaaaatgtg tttttcaatt tccaagcaca tggctttggg 43800

tttgtttttt tttttcccta gttgctctag aaaaaaaaat tgactgcatg aggtggctca 43860

tgcctgtaat cccagcactt tgggaggccg aggcgggcag atcacctgag gtcaggagtt 43920

cgagaccagc ctggccaaca tgccaaaacc tcgtctctac taaaaataca aaattagccg 43980

ggcatggtgg cacacacctg tagttccagc tactcgggag gctgaggcag gagaatcact 44040

tgaaccggga ggcagtggtc acagtgagcc aagattgtgc cagtgtactc cagcctgggc 44100

aacaagagca atactccgtc tcaaaaaaaa aaaagaaaaa aagaaaaaat caacttctaa 44160

ctaaatggca ctgtgatcaa agaacttggt ctgtgtgaca ccagttcttg cagttggttg 44220

agaagtgctc catgacacat tatccacaac cagtggacag aatatgtcac caccaagcat 44280

gggtgcagga ttctacatat attcattacc tctgtcttat ccgtttgttg tgaaagtatt 44340

ttctcttctt actgtcattt tgtttacttg atttatcaac tgatggaata cgtgttaaaa 44400

cctactgtgg tgatggacac atcagtttct ccacagagtt tggtcatttt cattttatta 44460

tttattttga ggccacattt tcagttttag atacagtttc acattcctgg tgtattgaac 44520

cctttggtat tatgaagcga ccatactaat atgttatttt tttttaaatt attatttatt 44580

ttatttattt ttctttcttt tttttttttt tgagacaggg tctccctctg tcacccagtc 44640

tggagtgcag tggtgcaatc tcagctcact gcaacctccg cctcctgggc tcaagtggtc 44700

ctcccacctc agcctcctga gtagctggga ttacaggctt gcaccagcgc gcctggctaa 44760

gtttttgtat ttttagtaga gacagggttt tgccatgttg cccagactgg tctcaaactc 44820

ctgggctcaa gcagttcacc agccttggcc tctaaaagtg ttgggattac aggtgtgagc 44880

caccacgccc ggccatccca gttttttatt ttcctccttt ctgtgtcttt atgcatcagt 44940

gaggtggagc ttttgaaaat gtcagatagc tagatttttt ttttttgaga cggagttttg 45000

ctcttgttgc ccaggctgga gtgcaatggc gtgatctcgg ctcactgcaa cctccacctc 45060

ccaggttcaa aacaattctc ctgccgcagc ctcctgagta gctgggacta caggcacgtg 45120

ccaccacacc cggctaattt tgtattttta gtagagatgt ggtttcacca tgttggccag 45180

gctggtctca aactcctcac ctcagctgat cacccgcctc agcctcccaa agtgttggaa 45240

ttacagacgt gagccaccat gcccagctga tagctagatt tttttttttt tttttttttt 45300

gagacggagt cttgctctgt cgcccaggct ggagtgcggt ggcgcgatct cggctcactg 45360

caagctccgc cccccgggtt catgccattc tcctgcttca gcctccagag tagctgggac 45420

tacaggtgcc cgccaccacg cgtggctgat tttttttttt tttttttttt tgtattttta 45480

gtagagacgg ggtttcaccg tgtcagccag gatggtctcg atctcctgac ctcgtgatcc 45540

gcccgcctca gcctcccaaa gtgctgggat tacaggcgtg agccaccgca cccggcctcg 45600

agatttttta aaaaatccat tgtctgtttc gtcctttaac atctatttga tttctgatgt 45660

atttagattc atttatagca tatttgtgca ttctattttt ccatattttc tgttctttta 45720

aattttcctt ctcctttctt gccttctctt agacttttaa aaaaattgta gtaaaataca 45780

catcacctaa aatttaccat ttttaaatgt acagttcagt ggcattaaat acaaattgga 45840

ggccaggcat ggtggctcat gcctggaatc ccagcacttt gagaggctga ggtgggagga 45900

ttgcttgaac ctggaggtcg aggttgcagt gaggtgtgat cacactactg ctatagccca 45960

ggcaacagag tgagaaccta tctaaaaaaa caaacaaaca aacaaacaaa cagattggca 46020

tcacacagtg tgtgtgctgc tgtgctgtgc tgttatcatt taacattaaa acataaacat 46080

cagcactgtt atggactgaa tgtttgtgtc cccccaaatt ccagtgttga agctcaaacc 46140

tccaatgtga tgttatttgg agatggggta tttgagaggt ggttaggttt agatgaaatc 46200

aggagggagg gtgggtttcg catgatgaga ttagtgtcct tataacaaga gacagcagag 46260

agcttgcttt ctccaccatg tgagtatgca atgagaaggc agccatctcc aagtcagaaa 46320

gagagccctc accaggggct gaatgtgctg acaccttgat cttggacttc tagcctccag 46380

aactgtgaga aatttcagtt gaataggccc cccagtctgt actattttgt tatagcttga 46440

gcagactaat acacccatgt tatgaaaagc tataaacatc cctttaaagg ccacctaacg 46500

cgtcgtatta cctcatgcat cttctctgct gagctttcca aaagagtggc ttaggctggg 46560

cctggtggct catgcctgta atcccagcac tttgggaggc caaggcaggt ggatcacctg 46620

aggtcaggag ttcaagacca gcctgaccaa catggagaaa tcccgtccct actaaaaata 46680

caaaattagc tgggcgtggt ggcgcatgcc tgtaatccca gctacttggg aggctgagcc 46740

aggagaatca cttgaacctg ggagatggag ggtgcagtga gccgagatcg tgccattgca 46800

ttccagcccg gccaacaaga gcgaaactcc atctcaaaaa aaaagagtgg ctggccgggc 46860

gcagtggctc acgcctgtaa tccccgcact gtgggaggcc gaggtgggtg gatcacgagg 46920

tcaggagttc aaaaccagcc tggccgagat ggtgaaaccc cgtctctact aaaaatagaa 46980

aaactagcca ggcgcagtgg caggcgcctg taatcccagc tacttgggtg gctgaggcag 47040

gagaatcgct tgaacctggg gggcggaggt tgcagtgagc caagatggtg ccactgcact 47100

ccagcctggg tgacacagtg agaccccgtc tcaaaaaaaa aaaaaaaaaa aaaaaaagtg 47160

gcgtatgttc ccggtctcca cctctcattc gccagcccct gtgaccaaac ccctgggatt 47220

agatctccat cgggtccctc ccctcaccct aacctcacat gcagtggttc ctgagaagct 47280

gctgtttatc tgatcacggg atccagcagg atctgagggc tcattcactg aatctgtact 47340

acccaacaag gcaggcttgt ccttacccac acttttcagg tgaaaactta atcagattca 47400

agcgtttatc ttagtggcct cttcgtggtg tgtgatttcc ttcttgcagc tctttactgg 47460

ctaaaactct tcactgggag ttaaaccagg tgtggtcctt gaccctcatc attttgtccc 47520

atctcctatg ctgggctctg tatccttgtc tccaagcagt ctcgtgtgac agggaagtta 47580

cctattattg tatggtcccc tcttcttgtc acctctggtg tggcacttgt tccatttctt 47640

gttcaatgaa tgtgatttgt gaatgtgcca catctgtggg aggaaggcag ttcgcagcaa 47700

gagttgtagt tcccttgctt tgccctgagg gccaggactt tacactagat ttttttgttg 47760

cattcctgca ctcataaagt acacgtaaac ttagaggtgg tgtgttaaat gctagaactt 47820

aaaactgagt tcagctaggc tcaagtgccc cacaattttt gtcccaccag acaatttcat 47880

gaatatctac agattgttcc ctcaatatgc ctcaatattg aggcatattg atttggggag 47940

cacagacaag tcaaggttga tgtgcatccg ctgaacatga tcttaagttg ttgaagtagg 48000

tattattaat gaacgaacgt ctattttagc tccaagagcc cctggtcttc cagaagagac 48060

tcccaatgat cttccagaag cagctgggct tcttttggtc ctcccccacc caggtgcagt 48120

cccgttctcc ctgcctgggt gagtttgatt cctctgccgt ggaataggct aggctgctac 48180

ctcttgagcc tcttccacca ggccatccat taggtggcga cagagagcac taaaaggaac 48240

tactatggaa ataaaacttg ttttgcttct tggggggaaa aaaaaagaaa aaggcttgtg 48300

gggcgtgtgt gcattttagt cagattttac tgtgcaaaac atttgagaga tttctgccct 48360

ctttctccct tccattcttc tcaacccact gggcgcccta ccacccctgt ctccttcaag 48420

ataaggtaga tcagaagacc aaatagacaa atgccatgtc cactgttttc tgtcacagtt 48480

gatagccata ccagtcaccc aagctggaaa cccaacaggc cctgcccctt cactgcccac 48540

atccaaggga gccaccaagt cccacaaatt gttcctttaa ctgtttgtcc ctcttttgtc 48600

ttctgtcatc ccttctaccg ctgtcctggt tcaatccctc atcttgctct gggactccca 48660

tgataactta tgatctttcc tccacctctc tatcttccac acaatcccta tcatcaatct 48720

cattcccctg ctttaaaccc aagagtggca ccctgtgttt gagtgacatc agggcccctc 48780

atgtggctcc tgcttctctt tgtggcccct tctgttgctg ctgcttttct tatggacata 48840

gccctcagca gtccaggact gcttgtagtt cctcccaggt accatgtttc ttgcactcct 48900

ggcctttgtc attggctccc ctgcccaagt ccccttctct tctcctttcc ttcccctgtg 48960

gccttgcctt gtcgtgctta tccttgaaga ccctgtcagg tatctcttcc ccaaagagcc 49020

ctgcctaacc accgcccccc tcccccccgc gacacacaca cctgggttag gccctgcctc 49080

tccacgcctg tgcctcctgc caagtcctca gaggtctctg atcccatgtg cctccaggca 49140

tggcataggg ggactgtgac acagcctctc ggcccacaga cccctgattg tgggcttgct 49200

cactgcccca gtcagtcctc ttattgagga ttccgtgcca gttcctgact ccagcacaca 49260

cacgcatgta ggccgccctc ctagtgcctg gctccttccc tgtctcttct gccactcatt 49320

cccatggtcg tatcctggag ttggtcatct ctggaactcc acccacggag ttctaactta 49380

gctctgccta tgatgaggac ttactgtctt cccagcttgc agtgtcaacc actgtttgat 49440

gcccctggga ctccattggt cttccctcct cttgtgaact gtgcccatca ccagacgaac 49500

ttgttttaga atttaccatc gtaactgagc tcccttcagc gcatacctcc ttggctcctg 49560

ctcagtctct gtttctcctt ttagccaaac tctctaagag ttctctccac tcatgggccc 49620

tcttccttgc ctgctgctcc ttgctcagct tgctccatga acacttgctt ttccacattc 49680

catgaaataa tcttaacagt catcagtggc tgtcactcct tctcagccct ttccttgtgt 49740

ctcttctggt acctcatact ctccaggtgg ccatctcacc tcacatcttg aaccctctgg 49800

gtttgggaga attctcacct cacgagttga tgggaggcct ggcgacctct gattgtagtc 49860

actgcaaatg ccagatgctt atttgcctag cttccctttc ggtcagggca gcagttggca 49920

aacggtatct caagggccaa gtccggccca ctgcctgttt tcgtaaatga agctttacca 49980

ggacccagtt ccaccccgtc ctttgtgcat tgtccaagac tgctctcctg tacaacagca 50040

ggtgggctgg ttgtgacaga tactgtctgg cccacaaggt ggaaaatact atctggcccc 50100

ttacagaaag aaattacctg agttctacac gagggtgtag gtatgtaccc tggcctctgc 50160

cagtcagact cacatgctag gaggagcagg gccaggcagg agagccgtgt ctcctggcat 50220

tggggctgca ggaaagacga gctcctaggc cacagtgcca gtggccagtg ctgctgcctt 50280

gcagggtcgc acaggcagtg ctggctgttt ggactggctt ggcagaagga tttgaagtat 50340

tgttcctagc caactggact caaacctgtt cctccaaccc tctagggatt ctcttatcac 50400

ccagtagcct tttcccccta gttttaaaaa ttgcagtaaa atacacaaaa gataaaatgt 50460

actgccttaa ccatttttaa gtgtacaatt ctgtggtatt gagcacattt atattgtgca 50520

accatcacca catgcatctt caatagcttt tcatcagtcc ttgtctgcat aaatcagcta 50580

gagttggttt ctattgattg ccaccaataa tcttgacaga tatgtctgat cttccttctc 50640

tgcactgact cctctttctc tgtctcctct gctgattctt tctcctcatc acaactccaa 50700

atagcagggg gagggttgtt tacacttata ttcagcacgc cctcacgcac tcgcctggct 50760

cttcagacct ctcccctgaa ctccagattc atacatccag ctgcccttta ggcctctaga 50820

aagctaaata cctcagtatc aaaaacatga gaaacggaag ccagagtcct ggatctctcg 50880

ccctttcgct gtcccggtgc ccatgccctt cagggatggc acctagacca gctccctgca 50940

tccctgccta gactctgcag tagtctcacg tggcagaccc ggaggtcact ctcctctctg 51000

gatgccctgc ggacactccg gttagcctca gctgcaagag cctcctcacc caaggtcacg 51060

ccatttccag gacagcaccc tggtgactga gcgaggtaag ggtacaaggc ccttataggg 51120

ccttctcttt gacttcttcc cacctccctt tatggatgtc agttcctgat gaacatctta 51180

cacctcaaac cgtgacccaa catctgcttc tgcagagccc ccctgtggca tcttgtgccc 51240

tcctgcccca ctccctctac tctatatctt cccatagtta gaatgggctt tttttttttt 51300

tttttgagat aaggtctccc tttgtcaccc aggctggaat gcaatggcac agtcacgact 51360

cactgcagcc tcaacctcct gggctcaagc agtcctccca cctcagcctc ccaagtagct 51420

gagactacag gtatgcacca ccacacctgg ctaattttta tattttttgt agagactggg 51480

gtctccctat gttgcccggg ctggtctcga actcccaggc tcaagcaatc ctcccgcctt 51540

ggcctcccaa aatgcaggat tacaggcgtg taccagtgtg cccagcctca gatgggctct 51600

ttgaaaaatg tttattagat caggttactc ccttgagaaa aaccctctca gaataaaacc 51660

cgaagtccat tacttgaaag cagagccagc ttcatgggcc tttgtaactg cccatgggct 51720

tgctggaacc caggcttggt ttaatactct actctcacca tctcaaaatt cttaattttt 51780

gaacaagagg ccctgcattt tcattttgca ccaggctcca caaattgtgt aactgggcct 51840

gcctcagcag ccacatggaa catgatccct ccttgcctat ttgagctcat tctctaccat 51900

tctctccctt gctcacctgg ctcccactgc tcttgctctt cctggaactt gccataatgt 51960

tgctagagtc agagccttgg ctcttgctgc tcctcattgg ctggacccct ctttacttga 52020

cctgctccct caccactcac ttccttcaga tctgtgttca gatttcgtct tctcagagag 52080

gcttttggcc cctgtccatc tctctgaatt tacctctgac ctctccccca ccaccactgc 52140

gctaagctgc ctcagaactt tgtagacatt ctgtctggtc ttctgatgtt tcccccttgg 52200

aagaatccca aggtgcctga agaatgcttt ctttatcctt gaagttgagt aggttgacta 52260

gagtctggct tgctattgag cattctttat caaattgtcc tgggaacatg gtgtattctt 52320

tcagtctgca gatgtagtcg ttgctgtttc aggtcagctc tcttatggat ctttgacagc 52380

atcttctgtt ccatttgttg agttctgtac ttcagggaca caaattcctc atgttggatt 52440

gtctttgtct ctcttccaat gctattagct ttgccgtaat tggtttagct tttgtctttt 52500

tcatctgcat tcactttgtc taatttgatt ttcagctatg tatattctgt ttctggctgt 52560

ttttcaatgt atttattagt ttcataatga tgtgttttgg tctgcagttt gtttctctag 52620

gttggaaatt tgtcttttca tcttattctg ttttatcatc ccatctttga actcttttat 52680

tgggaacatg ttcttatgaa gttgtgggga atttttttcc cttccttgtg tattctcttc 52740

ttggtgggag actttgcctt tctcgtgcca tctccctccc tgggcctctt ttttttcttc 52800

tggccataat atgtttgcct agttaccatg tcacttcttt tcgtcttggc tcaggcttgg 52860

atggctctgc atagtcgttc tgtttgcttt gagacagtgg aggaattctt ggctctctct 52920

tcccagtttc ttggcatctt ctcttgctgt tttcccctct gagctatcgc gtgcaggctt 52980

gttatcttgt atccggagag aatttgcacg ctggagggag ctgcagccat gtagtcttca 53040

gccctatctg gattcttctc tttgttctaa gaactgtgtt ggatgtctta ctaaggctca 53100

ctctagctgc acgagggatg tgtgtgccat ttccatgggg atagggggca cctcagtctc 53160

tgggtggttc cataatctgt gtatacctaa gagcagttgc ttcccacaga gctgggctgg 53220

ctcactgggc actttgccat ttctcctgca cctcccagct ggagtttctg ggtctaaaag 53280

gaaaaagtga aggactccca cttggttgct tctctccagc ttactgactg caaattccca 53340

gggcgttgcc cgctccctag ggtggtttct gggggacggg gcaggagcct ggctttgctg 53400

ctgctttgtc ctctggagtc ttttctcaga ctgctttgaa ttacacccct ttcctttgtg 53460

tgctgaaatc ttcccttcac actctccttc cctgcctttc ttttgtgtct tatcttacta 53520

ggactggaag agggcagttg tgcaggaggt ctgcatctaa ttccctaatc catgtgagag 53580

tgctcctgtt gtgtgctttg tgacatgatg gtgagaaata ctccctagag cagtggctac 53640

cagaaggaag ccatcccccc ccctgcacac acacacacac acacacacac acacacacac 53700

acacacacac actccttctg gctgtcctca gatgcctatt ctaggtaaat gtcagattcc 53760

aaagaaaatc cagttgagat cttttttctt tttttgagac gagagtcttg ctgtgttgcc 53820

caggctggag tgcagtggca caatctcggc tcactgtaac ctccgcctcc tgggttcaag 53880

cgattctcct gcctcagcct cccaagtagc tgggactaca ggcgcgcacc accacaccag 53940

gctaaaattt ttttgtattt ttagtagaga cagggtttca ctgtgttggc caggctggtc 54000

tcgaactcct gacctcgtgt tctgcccgcc tcagcctccc agagtgctgg gattacaggc 54060

ttgagccatc gcgcccggcc tccagttggg atcttgactg gaatgactgg tgttcaatca 54120

ttatagtttc cacctaattt gtatttgtac acaggacagt tactaatttg ttggtacttg 54180

tttgatcccc agtccctaga gttgtttatg gggtggagct tcagtccctg ctgcttcccc 54240

tgtggcagca gctggagtca gggtggggac ccagggtgct gctggcagat tcttgagaca 54300

ggtagaatct cctctatatt ggtgtctctc tctgtcccag cagtgcccag gaaaacctgg 54360

ccagcctgtc actgacctct ccacctaggg cactggtggt tcaggtcctt ctatttgcca 54420

ccggcaaacc gtacttctgc cagtctggct cttaggccca gtttctctga tcttgcagat 54480

tttcttgggc tctgctacgg aatcctcata cctccggcag gtccctcctt tgcccatgtg 54540

tttaactgtg gtggaatcgt gtgagagctg cttctctcgc atggatccca gccacaccac 54600

attctacagc ggttcctctg aaggcattga tagagatatt tcctcctgtt ttgcatttcg 54660

ttggtcattt cagtagaatc agggtgaaat aaacatgggg gctcagatgt cagcattacg 54720

aaccaagtac gtcaggcagg ctgatgtgga ctgacctaca ctagtgagac gcaagatgac 54780

gaaaacaagg gcactcactc caagttactg atgagatgtt tggatcaaat gagccagtcc 54840

ttaagcagag ttctctagta aaagagatct cctttctgcc ctttcttgtt ccccaaaatg 54900

tgttgccttc atggtgaaaa tttattttgg cagattttct cttctttgat aaaagcagcc 54960

aacactttgt taaaagtctg tgaaacttat ttacatgaag tatgtaaagg taagaaaaaa 55020

acattatcaa caagaaatgg agaaagccag cagctgagga cagaaaagtc atgcacagtg 55080

tcagtgtcta tggaaacagg ccacttggac cttgaggact aggtatttgg aattggaggt 55140

gagcttggcc tggtgagtct ctaaccactt gtgtgtagga tcagtgtgag aaccctgcta 55200

gaatatagtg gcagagatgc aaggggaaat cattggagaa gttaccaggg aatgatgagc 55260

taatctgaaa aaaatacatg tttctaagtt gggcgtggta gctcatctgt agtcccagct 55320

acttgagagc ctgaggcagg aggatcgctt gagcccagcc tgggcagcac agcgagaccc 55380

tatctcccta aaaaaaactt tttcgttgtt tagttttggg attttttttt ttcctggtct 55440

tttttccccc tttttgtgaa taacgggatc tcactatgtt gcccaggcag atctcgaact 55500

cctgggccca agcaatcctc ctgcctctgc cttcctaaga ttacaggtat gagccactgt 55560

gttaagcaaa aaaacttttt taaatgaaaa tcatttttta aaagacaggc tttccagggg 55620

agggtattat tccacttata tgaagtgtca acagtaggca gatttgtgga gacaaataga 55680

ttagtggtta ccaggggctg agaggagtgg gagtggggag caactgctta atgggtaaag 55740

ggttgtcttt ggaactagag agtagtgatg gtcgcatgac attgtgaatg tactaaatgc 55800

tattaatgat aaattttatg ttatgtgtat tttaccacaa ttaaaaaaaa aagatcaaat 55860

gtcctcagaa tagccaacaa ccttccactt ggctaaatgc ctactcatta aacttcttga 55920

actaaattcc tttctgattg tcatggttat tgtgtcctgg gcttcagagt ttcacattca 55980

ggttggcttg gtccagtctg tcatgtatca ctataggtcc ccacattggc ctcttcctca 56040

gacggacagc ccatctatct gccggggctc tgtgccacag ccagatagac ttgctctgag 56100

acagctgtgt gggctctgag cactggccag gcatcacaaa acctatcttt atgatttaga 56160

ataattggtg gtcagctgct gttttaatgt tgttgttttt tttaatttag atataattca 56220

cataccatga aatttactca tttaaagtgt acaattcatt cttcagtata attcataggc 56280

tcacagaaaa aattgtttaa aaataaaatg tgcaattcag tgtcttttag tacattcaca 56340

gagttgtgca accatcgcct ctgtgtcatt ccagaacact ttcagcaccc aaaagaaacc 56400

ccagacacag gagcagtcac ctcttattac ccgcagcccc tggcaacaac tcatccactt 56460

cctgtctcta tggatttgcc tattctggac atttcctata aatggaatta tgcactattt 56520

ggccttttgt gtctggcctc tttcactgag cgtaatgtcc tcaaggttca tctgcattgt 56580

agcatgtgtc agaatttctt tcctttttga ggctgaatga tattatatcc tatagataat 56640

gaggttttga ttatccaccc atcccttggg aatgcatatt tgggttgccc ccaccatttg 56700

gctgttgtaa actgtgctgc catgaacact ggtgtacgga tatctgtttg gttactggtt 56760

ttggtttttt gtttgtttgt tttggttttt tgagacaagg tcttgctctg tcgcccaggc 56820

tggattacag tggcacgatc tctgctcact gcaacctcca cctcccaggt tcaagcaatt 56880

ctcctgcctc agtctcctga gtagctggga ctacaggtag cactcaccac catgcccggc 56940

taaatttttt tgtattttta gtagagacaa ggtttcgcca tattggccag gctggtctca 57000

aactcctgac ctcaggtgat ccacccacct cagcctccca aagtgctggg attacagacg 57060

tgagccaccg cacccggcat agttgtggct ttttgagagt gtatggctag gagtaaaatt 57120

gccaggtcat atggtaactc catgtttaac atttgagaaa ctgccaaact gttctccaca 57180

gcaggaattt tttaacctgt atgtggtggg cttgtgtttc ggttttcatt ttacacatct 57240

ataaagatga gatttgctgt atggcactgg ttgcctgtat ttggggaggg ttctgctttt 57300

ggttggcaag aactgcattt tatttaagct tagcaaaaca taactggttt ctcgcatctt 57360

ctcaaaagtg gaggattaag aaatggactg cgaattcaga gcagggcagc tgaacctcag 57420

gctccaccct tgtagccttc aagctgaacc tcattctctc ttgctccctg gagaccactg 57480

agacactctg cctgtgccag tttgatttct cacattttta aagggccaaa gcttgtgtct 57540

caaagtgcta tagcctttat tgattcatgc agagaagcct ccttgattcc gtaattctgc 57600

agctaatact ggaagtagaa agaattggaa acaccatctg gatgacactt tagggtggaa 57660

gcagccagta caaggggggg ctcattattt cctctggtcc cagactgttc acctggagct 57720

gtagccacca ccctgccctt aggttaactg cctcgagtgg tagtttagct ctttgtgctg 57780

tgccgaggga taactggaag tgaaaggtgc tgagaaatgc catctcctga aagtggcgag 57840

catgagtgaa tttacgaaag gttgggatat tgctggggct ctggaagttt ctctggagct 57900

cactccaggg gacaggaggg ggctggattt ccaattcaag tgaaaaatac ctttcatctg 57960

ccttgttcac ctggcttttt tgcctttttg taaaatctga aaacctcagg gattgagtag 58020

tctttcctta actgcagttg cctgtctggc cacacctgcc agctgttgct tgtacccctg 58080

taatttgcac ggccttccgg gcctttctca caagatcact gcaggtcaca ttcatgagga 58140

aaatgcaggc agttcctgcc atcagacccc tcaggatgtc atggtttggc ctgaaaacaa 58200

gattcctgca actctaattt tcctttgcta gatcaaatga aggatttgat ctaatgtttg 58260

cattctagca gcaaaatcat tgaatatttt atttcttaag agccttactt catattttgt 58320

aggtatttta agattttgta aaggcctttc tgcttcaacg tgtgatgtgt gcattcttag 58380

aaaaagatct tgtgttctgt aaatcacaca ataaaaacat gagttcgtgc aggaaaaact 58440

ggggcggggt ggatcactcc aaacttgtgt ggtgtggtaa ctggagctca ctgatgaaac 58500

catgaacagt tctggctgaa agaaccccac agtacactga ggtctgttgg catcgccgcc 58560

agcaccgccc cggtcccttt gtgcgcgcca ccacacctgg ctaatttttt atttttagta 58620

gagagggggt ttcaccatgt tggtcaggat ggcctcgaac tgacctcgtg atccgcccac 58680

ctcagcctcc caaagtgctg ggattacagg tgtgagcctc cgtgcccagc cttcagcttt 58740

ttttcttaat gtctttgtgt aatatgaagg cattctcttt aattgttaaa aagcttgcct 58800

accactgctt caaaatatta ctgtcagttg gcatagcttc tgattatatt gatgtcatct 58860

cccttctttt aaacatgtta ccatattggc actgtatttc ccctagccca ttgatcactt 58920

gagagttagt catagtcctc gtgctgtttc actcctaaac gtttaagcat gcctttccta 58980

agagcagaca gtacagttaa gacactcagg aagtttagca atgagctaac acagaacctt 59040

acatttctcc acatacccca taaatgcctt ttttagagcc tttgagcctg gaatgcagag 59100

tccaggactg tgtgttgtat tcgattgtgt catcccttca gtctccttta tcagaaatgt 59160

tcccccaccc ccttgttttc tctcatcatc ttgagtccag accgtggttg catggcacgc 59220

cctctctgga ctcttcctgc tgtctcctca cggtaagctt cagtctccta ctcgtgatgc 59280

tggttcatct cagcagcact gaggggcctg agctcagttt ggtcgtgtgt taaggtggtg 59340

cctgccagat ttctccacag aaaagggccc cgtactttta tttgctcttc agcctgtgta 59400

ttccttcctt tcttgccagc ttgtgggtgg cttctctagc agcttctgta agatactcag 59460

tttggcagtt gtagttgttt cagcaggaga ggtgtctgca tacctgacca ccacatggct 59520

agaagtcgat ccatcctctg gcgtaaccat cctccatgct actgtccctg gggcactgag 59580

gccctcctca gtgacttcct ccacctcatc ttccgccatc tccaagccac cagtgtccaa 59640

cggactactg atatgtccca aactcatcat ttgtttattc accaattcag agcccacctt 59700

tttttcagca ctgagctagc ctctccttgc tagaagctta cggtcgaagg tctccagcca 59760

tcagaagaag cacgtggagc gctgcgtccg tgttgtggtt attcatccag catgtgttga 59820

gtaagggttg cacctgtgcc tggcattatg cattgagcgg ggagatgggg gttggcacgc 59880

acagtggggt gttctaagta cactgagggc tcgggtgccc tggctcatag agcagggagg 59940

gaggcaggag cagggaaggt gtctcagaag tgccatcttt tttttttttt ttttgggaag 60000

tggaatcttg ttctatcgcc tggtctggag tgcaatggca ctatctcaac tcactgcagc 60060

ctccgcctcc caggttcaag cgattctcat gcctcagtcc ccgagtagct gggaccacag 60120

gcgcacacca ccacacccgg caaatatttt gtatttttag tagaggcgga gttttgccat 60180

gttggccagg ctggtcttaa actcctggcc tcaagtgatc tgctcacctc ggcctcctaa 60240

ggtgttggga ttacaggcat gagccactgc gcccggtcta gaagtgccat cctaactgaa 60300

cctgaaagat gaaagttctc cagatgaacc tgaaagttct ccaaatgaaa aggtgggagg 60360

gggtgacagg gttaggccca gagcctctgg tgacacaggg tgggcatcat tggtcacttt 60420

ttcctcgagg gaggggcgtc acacgggtga tagggtggga gctataacca tgttgatagt 60480

gccgcctctg cccatctggc ctggcatgcc ctgagccctc tgtcccacct gtggaactca 60540

taagccctga cagcccactc actcctgatt cattatccac acccctgtgc ttccgctgtg 60600

cctggagcaa gctttcttca gggggaaggg aggctggaac tatgttgtag ttacctattt 60660

gtcctccctt ccaaactgtg agttcttgga ggtggaagga tgctgcagga tctggctcag 60720

gacgaaggca gttggtgaac agacacgtgt gtttttgact cacggtgatc tcagacaagt 60780

tcctctgtct agtcgaactt ctttttttcc atctgtaaca ctcaggagtt gaataggtgg 60840

tttttctgag gatacttcaa ctgtaaaatg tatgaacttg tgaactagct atttagttct 60900

cctcataatc aagattgtgt gtgtgtgggg ggttctgatt agagggagga tgaagagagg 60960

tgtatggggt tttttttgtt ttgttttctg tttgtttgtt tgtttttgag atggagtctc 61020

actctgtcac ccaggctgga gtgcagtggc acgatttcgg ttcactgcaa cctctgcctc 61080

ctgggttcaa gtgattctcc tgcctcagcc tcctgagtaa ctgggattac aggcatgcac 61140

caccatgccc agctaatttt tgtattttta atagagatgg ggttttcgcc atgttggcca 61200

ggctggtctc gaactcctga cctcaggtga tccacctgcc tcggcctccc aaagtgctgg 61260

gattataggc atgagccacc acacctggca ggtttctttg aaaaagtttg tgtttcggca 61320

aacaccataa accccctggg ggacagcctt ggggagtcac ctggcaccct agcccagcct 61380

ccctcccttg ggtcctgcag tgaaggctta gtgagggtgt gcaaatgccc aggtcaccct 61440

gggactgggc aggccctctg ggctaagggt aaactcattt ggaatacctg ttttctatca 61500

ttgtttttta tttgttaaat ttaaagggta caagtgcagt tttgttgcgt ggatatattg 61560

tatagtagtg aagtctgagc tttcagtgta accatcacct gaatagtgga cattgtaccc 61620

gttaagtaat gtctcatccc tcaccccctc ccacccttcc cagtctctcc agtgtctgcc 61680

attcctcact ctgtccatgt gcacatgcta ttcagctcct gcttctaagt gagaacgtac 61740

ggtatttgac tttctgtgtc tgagctgtgt cactgaagac aatggactcc agctccatcc 61800

acgtttttta tcatttttac ctgcactcca cacccagcac aatccaggct tctttgtggg 61860

ttttttgaaa tttgtcttta attataaaag tagcagccag caaattaaca aacacccatg 61920

tgcctttcat tgcacagaat tgaaaatcat catactatat ttgcttcaag taatttccat 61980

tagaaagaac tagaatatta cagtagagtt aaagaccctt tatttcccat cttcagtgct 62040

cttaaaagtt catttagggc caggcatggt ggctcacacc tataatccca gcactttgag 62100

aggcccaggt gggtggatca cctgaggtca ggagttcaag accagcctgg ccaacatggc 62160

gaaaccccat ctctactaaa aatacaaaaa ttagctgggc gtggtggtgc gcatctgtaa 62220

tctcagctac tcgggaggct gaggcaggag aatcgcttga actcaggagg gaggcagagc 62280

ctgtatgcag taagccgaga ttgcgccacc acaccccagc atgggtgaca gagcaagact 62340

ccgtctcaaa aaaaaaaaaa aaaaagttca ttcattgtac acttagaaat agttaaaaag 62400

gtaaactttg ttttgtgtgt gtgtgtatat atatatatat atatatatat atatatatat 62460

atatatatgt attttttttt gagacatata tgtgtctggt ttgttcgccc aggcaggagt 62520

gcagtggcat gatcaacggc tcactgcagc ctcaacttcc taggctcaag tgatcctcct 62580

gcctcagcct cccgagtagc taggattaca ggcacacacc accatgccca gctaattttt 62640

tttttttttt tttttttttg tagagacagg gttttgctat gttgcccagg ctggtctcaa 62700

actcctgagc tcaagcgatc cacctgcctc acctcccaaa gtgctgggat tacaagtgta 62760

agccaccaca cctgacctgt tttgtatatt ttaccacaat aaaaagcctt taaaacccca 62820

agcagacagt tcattttcat tcaggcccaa ctcagaatct gatcacagcg gggtttcccc 62880

cctttctagc gagtagctga agaactgttt tctctccttg atggtataac tgtctctgtg 62940

ggtgttgctc ccctgccgct ccagtggttt tgtttttgtt tttgtttttt tgagacggag 63000

tcttgcgatc tcagctcact gcgacctctg cctcctgggt tcaggcgatt cttctccctc 63060

agccttccat gtagctggga cttacaggca cctgccacca cgcccggctc atttttgtat 63120

ttttagtaga gacggagttt caccatgttg gccaggttgg gctcgaactc cagacctcag 63180

gtgatccacc tgcctcagcc tcccacagtg ctgggattac aggtgagagc cactgcaccc 63240

agccggcccc tcagtctttt ccttctcaat cagtggcacc accatcttcc caggctttgg 63300

acatggtccc tgactcaccc ttgcccctca cccccacact aatccacctg cgagctctgt 63360

tgcttcacca cctagaccag ccccaaatcc tcaactgccc ccaccctggg ccacacctgg 63420

accactgcta gaggcctctc atgggccctc cctgtttttc tcttgcactc cccgggcttt 63480

ctggcacaag atgccccaga agcagaatca catatctctc ctgggagcca atctagtgtg 63540

tttactgccc ctggagtgtt attgttggcc ttagaatatg tcccactaca ggtttgcaga 63600

gcactgtagt caaaagtcat ttgaaataaa tcttttctct gtggtatatt gtcaatttga 63660

tatagaatta aatttgtttc ttttcttttt tcttttcttt tttttttttt ttaagagaca 63720

gggtcttggc caggcgtggt gtctcatgcc tgtaatcccg gcactttggg aagccaaggt 63780

gggtggatcg cctgaggtca ggaattcaag accaacctgg ccagcatggt gaaaccccgt 63840

ctctgctaaa aatacaaaaa ttagccgggc gtgatggcag gtacctgtaa tcccagctac 63900

tcgggaagct gaggcaggag aaggcttgaa ctcaggagtc ggaggttgca gtgagccaag 63960

atcacgccat ggcactctag caagactctg tctcaaaaaa aaaaaaagag agagagagag 64020

acagggtctt gctctgtcac tcaggctgga gtgcagtgat gtagtcatgg ctcactgcag 64080

cctcatactc ctgacctcag gtgatcgacc cgcctcggcc tcccaaagtg ctgggatcgt 64140

aggctgaagc caccatgcct ggcccgaact catttgtttt tatttgcatt aagtgtaata 64200

aggttttgtt acttttagtt tgaattttat tttgggtaat ataaacattt acatgattca 64260

gaagtcagaa ttacactgag gcatgttcag ctaggcctca ctcctgtgcc tgtaccctta 64320

cctttttccc cctaccccat gcagggaatc aatttcatta gttcctggtg tgtccttcct 64380

taataccccc ccttctttct ttggtagaat gcagtagata tgtttgcgct ttgctccctt 64440

tgcttcacag tggatcctgg aaatgactcc atcgcagttc ttagagctct tgtttagtcc 64500

ctttggatct gcacagtact ccagtgtgtg ggcgcaccat aagtttattc agcaagtgcc 64560

ctggtgatag ggatcggggg tattggaagc ctttggctgg taaaaataat gttgtagcaa 64620

ataacatcat gcatatgttc tttgagattt ctggaggtgt ctctttagca tagatttcta 64680

gaaggcattc cttacgtcac gtttaggttt atagtttgac ctcatggtga ttaaggtatt 64740

tgggaaatgc aaatgagagt ttcgaagaag ccagatcttt cccattagtt cactgttttt 64800

ctctgacatt aggaccgtcc gggttctagg gagcagccct ggcgttaggc agtgccatga 64860

tggattgtgt agaagtagcg attcccatct gtctgctttc ttggcccact ctgctggtgg 64920

ctccccttcc ctccctcctt tatggggagc tggggagctg cctaggggtc cattctcaaa 64980

ggctgatctc tggtgggcaa caggccacac ctagctttcc agggttcttc gtcatttccc 65040

attgagagct gtaagactca gagacatgaa aaggaagctc tggctgggca cggtggctca 65100

cgcctgtaat cccagcactt tgggaggctg aggcgggtcg atcacctgag gtcaggagtt 65160

cgagaccagc ctgcccaaca tggtgaaacc ccgtctccac taaacataca aaaattagcc 65220

tggcgtggta gcacacgcct gtgatcccag ctactccgga ggctgaggca ggagaataac 65280

ttgaacccgg caggcggagg ttgcgatgag ccgatattat gccatcggca acagagcgag 65340

actccatctc aaaaaaaaaa caaaaaacaa aacaaggcga gctctgtgct gggacagatt 65400

agggacccct ctttacagca agaaagactg ctctgtgggt tgtaggatgc ctttgtgtat 65460

gcagtggctc taggtgactc tggcagccac actctgggcc ctaaacttct ggaggaagat 65520

acaggatagg gaggaactca ggggtgagtc atggtgggga caagacattc cctcactcta 65580

agaccttgtc actagattgg aacatctctt gcctccctac acctgacctg atggttctgg 65640

agagatacgt ccttgcagct tctgagtccc agcactaagc agcctttggt aacttcccta 65700

catcatttga gttctggttt cctaaggatg cttgccagtg agtgccatgg tgccctcatt 65760

gcacagtctg tgcagtgtag acaagagggg aagtctcttg ggtagaccca gccgcaaggc 65820

ggtgactagc actgatgtga accacatggg acaggggagt tgtggggctg agaacacgga 65880

gggtgggagt agtcatgctc ttttccagaa tgaactgcta acgaagggac tcgcaggtgg 65940

ctgctgcttc tttccaagct gcccctgttg ttgcagaggc tctggagtcc taggaggttt 66000

cacggtggca tactcgacag agtactagag catcatggcc agatagtgat gctggggtgt 66060

ggggcctcac ggtggccatt tctgacgaga ccccaccggg ccaaagtgat gtgtagagaa 66120

ggagctgctt cggtcaccag aaaagaacgg ggaagcctcc cacactagaa taggtagggt 66180

gctcttctcc accggcagga aggggatgta tggctctgcc tggaccacac ctttttcctt 66240

gctcttccct ccgcatctgc tgtggccgag gccattcctc atcagggaac atgtgttaga 66300

ggctcacgcc acctgggacc acttgtctta tcacccccag gaccctaggc ggtagtttcc 66360

tgtggcctga gttagctgat atttatatag tgccattgtg ccttttctcc tgtgatgctc 66420

acagtaagga tgcctagatg gggttacctg ttgtcaagat aaggaaactg aagcacagaa 66480

tgctgaggtc atttgctggg ttcatgtttg gaaagcggca aaggatttca gtgcaggttg 66540

gctggctcca aacctgtgtg tgctttccat gacactgtac tgtgtgcctc attgagcctc 66600

attctagaaa accaaaaaca cacccaaggc ccggccttca caaaggagac ccctccccca 66660

tttggctccc tttccagcag tcgacggcct cttgtcagcc atcgagccca gagtcccttg 66720

aagtgcgact catgctgggg tggtatgctc aggagccgca gtgtttccgc tcagaggaaa 66780

gggctctgat tctcctgcag tgctaggaga cttgtgggtg gccacagtgc aggtcaggca 66840

caccggccag caccacccac agcccaaatt cctaaagaaa tatttgggtc ccagcttggc 66900

ccgagtctct gttgtcctgg ggaaggacat caagatctga gtgtatgatg gcctggggcc 66960

ttgcatgtgg tgggggtcca agcctgcctc tgctcacttg ttctgcagac tggcatgttc 67020

tctgtgatac ttacatactt gtttaacact tcagggaaga aaagtcagaa gaccaggacc 67080

tccagggcct caaggacaaa cccctcaagt ttaaaaaggt gaagaaagat aagaaagaag 67140

agaaagaggg caagcatgag cccgtgcagc catcagccca ccactctgct gagcccgcag 67200

aggcaggcaa agcagagaca tcagaagggt caggctccgc cccggctgtg ccggaagctt 67260

ctgcctcccc caaacagcgg cgctccatca tccgtgaccg gggacccatg tatgatgacc 67320

ccaccctgcc tgaaggctgg acacggaagc ttaagcaaag gaaatctggc cgctctgctg 67380

ggaagtatga tgtgtatttg atcaagtaag taagagcaac tcctatctct acagggcagg 67440

gagggcaggg acaaggatcc ctcatggagc aggaaaatgt atgtgcccag ggtggggtcg 67500

gggggaacat aaacaatgaa cactgagacc aggtgtgctt gaaatgaccg tgtacagagg 67560

tcgctgccct gagtgggaag ttctcaaggt agcaggccct ctatcctctc cacacctcaa 67620

gtctttatct ggggatggaa tagctgcgga agcagaggaa cttgcagagc taggggttca 67680

gaggggtgaa gaagcatgtt tcagttctgc cttttaaatg atcccaaaaa ggttagcagt 67740

tttcaaatga catttgcaga cagcctcatt taattccatg agaagggtga gcaaaggatt 67800

atcttgttga aactgattcc tggagagact gagcaccgta cctgagttca aacttgggaa 67860

tgttctagat ggtgactcag gcccaggcac caaccagcag aatgggcctc agcctgacaa 67920

cccttctgta ccaggcctga ctctttggtt gctgaacttt ggagaggcct gggggggtca 67980

gcggcaggca gacgagtgag tggctttggt gacaggtcct caggggcagc caggcagtgt 68040

gactctcgtt caatagtaac gtttgtcaga gcgttgtcac caccatccgc tctgccctat 68100

ctctgacatt gctatggaga gcctctaatt gttccttgtg tctttctgtt tgtccccaca 68160

gtccccaggg aaaagccttt cgctctaaag tggagttgat tgcgtacttc gaaaaggtag 68220

gcgacacatc cctggaccct aatgattttg acttcacggt aactgggaga gggagcccct 68280

cccggcgaga gcagaaacca cctaagaagc ccaaatctcc caaagctcca ggaactggca 68340

gaggccgggg acgccccaaa gggagcggca ccacgagacc caaggcggcc acgtcagagg 68400

gtgtgcaggt gaaaagggtc ctggagaaaa gtcctgggaa gctccttgtc aagatgcctt 68460

ttcaaacttc gccagggggc aaggctgagg ggggtggggc caccacatcc acccaggtca 68520

tggtgatcaa acgccccggc aggaagcgaa aagctgaggc cgaccctcag gccattccca 68580

agaaacgggg ccgaaagccg gggagtgtgg tggcagccgc tgccgccgag gccaaaaaga 68640

aagccgtgaa ggagtcttct atccgatctg tgcaggagac cgtactcccc atcaagaagc 68700

gcaagacccg ggagacggtc agcatcgagg tcaaggaagt ggtgaagccc ctgctggtgt 68760

ccaccctcgg tgagaagagc gggaaaggac tgaagacctg taagagccct gggcggaaaa 68820

gcaaggagag cagccccaag gggcgcagca gcagcgcctc ctcacccccc aagaaggagc 68880

accaccacca tcaccaccac tcagagtccc caaaggcccc cgtgccactg ctcccacccc 68940

tgcccccacc tccacctgag cccgagagct ccgaggaccc caccagcccc cctgagcccc 69000

aggacttgag cagcagcgtc tgcaaagagg agaagatgcc cagaggaggc tcactggaga 69060

gcgacggctg ccccaaggag ccagctaaga ctcagcccgc ggttgccacc gccgccacgg 69120

ccgcagaaaa gtacaaacac cgaggggagg gagagcgcaa agacattgtt tcatcctcca 69180

tgccaaggcc aaacagagag gagcctgtgg acagccggac gcccgtgacc gagagagtta 69240

gctgacttta cacggagcgg attgcaaagc aaaccaacaa gaataaaggc agctgttgtc 69300

tcttctcctt atgggtaggg ctctgacaaa gcttcccgat taactgaaat aaaaaatatt 69360

tttttttctt tcagtaaact tagagtttcg tggcttcagg gtgggagtag ttggagcatt 69420

ggggatgttt ttcttaccga caagcacagt caggttgaag acctaaccag ggccagaagt 69480

agctttgcac ttttctaaac taggctcctt caacaaggct tgctgcagat actactgacc 69540

agacaagctg ttgaccaggc acctcccctc ccgcccaaac ctttccccca tgtggtcgtt 69600

agagacagag cgacagagca gttgagagga cactcccgtt ttcggtgcca tcagtgcccc 69660

gtctacagct cccccagctc cccccacctc ccccactccc aaccacgttg ggacagggag 69720

gtgtgaggca ggagagacag ttggattctt tagagaagat ggatatgacc agtggctatg 69780

gcctgtgcga tcccacccgt ggtggctcaa gtctggcccc acaccagccc caatccaaaa 69840

ctggcaagga cgcttcacag gacaggaaag tggcacctgt ctgctccagc tctggcatgg 69900

ctaggagggg ggagtccctt gaactactgg gtgtagactg gcctgaacca caggagagga 69960

tggcccaggg tgaggtggca tggtccattc tcaagggacg tcctccaacg ggtggcgcta 70020

gaggccatgg aggcagtagg acaaggtgca ggcaggctgg cctggggtca ggccgggcag 70080

agcacagcgg ggtgagaggg attcctaatc actcagagca gtctgtgact tagtggacag 70140

gggagggggc aaagggggag gagaagaaaa tgttcttcca gttactttcc aattctcctt 70200

tagggacagc ttagaattat ttgcactatt gagtcttcat gttcccactt caaaacaaac 70260

agatgctctg agagcaaact ggcttgaatt ggtgacattt agtccctcaa gccaccagat 70320

gtgacagtgt tgagaactac ctggatttgt atatatacct gcgcttgttt taaagtgggc 70380

tcagcacata gggttcccac gaagctccga aactctaagt gtttgctgca attttataag 70440

gacttcctga ttggtttctc ttctcccctt ccatttctgc cttttgttca tttcatcctt 70500

tcacttcttt cccttcctcc atcctcctcc ttcctagttc atcccttctc ttccaggcag 70560

ccgcggtgcc caaccacact tgtcggctcc agtccccaga actctgcctg ccctttgtcc 70620

tcctgctgcc agtaccagcc ccaccctgtt ttgagccctg aggaggcctt gggctctgct 70680

gagtccgacc tggcctgtct gtgaagagca agagagcagc aaggtcttgc tctcctaggt 70740

agccccctct tccctggtaa gaaaaagcaa aaggcatttc ccaccctgaa caacgagcct 70800

tttcaccctt ctactctaga gaagtggact ggaggagctg ggcccgattt ggtagttgag 70860

gaaagcacag aggcctcctg tggcctgcca gtcatcgagt ggcccaacag gggctccatg 70920

ccagccgacc ttgacctcac tcagaagtcc agagtctagc gtagtgcagc agggcagtag 70980

cggtaccaat gcagaactcc caagacccga gctgggacca gtacctgggt ccccagccct 71040

tcctctgctc ccccttttcc ctcggagttc ttcttgaatg gcaatgtttt gcttttgctc 71100

gatgcagaca gggggccaga acaccacaca tttcactgtc tgtctggtcc atagctgtgg 71160

tgtaggggct tagaggcatg ggcttgctgt gggtttttaa ttgatcagtt ttcatgtggg 71220

atcccatctt tttaacctct gttcaggaag tccttatcta gctgcatatc ttcatcatat 71280

tggtatatcc ttttctgtgt ttacagagat gtctcttata tctaaatctg tccaactgag 71340

aagtacctta tcaaagtagc aaatgagaca gcagtcttat gcttccagaa acacccacag 71400

gcatgtccca tgtgagctgc tgccatgaac tgtcaagtgt gtgttgtctt gtgtatttca 71460

gttattgtcc ctggcttcct tactatggtg taatcatgaa ggagtgaaac atcatagaaa 71520

ctgtctagca cttccttgcc agtctttagt gatcaggaac catagttgac agttccaatc 71580

agtagcttaa gaaaaaaccg tgtttgtctc ttctggaatg gttagaagtg agggagtttg 71640

ccccgttctg tttgtagagt ctcatagttg gactttctag catatatgtg tccatttcct 71700

tatgctgtaa aagcaagtcc tgcaaccaaa ctcccatcag cccaatccct gatccctgat 71760

cccttccacc tgctctgctg atgacccccc cagcttcact tctgactctt ccccaggaag 71820

ggaagggggg tcagaagaga gggtgagtcc tccagaactc ttcctccaag gacagaaggc 71880

tcctgccccc atagtggcct cgaactcctg gcactaccaa aggacactta tccacgagag 71940

cgcagcatcc gaccaggttg tcactgagaa gatgtttatt ttggtcagtt gggtttttat 72000

gtattatact tagtcaaatg taatgtggct tctggaatca ttgtccagag ctgcttcccc 72060

gtcacctggg cgtcatctgg tcctggtaag aggagtgcgt ggcccaccag gcccccctgt 72120

cacccatgac agttcattca gggccgatgg ggcagtcgtg gttgggaaca cagcatttca 72180

agcgtcactt tatttcattc gggccccacc tgcagctccc tcaaagaggc agttgcccag 72240

cctctttccc ttccagttta ttccagagct gccagtgggg cctgaggctc cttagggttt 72300

tctctctatt tccccctttc ttcctcattc cctcgtcttt cccaaaggca tcacgagtca 72360

gtcgcctttc agcaggcagc cttggcggtt tatcgccctg gcaggcaggg gccctgcagc 72420

tctcatgctg cccctgcctt ggggtcaggt tgacaggagg ttggagggaa agccttaagc 72480

tgcaggattc tcaccagctg tgtccggccc agttttgggg tgtgacctca atttcaattt 72540

tgtctgtact tgaacattat gaagatgggg gcctctttca gtgaatttgt gaacagcaga 72600

attgaccgac agctttccag tacccatggg gctaggtcat taaggccaca tccacagtct 72660

cccccaccct tgttccagtt gttagttact acctcctctc ctgacaatac tgtatgtcgt 72720

cgagctcccc ccaggtctac ccctcccggc cctgcctgct ggtgggcttg tcatagccag 72780

tgggattgcc ggtcttgaca gctcagtgag ctggagatac ttggtcacag ccaggcgcta 72840

gcacagctcc cttctgttga tgctgtattc ccatatcaaa agacacaggg gacacccaga 72900

aacgccacat cccccaatcc atcagtgcca aactagccaa cggccccagc ttctcagctc 72960

gctggatggc ggaagctgct actcgtgagc gccagtgcgg gtgcagacaa tcttctgttg 73020

ggtggcatca ttccaggccc gaagcatgaa cagtgcacct gggacaggga gcagccccaa 73080

attgtcacct gcttctctgc ccagcttttc attgctgtga cagtgatggc gaaagagggt 73140

aataaccaga cacaaactgc caagttgggt ggagaaagga gtttctttag ctgacagaat 73200

ctctgaattt taaatcactt agtaagcggc tcaagcccag gagggagcag agggatacga 73260

gcggagtccc ctgcgcggga ccatctggaa ttggtttagc ccaagtggag cctgacagcc 73320

agaactctgt gtcccccgtc taaccacagc tccttttcca gagcattcca gtcaggctct 73380

ctgggctgac tgggccaggg gaggttacag gtaccagttc tttaagaaga tctttgggca 73440

tatacatttt tagcctgtgt cattgcccca aatggattcc tgtttcaagt tcacacctgc 73500

agattctagg acctgtgtcc tagacttcag ggagtcagct gtttctagag ttcctaccat 73560

ggagtgggtc tggaggacct gcccggtggg ggggcagagc cctgctccct ccgggtcttc 73620

ctactcttct ctctgctctg acgggatttg ttgattctct ccattttggt gtctttctct 73680

tttagatatt gtatcaatct ttagaaaagg catagtctac ttgttataaa tcgttaggat 73740

actgcctccc ccagggtcta aaattacata ttagagggga aaagctgaac actgaagtca 73800

gttctcaaca atttagaagg aaaacctaga aaacatttgg cagaaaatta catttcgatg 73860

tttttgaatg aatacgagca agcttttaca acagtgctga tctaaaaata cttagcactt 73920

ggcctgagat gcctggtgag cattacaggc aaggggaatc tggaggtagc cgacctgagg 73980

acatggcttc tgaacctgtc ttttgggagt ggtatggaag gtggagcgtt caccagtgac 74040

ctggaaggcc cagcaccacc ctccttccca ctcttctcat cttgacagag cctgccccag 74100

cgctgacgtg tcaggaaaac acccagggaa ctaggaaggc acttctgcct gaggggcagc 74160

ctgccttgcc cactcctgct ctgctcgcct cggatcagct gagccttctg agctggcctc 74220

tcactgcctc cccaaggccc cctgcctgcc ctgtcaggag gcagaaggaa gcaggtgtga 74280

gggcagtgca aggagggagc acaaccccca gctcccgctc cgggctccga cttgtgcaca 74340

ggcagagccc agaccctgga ggaaatccta cctttgaatt caagaacatt tggggaattt 74400

ggaaatctct ttgcccccaa acccccattc tgtcctacct ttaatcaggt cctgctcagc 74460

agtgagagca gatgaggtga aaaggccaag aggtttggct cctgcccact gatagcccct 74520

ctccccgcag tgtttgtgtg tcaagtggca aagctgttct tcctggtgac cctgattata 74580

tccagtaaca catagactgt gcgcataggc ctgctttgtc tcctctatcc tgggcttttg 74640

ttttgctttt tagttttgct tttagttttt ctgtcccttt tatttaacgc accgactaga 74700

cacacaaagc agttgaattt ttatatatat atctgtatat tgcacaatta taaactcatt 74760

ttgcttgtgg ctccacacac acaaaaaaag acctgttaaa attatacctg ttgcttaatt 74820

acaatatttc tgataaccat agcataggac aagggaaaat aaaaaaagaa aaaaaagaaa 74880

aaaaaacgac aaatctgtct gctggtcact tcttctgtcc aagcagattc gtggtctttt 74940

cctcgcttct ttcaagggct ttcctgtgcc aggtgaagga ggctccaggc agcacccagg 75000

ttttgcactc ttgtttctcc cgtgcttgtg aaagaggtcc caaggttctg ggtgcaggag 75060

cgctcccttg acctgctgaa gtccggaacg tagtcggcac agcctggtcg ccttccacct 75120

ctgggagctg gagtccactg gggtggcctg actcccccag tccccttccc gtgacctggt 75180

cagggtgagc ccatgtggag tcagcctcgc aggcctccct gccagtaggg tccgagtgtg 75240

tttcatcctt cccactctgt cgagcctggg ggctggagcg gagacgggag gcctggcctg 75300

tctcggaacc tgtgagctgc accaggtaga acgccaggga ccccagaatc atgtgcgtca 75360

gtccaagggg tcccctccag gagtagtgaa gactccagaa atgtcccttt cttctccccc 75420

atcctacgag taattgcatt tgcttttgta attcttaatg agcaatatct gctagagagt 75480

ttagctgtaa cagttctttt tgatcatctt tttttaataa ttagaaacac caaaaaaatc 75540

cagaaacttg ttcttccaaa gcagagagca ttataatcac cagggccaaa agcttccctc 75600

cctgctgtca ttgcttcttc tgaggcctga atccaaaaga aaaacagcca taggcccttt 75660

cagtggccgg gctacccgtg agcccttcgg aggaccaggg ctggggcagc ctctgggccc 75720

acatccgggg ccagctccgg cgtgtgttca gtgttagcag tgggtcatga tgctctttcc 75780

cacccagcct gggatagggg cagaggaggc gaggaggccg ttgccgctga tgtttggccg 75840

tgaacaggtg ggtgtctgcg tgcgtccacg tgcgtgtttt ctgactgaca tgaaatcgac 75900

gcccgagtta gcctcacccg gtgacctcta gccctgcccg gatggagcgg ggcccacccg 75960

gttcagtgtt tctggggagc tggacagtgg agtgcaaaag gcttgcagaa cttgaagcct 76020

gctccttccc ttgctaccac ggcctccttt ccgtttgatt tgtcactgct tcaatcaata 76080

acagccgctc cagagtcagt agtcaatgaa tatatgacca aatatcacca ggactgttac 76140

tcaatgtgtg ccgagccctt gcccatgctg ggctcccgtg tatctggaca ctgtaacgtg 76200

tgctgtgttt gctccccttc cccttccttc tttgcccttt acttgtcttt ctggggtttt 76260

tctgtttggg tttggtttgg tttttatttc tccttttgtg ttccaaacat gaggttctct 76320

ctactggtcc tcttaactgt ggtgttgagg cttatatttg tgtaattttt ggtgggtgaa 76380

aggaattttg ctaagtaaat ctcttctgtg tttgaactga agtctgtatt gtaactatgt 76440

ttaaagtaat tgttccagag acaaatattt ctagacactt tttctttaca aacaaaagca 76500

ttcggaggga gggggatggt gactgagatg agaggggaga gctgaacaga tgacccctgc 76560

ccagatcagc cagaagccac ccaaagcagt ggagcccagg agtcccactc caagccagca 76620

agccgaatag ctgatgtgtt gccactttcc aagtcactgc aaaaccaggt tttgttccgc 76680

ccagtggatt cttgttttgc ttcccctccc cccgagatta ttaccaccat cccgtgcttt 76740

taaggaaagg caagattgat gtttccttga ggggagccag gaggggatgt gtgtgtgcag 76800

agctgaagag ctggggagaa tggggctggg cccacccaag caggaggctg ggacgctctg 76860

ctgtgggcac aggtcaggct aatgttggca gatgcagctc ttcctggaca ggccaggtgg 76920

tgggcattct ctctccaagg tgtgccccgt gggcattact gtttaagaca cttccgtcac 76980

atcccacccc atcctccagg gctcaacact gtgacatctc tattccccac cctccccttc 77040

ccagggcaat aaaatgacca tggagggggc ttgcactctc ttggctgtca cccgatcgcc 77100

agcaaaactt agatgtgaga aaaccccttc ccattccatg gcgaaaacat ctccttagaa 77160

aagccattac cctcattagg catggttttg ggctcccaaa acacctgaca gcccctccct 77220

cctctgagag gcggagagtg ctgactgtag tgaccattgc atgccgggtg cagcatctgg 77280

aagagctagg cagggtgtct gccccctcct gagttgaagt catgctcccc tgtgccagcc 77340

cagaggccga gagctatgga cagcattgcc agtaacacag gccaccctgt gcagaaggga 77400

gctggctcca gcctggaaac ctgtctgagg ttgggagagg tgcacttggg gcacagggag 77460

aggccgggac acacttagct ggagatgtct ctaaaagccc tgtatcgtat tcaccttcag 77520

tttttgtgtt ttgggacaat tactttagaa aataagtagg tcgttttaaa aacaaaaatt 77580

attgattgct tttttgtagt gttcagaaaa aaggttcttt gtgtatagcc aaatgactga 77640

aagcactgat atatttaaaa acaaaaggca atttattaag gaaatttgta ccatttcagt 77700

aaacctgtct gaatgtacct gtatacgttt caaaaacacc ccccccccac tgaatccctg 77760

taacctattt attatataaa gagtttgcct tataaattta cataaaaatg tccgtttgtg 77820

tcttttgttg taaaaatcaa gtgatttttt cataaggttc ttttactatt ggaaaagatg 77880

ggcagcacgc agttttattt tatttttgta agttttttaa tacatgtgaa agcaaagaat 77940

actcagcatg cctttctaag tgacgcgttt gcaccttttg ttgggaagta ctgtatcctg 78000

tgctgttagc attctcgata aatctctctg tgaaagtgac tcaaggtctg ggctttcatt 78060

ataagacaga agtccccctc cagctcacat gacagcatgg tgctgcgttt cctcattgga 78120

tctggctgtc cctggacaca ggtagctgcc ttcaggcctg ccacgagcgg ccaagggaag 78180

cctcctccat atgctggcct cgctggcccc tcagcttctt ccaagccagt gctctccagg 78240

cacactgctc cagcgtgtga cgggaagggc ctggcatgag tcagcctgca gcacaacctc 78300

cctgctccag acccgtatgg taggggcacc ccctaggtct ggatgtgctg tggtgctttt 78360

ggacaccccc acccccgcag gctgtggctc ctcctgtgtc tcattctggc caggaccctc 78420

acgtgccctc tgttgactgc taacgtggtt ctctgaccag gcaagggcag gctgaggggt 78480

ttgcccaaag ggggccccct tgttactggc ttccttggct ctcaggagca gcctcaccag 78540

gttggtaagg ggctggagga gacaactgct caaaggagtc cagcttcaca tgcacatgct 78600

agaaggtacc ctcggaaggc ctggccttca aaggtagatc ccagggttga aaagtcaact 78660

tgtatgcatt gagcatctcg tatgccagcc ctgttccgtg agctgatggg cctttgtgtg 78720

taagtaggac caagtgcccc cgtggaggtt agcatgggtg tgcagtcatt tcagatactt 78780

gagttggtac atctcagtaa agtctgtccc gtgagaagcc atgggtttca tggtatggtt 78840

ggcatcttcc ttgggagtgg ccacagtggt ggtggcttca ggaaagagac tccaacaggg 78900

gccagctgtg ggccttgggc acttctcgtt tctaggaaaa gtcctaagtc tgtagggcta 78960

ggggtgggga accccttcgc tgtcaggatc aagagggcaa ggggaactgt cgctggagga 79020

gacatccagc tggagaaaca aaagagtaag tctgcgttgc tgcttgtggg gtcttcccca 79080

tctcagggcg gggaccgggg gtggcggtcc agacaagtaa tcaaggacga tgcccaggag 79140

gggacaggta cggggtggca ggagctctgc cggcgggctc aggaagcctt caccacagct 79200

gcctgagctc acccttgcca aatgagggct ggggcagcag caacgcatac actcacggct 79260

gtggcgggca gcgttctcgg catatttcag gacacctaag gagactgaat ggctcaaggc 79320

tgctgccgtg tgcagggggc tagacgtggg gcgggcaggc agggctcctg gtaacagccc 79380

tgcaggccgc agtggagagc agggttccgg cagggccgcc caggagcttt cggaaggccc 79440

ggccccggcc cctttccgag cagcccgggc ctccgccctg ccctctgtcc ccaacgccgg 79500

gagccgccgt tcgtcctcca gagccccgcc cggcgagccc cgggaggccg atcgccgctc 79560

gcggaacccg ccgggacccg ggccctcccc ggcgcggggc gcccccgtgt gacccagcgc 79620

gcggccgcgg cgcgcaagat ggcggcgggc ccgggcaccg ccccttccgc cccgccgggc 79680

gtcgcacgag gccggctcga aggggaagtg agtcagtgtc cgcggacccg gccggcccag 79740

gcccgcgccc gccgcggccc tgagaggccc cggcaggtcc cggcccggcg gcggcagcca 79800

tggccggggg gccgggcccg ggggagcccg cagcccccgg cgcccagcac ttcttgtacg 79860

aggtgccgcc ctgggtcatg tgccgcttct acaaagtgat ggacgccctg gagcccgccg 79920

actggtgcca gttcggtggg tggcggcggg ctgccggggg gcgggaggcg cgcgggctcc 79980

tggcgccgac gcctgacgcc c 80001

<210> 3
<211> 20
<212> DNA
<213> Artificial sequence

<220>
<223> Primer

<400> 3
tatttgatca atccccaggg 20

<210> 4
<211> 20
<212> DNA
<213> Artificial sequence

<220>
<223> Primer

<400> 4
ctccctctcc cagttaccgt 20

<210> 5
<211> 22
<212> DNA
<213> Artificial sequence

<220>
<223> Primer

<400> 5
aggagagact ggaagaaaag tc 22

<210> 6
<211> 20
<212> DNA
<213> Artificial sequence

<220>
<223> Primer

<400> 6
cttgaggggt ttgtccttga 20

<210>  7
<211>  23
<212>  DNA
<213>  Artificial sequence

<220>
<223>  Primer

<400>  7
ctcaccagtt cctgctttga tgt                                              23


<210>  8
<211>  22
<212>  DNA
<213>  Artificial sequence

<220>
<223>  Primer

<400>  8
aggagagact ggaggaaaag tc                                               22


<210>  9
<211>  25
<212>  DNA
<213>  Artificial sequence

<220>
<223>  Primer

<400>  9
cttaaacttc agtggcttgt ctctg                                            25


<210>  10
<211>  21
<212>  DNA
<213>  Artificial sequence

<220>
<223>  Primer

<400>  10
cgggggacat aaaagttatt g                                                21


<210>  11
<211>  22
<212>  DNA
<213>  Artificial sequence

<220>
<223>  Primer

<400>  11
tgcattgttt taccagtgtc aa                                               22


<210>  12
<211>  20

<212>  DNA
<213>  Artificial sequence

<220>
<223>  Synthetic oligonucleotide

<400>  12
aactctctcg gtcacgggcg                                                   20


<210>  13
<211>  20
<212>  DNA
<213>  Artificial sequence

<220>
<223>  Synthetic oligonucleotide

<400>  13
cacactgacc tttcagggct                                                   20


<210>  14
<211>  20
<212>  DNA
<213>  Artificial sequence

<220>
<223>  Synthetic oligonucleotide

<400>  14
gatcactgga acacaatggt                                                   20


<210>  15
<211>  20
<212>  DNA
<213>  Artificial sequence

<220>
<223>  Synthetic oligonucleotide

<400>  15
cgtgccatgg aagtccttcc                                                   20


<210>  16
<211>  20
<212>  DNA
<213>  Artificial sequence

<220>
<223>  Synthetic oligonucleotide

<400>  16
ggtttttctc ctttattatc                                                   20

## Description

### Statement of Government Support

This invention was made with government support under P30HD024064 and 5R01NS057819 awarded by the National Institutes of Health. The government has certain rights in the invention.

### Sequence Listing

The present application is being filed along with a Sequence Listing in electronic format. The Sequence Listing is provided as a file entitled BIOL0263WOSEQ_ST25.txt created March 2, 2016, which is 120 Kb in size. The information in the electronic format of the sequence listing is incorporated herein by reference in its entirety.

### Field

Provided are methods for modulating expression of methyl CpG binding protein 2 (MECP2) mRNA and protein in an animal. Such methods are useful to treat, prevent, or ameliorate neurological disorders, including MECP2 duplication syndrome, by reducing expression and amount of MECP2 mRNA and protein in an animal.

### Background

Methyl CpG binding protein 2 (MECP2) is located on chromosome Xq28 and plays a fundamental role in epigenetics, controlling chromatin states, and expression of thousands of genes (Chahrour et al., Science, 2008, 320:1224-1229; Nan et al., Nature, 1998, 393:386-389; Jones et al., Nat. Genet., 1998, 19:187-191). MECP2 expression must be maintained within a fairly narrow range to assure proper gene expression and neuronal function (Nan et al., Nature, 1988, 393:386-389). MECP2 duplication syndrome caused by overexpression of MECP2 is characterized by autism, intellectual disability, motor dysfunction, anxiety, epilepsy, recurrent respiratory tract infections, and early death, typically in males (Ramocki et al., Am J Med Genet A, 2010, 152A:1079-1088). Underexpression of MECP2 is associated with Rett Syndrome, which is characterized by normal early growth and development followed by a slowing of development, loss of purposeful use of the hands, distinctive hand movements, slowed brain and head growth, problems with walking, seizures, and intellectual disability, typically in females (Weaving et al., J Med Genet, 2005, 42:1-7).

Currently there is a lack of acceptable options for treating such neurological disorders. It is therefore an object herein to provide methods for the treatment of such disorders.

### Summary

Provided herein are methods for modulating expression and amount of methyl CpG binding protein 2 (MECP2) mRNA and protein. In certain embodiments, compounds useful for modulating expression and amount of MECP2 mRNA and protein are antisense compounds. In certain embodiments, the antisense compounds are modified antisense oligonucleotides.

In certain embodiments, modulation can occur in a cell or tissue. In certain embodiments, the cell or tissue is in an animal. In certain embodiments, the animal is a human. In certain embodiments, MECP2 mRNA levels are reduced. In certain embodiments, MECP2 protein levels are reduced. Such reduction can occur in a time-dependent manner or in a dose-dependent manner.

Also provided are methods useful for preventing, treating, and ameliorating disorders and syndromes associated with MECP2 overexpression. In certain embodiments, a disorder associated with MECP2 overexpression is a neurological disorder. In certain embodiments, the neurological disorder is MECP2 duplication syndrome. In certain embodiments, MECP2 duplication syndrome is characterized by having additional copies of MECP2, which leads to overexpression of MECP2.

In certain embodiments, MECP2 duplication syndrome is characterized by autism, intellectual disability, motor dysfunction, anxiety, epilepsy, recurrent respiratory tract infections, and early death. In certain embodiments, MECP2 duplication syndrome is inherited in an X-linked pattern.

In certain embodiments, methods of treatment include administering a MECP2 antisense compound to an individual in need thereof. In certain embodiments, methods of treatment include administering a MECP2 modified antisense oligonucleotide to an individual in need thereof.

In certain embodiments, MECP2 levels are reduced sufficiently to prevent, treat, and ameliorate symptoms of MECP2 duplication syndrome, but not enough to cause symptoms of Rett Syndrome.

### Brief Description of the Drawings

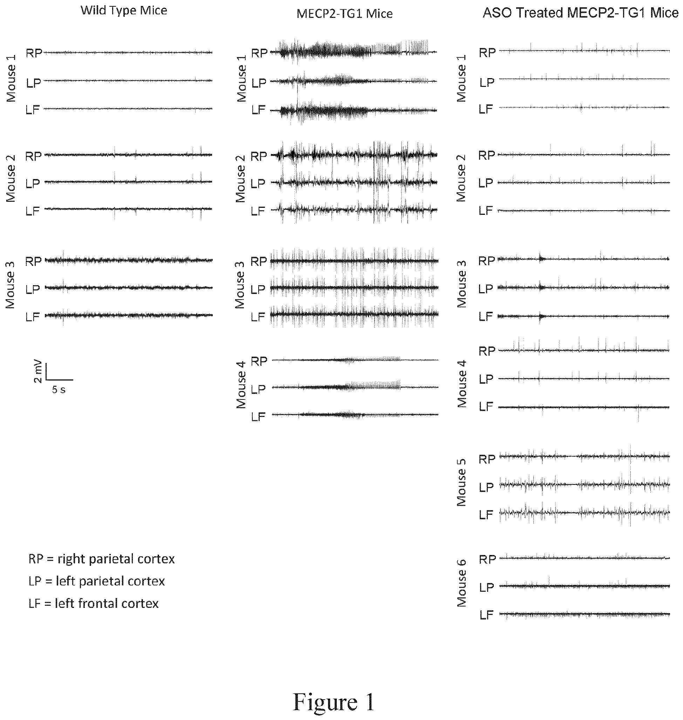

**Figure 1** displays representative EEG traces for WT mice, MECP2-TG1 mice without Isis No. 628785 treatment, and MECP2-TG1 mice that received treatment with Isis No. 628785.

### Detailed Description

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Additionally, as used herein, the use of "and" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described. All documents, or portions of documents, cited in this disclosure, including, but not limited to, patents, patent applications, published patent applications, articles, books, treatises, and GENBANK Accession Numbers and associated sequence information obtainable through databases such as National Center for Biotechnology Information (NCBI) and other data referred to throughout in the disclosure herein are hereby expressly incorporated by reference for the portions of the document discussed herein, as well as in their entirety.

### Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis.

Unless otherwise indicated, the following terms have the following meanings:
"2'-O-methoxyethyl" (also 2'-MOE and 2'-OCH₂CH₂-OCH₃ and MOE) refers to an O-methoxyethyl modification of the 2' position of a furanose ring. A 2'-O-methoxyethyl modified sugar is a modified sugar.
"2'-MOE nucleoside" (also 2'-O-methoxyethyl nucleoside) means a nucleoside comprising a 2'-MOE modified sugar moiety.
"2'-substituted nucleoside" means a nucleoside comprising a substituent at the 2'-position of the furanose ring other than H or OH. In certain embodiments, 2' substituted nucleosides include nucleosides with bicyclic sugar modifications.
"5-methylcytosine" means a cytosine modified with a methyl group attached to the 5 position. A 5-methylcytosine is a modified nucleobase.

"Administered concomitantly" refers to the co-administration of two pharmaceutical agents in any manner in which the pharmacological effects of both are manifest in the patient at the same time. Concomitant administration does not require that both pharmaceutical agents be administered in a single pharmaceutical composition, in the same dosage form, or by the same route of administration. The effects of both pharmaceutical agents need not manifest themselves at the same time. The effects need only be overlapping for a period of time and need not be coextensive.

"Administering" means providing a pharmaceutical agent to an animal, and includes, but is not limited to administering by a medical professional and self-administering.

"Amelioration" refers to a lessening, slowing, stopping, or reversing of at least one indicator of the severity of a syndrome or condition. The severity of indicators may be determined by subjective or objective measures, which are known to those skilled in the art.

"Animal" refers to a human or non-human animal, including, but not limited to, mice, rats, rabbits, dogs, cats, pigs, and non-human primates, including, but not limited to, monkeys and chimpanzees.

"Antibody" refers to a molecule characterized by reacting specifically with an antigen in some way, where the antibody and the antigen are each defined in terms of the other. Antibody may refer to a complete antibody molecule or any fragment or region thereof, such as the heavy chain, the light chain, Fab region, and Fc region.

"Antisense activity" means any detectable or measurable activity attributable to the hybridization of an antisense compound to its target nucleic acid. In certain embodiments, antisense activity is a decrease in the amount or expression of a target nucleic acid or protein encoded by such target nucleic acid.

"Antisense compound" means an oligomeric compound that is capable of undergoing hybridization to a target nucleic acid through hydrogen bonding. Examples of antisense compounds include single-stranded and double-stranded compounds, such as, antisense oligonucleotides, siRNAs, shRNAs, ssRNAs, and occupancy-based compounds.

"Antisense inhibition" or "inhibition" means reduction of target nucleic acid levels in the presence of an antisense compound complementary to a target nucleic acid compared to target nucleic acid levels or in the absence of the antisense compound.

"Antisense mechanisms" are all those mechanisms involving hybridization of a compound with a target nucleic acid, wherein the outcome or effect of the hybridization is either target degradation or target occupancy with concomitant stalling of the cellular machinery involving, for example, transcription or splicing.

"Antisense oligonucleotide" means a single-stranded oligonucleotide having a nucleobase sequence that permits hybridization to a corresponding segment of a target nucleic acid.

"Base complementarity" refers to the capacity for the precise base pairing of nucleobases of an antisense oligonucleotide with corresponding nucleobases in a target nucleic acid (i.e., hybridization), and is mediated by Watson-Crick, Hoogsteen or reversed Hoogsteen hydrogen binding between corresponding nucleobases.

"Bicyclic sugar" means a furanose ring modified by the bridging of two atoms. A bicyclic sugar is a modified sugar.

"Bicyclic nucleoside" (also BNA) means a nucleoside having a sugar moiety comprising a bridge connecting two carbon atoms of the sugar ring, thereby forming a bicyclic ring system. In certain embodiments, the bridge connects the 4'-carbon and the 2'-carbon of the sugar ring.

"Cap structure" or "terminal cap moiety" means chemical modifications, which have been incorporated at either terminus of an antisense compound.

"cEt" or "constrained ethyl" means a bicyclic nucleoside having a sugar moiety comprising a bridge connecting the 4'-carbon and the 2'-carbon, wherein the bridge has the formula: 4'-CH(CH₃)-O-2'.

"Constrained ethyl nucleoside" (also cEt nucleoside) means a nucleoside comprising a bicyclic sugar moiety comprising a 4'-CH(CH₃)-O-2' bridge.

"Chemically distinct region" refers to a region of an antisense compound that is in some way chemically different than another region of the same antisense compound. For example, a region having 2'-O-methoxyethyl nucleosides is chemically distinct from a region having nucleosides without 2'-O-methoxyethyl modifications.

"Chimeric antisense compound" means an antisense compound that has at least two chemically distinct regions, each position having a plurality of subunits.

"Co-administration" means administration of two or more pharmaceutical agents to an individual. The two or more pharmaceutical agents may be in a single pharmaceutical composition, or may be in separate pharmaceutical compositions. Each of the two or more pharmaceutical agents may be administered through the same or different routes of administration. Co-administration encompasses parallel or sequential administration.

"Complementarity" means the capacity for pairing between nucleobases of a first nucleic acid and a second nucleic acid.

"Comprise," "comprises," and "comprising" will be understood to imply the inclusion of a stated step or element or group of steps or elements but not the exclusion of any other step or element or group of steps or elements.

"Contiguous nucleobases" means nucleobases immediately adjacent to each other.

"Designing" or "designed to" refer to the process of designing an oligomeric compound that specifically hybridizes with a selected nucleic acid molecule.

"Diluent" means an ingredient in a composition that lacks pharmacological activity, but is pharmaceutically necessary or desirable. For example, in drugs that are injected, the diluent may be a liquid, e.g. saline solution.

"Dose" means a specified quantity of a pharmaceutical agent provided in a single administration, or in a specified time period. In certain embodiments, a dose may be administered in one, two, or more boluses, tablets, or injections. For example, in certain embodiments where subcutaneous administration is desired, the desired dose requires a volume not easily accommodated by a single injection, therefore, two or more injections may be used to achieve the desired dose. In certain embodiments, the pharmaceutical agent is administered by infusion over an extended period of time or continuously. Doses may be stated as the amount of pharmaceutical agent per hour, day, week, or month.

"Effective amount" in the context of modulating an activity or of treating or preventing a condition means the administration of that amount of pharmaceutical agent to an individual in need of such modulation, treatment, or prophylaxis, either in a single dose or as part of a series, that is effective for modulation of that effect, or for treatment or prophylaxis or improvement of that condition. The effective amount may vary among individuals depending on the health and physical condition of the individual to be treated, the taxonomic group of the individuals to be treated, the formulation of the composition, assessment of the individual's medical condition, and other relevant factors.

"Efficacy" means the ability to produce a desired effect.

"Expression" includes all the functions by which a gene's coded information is converted into structures present and operating in a cell. Such structures include, but are not limited to the products of transcription and translation.

"Fully complementary" or "100% complementary" means each nucleobase of a first nucleic acid has a complementary nucleobase in a second nucleic acid. In certain embodiments, a first nucleic acid is an antisense compound and a target nucleic acid is a second nucleic acid.

"Gapmer" means a chimeric antisense compound in which an internal region having a plurality of nucleosides that support RNase H cleavage is positioned between external regions having one or more nucleosides, wherein the nucleosides comprising the internal region are chemically distinct from the nucleoside or nucleosides comprising the external regions. The internal region may be referred to as a "gap" and the external regions may be referred to as the "wings."

"Hybridization" means the annealing of complementary nucleic acid molecules. In certain embodiments, complementary nucleic acid molecules include, but are not limited to, an antisense compound and a target nucleic acid. In certain embodiments, complementary nucleic acid molecules include, but are not limited to, an antisense oligonucleotide and a nucleic acid target.

"Identifying an animal having a MECP2 associated disorder" means identifying an animal having been diagnosed with a MECP2 associated disorder or predisposed to develop a MECP2 associated disorder. Individuals predisposed to develop a MECP2 associated disorder include those having one or more risk factors for developing a MECP2 associated disorder, including, having a personal or family history or genetic predisposition to one or more MECP2 associated disorders. Such identification may be accomplished by any method including evaluating an individual's medical history and standard clinical tests or assessments, such as genetic testing.

"Immediately adjacent" means there are no intervening elements between the immediately adjacent elements.

"Individual" means a human or non-human animal selected for treatment or therapy.

"Inhibiting MECP2" means reducing the level or expression of a MECP2 mRNA and/or protein. In certain embodiments, MECP2 mRNA and/or protein levels are inhibited in the presence of an antisense compound targeting MECP2, including an antisense oligonucleotide targeting MECP2, as compared to expression of MECP2 mRNA and/or protein levels in the absence of a MECP2 antisense compound, such as an antisense oligonucleotide.

"Inhibiting the expression or activity" refers to a reduction or blockade of the expression or activity and does not necessarily indicate a total elimination of expression or activity.

"Internucleoside linkage" refers to the chemical bond between nucleosides.

"Linked nucleosides" means adjacent nucleosides linked together by an internucleoside linkage.

"MECP2 antisense compound" means an antisense compound targeting MECP2.

"MECP2" means the mammalian gene methyl CpG binding protein 2 (MECP2), including the human gene methyl CpG binding protein 2 (MECP2). Human MECP2 has been mapped to human chromosome Xq28.

"MECP2 associated disorder" means any disorder or syndrome associated with any MECP2 nucleic acid or expression product thereof. Such disorders may include a neurological disorder. Such neurological disorders may include MECP2 duplication syndrome.

"MECP2 nucleic acid" means any nucleic acid encoding MECP2. For example, in certain embodiments, a MECP2 nucleic acid includes a DNA sequence encoding MECP2, an RNA sequence transcribed from DNA encoding MECP2 (including genomic DNA comprising introns and exons), and an mRNA sequence encoding MECP2.

"MECP2 mRNA" means any messenger RNA expression product of a DNA sequence encoding MECP2.

"MECP2 protein" means the polypeptide expression product of a MECP2 nucleic acid.

"Mismatch" or "non-complementary nucleobase" refers to the case when a nucleobase of a first nucleic acid is not capable of pairing with the corresponding nucleobase of a second or target nucleic acid.

"Modified internucleoside linkage" refers to a substitution or any change from a naturally occurring internucleoside bond (*i*.*e*., a phosphodiester internucleoside bond).

"Modified nucleobase" means any nucleobase other than adenine, cytosine, guanine, thymidine, or uracil. An "unmodified nucleobase" means the purine bases adenine (A) and guanine (G), and the pyrimidine bases thymine (T), cytosine (C), and uracil (U).

"Modified nucleoside" means a nucleoside having, independently, a modified sugar moiety and/or modified nucleobase.

"Modified nucleotide" means a nucleotide having, independently, a modified sugar moiety, modified internucleoside linkage, and/or modified nucleobase.

"Modified antisense oligonucleotide" means an oligonucleotide comprising at least one modified internucleoside linkage, modified sugar, and/or modified nucleobase.

"Modified sugar" means substitution and/or any change from a natural sugar moiety.

"Monomer" means a single unit of an oligomer. Monomers include, but are not limited to, nucleosides and nucleotides, whether naturally occurring or modified.

"Motif" means the pattern of unmodified and modified nucleosides in an antisense compound.

"Natural sugar moiety" means a sugar moiety found in DNA (2'-H) or RNA (2'-OH).

"Naturally occurring internucleoside linkage" means a 3' to 5' phosphodiester linkage.

"Non-complementary nucleobase" refers to a pair of nucleobases that do not form hydrogen bonds with one another or otherwise support hybridization.

"Nucleic acid" refers to molecules composed of monomeric nucleotides. A nucleic acid includes, but is not limited to, ribonucleic acids (RNA), deoxyribonucleic acids (DNA), single-stranded nucleic acids, double-stranded nucleic acids, small interfering ribonucleic acids (siRNA), and microRNAs (miRNA).

"Nucleobase" means a heterocyclic moiety capable of pairing with a base of another nucleic acid.

"Nucleobase complementarity" refers to a nucleobase that is capable of base pairing with another nucleobase. For example, in DNA, adenine (A) is complementary to thymine (T). For example, in RNA, adenine (A) is complementary to uracil (U). In certain embodiments, complementary nucleobase refers to a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair.

"Nucleobase sequence" means the order of contiguous nucleobases independent of any sugar, linkage, and/or nucleobase modification.

"Nucleoside" means a nucleobase linked to a sugar.

"Nucleoside mimetic" includes those structures used to replace the sugar or the sugar and the base and not necessarily the linkage at one or more positions of an oligomeric compound such as for example nucleoside mimetics having morpholino, cyclohexenyl, cyclohexyl, tetrahydropyranyl, bicyclo, or tricyclo sugar mimetics, e.g., non furanose sugar units. Nucleotide mimetic includes those structures used to replace the nucleoside and the linkage at one or more positions of an oligomeric compound such as for example peptide nucleic acids or morpholinos (morpholinos linked by -N(H)-C(=O)-O- or other non-phosphodiester linkage). Sugar surrogate overlaps with the slightly broader term nucleoside mimetic but is intended to indicate replacement of the sugar unit (furanose ring) only. The tetrahydropyranyl rings provided herein are illustrative of an example of a sugar surrogate wherein the furanose sugar group has been replaced with a tetrahydropyranyl ring system. "Mimetic" refers to groups that are substituted for a sugar, a nucleobase, and/or internucleoside linkage. Generally, a mimetic is used in place of the sugar or sugar-internucleoside linkage combination, and the nucleobase is maintained for hybridization to a selected target.

"Nucleotide" means a nucleoside having a phosphate group covalently linked to the sugar portion of the nucleoside.

"Off-target effect" refers to an unwanted or deleterious biological effect associated with modulation of RNA or protein expression of a gene other than the intended target nucleic acid.

"Oligomeric compound" or "oligomer" means a polymer of linked monomeric subunits which is capable of hybridizing to at least a region of a nucleic acid molecule.

"Oligonucleotide" means a polymer of linked nucleosides each of which can be modified or unmodified, independent one from another.

"Parenteral administration" means administration through injection (e.g., bolus injection) or infusion. Parenteral administration includes subcutaneous administration, intravenous administration, intramuscular administration, intraarterial administration, intraperitoneal administration, or intracranial administration, e.g., intrathecal or intracerebroventricular administration.

"Peptide" means a molecule formed by linking at least two amino acids by amide bonds. Without limitation, as used herein, peptide refers to polypeptides and proteins.

"Pharmaceutical agent" means a substance that provides a therapeutic benefit when administered to an individual. For example, in certain embodiments, an antisense oligonucleotide targeted to MECP2 is a pharmaceutical agent.

"Pharmaceutical composition" means a mixture of substances suitable for administering to an individual. For example, a pharmaceutical composition may comprise an antisense oligonucleotide and a sterile aqueous solution.

"Pharmaceutically acceptable derivative" encompasses pharmaceutically acceptable salts, conjugates, prodrugs or isomers of the compounds described herein.

"Pharmaceutically acceptable salts" means physiologically and pharmaceutically acceptable salts of antisense compounds, *i. e.,* salts that retain the desired biological activity of the parent oligonucleotide and do not impart undesired toxicological effects thereto.

"Phosphorothioate linkage" means a linkage between nucleosides where the phosphodiester bond is modified by replacing one of the non-bridging oxygen atoms with a sulfur atom. A phosphorothioate linkage is a modified internucleoside linkage.

"Portion" means a defined number of contiguous (*i.e.,* linked) nucleobases of a nucleic acid. In certain embodiments, a portion is a defined number of contiguous nucleobases of a target nucleic acid. In certain embodiments, a portion is a defined number of contiguous nucleobases of an antisense compound.

"Prevent" or "preventing" refers to delaying or forestalling the onset or development of a disorder or syndrome for a period of time from minutes to days, weeks to months, or indefinitely.

"Prodrug" means a therapeutic agent that is prepared in an inactive form that is converted to an active form (i.e., drug) within the body or cells thereof by the action of endogenous enzymes or other chemicals and/or conditions.

"Prophylactically effective amount" refers to an amount of a pharmaceutical agent that provides a prophylactic or preventative benefit to an animal.

"Region" is defined as a portion of the target nucleic acid having at least one identifiable structure, function, or characteristic.

"Ribonucleotide" means a nucleotide having a hydroxy at the 2' position of the sugar portion of the nucleotide. Ribonucleotides may be modified with any of a variety of substituents.

"Salt" means a physiologically and pharmaceutically acceptable salt(s) of antisense compounds, i.e., salts that retain the desired biological activity of the parent oligonucleotide and do not impart undesired toxicological effects thereto.

"Segments" are defined as smaller or sub-portions of regions within a target nucleic acid.

"Shortened" or "truncated" versions of antisense oligonucleotides taught herein have one, two or more nucleosides deleted.

"Side effects" means physiological responses attributable to a treatment other than desired effects. In certain embodiments, side effects include, without limitation, injection site reactions, liver function test abnormalities, renal function abnormalities, liver toxicity, renal toxicity, central nervous system abnormalities, and myopathies.

"Single-stranded oligonucleotide" means an oligonucleotide which is not hybridized to a complementary strand.

"Sites" as used herein, are defined as unique nucleobase positions within a target nucleic acid.

"Slows progression" means decrease in the development of the disorder or syndrome.

"Specifically hybridizable" refers to an antisense compound having a sufficient degree of complementarity between an antisense oligonucleotide and a target nucleic acid to induce a desired effect, while exhibiting minimal or no effects on non-target nucleic acids under conditions in which specific binding is desired, *i.e.,* under physiological conditions in the case of *in vivo* assays and therapeutic treatments.

"Stringent hybridization conditions" or "stringent conditions" refer to conditions under which an oligomeric compound will hybridize to its target sequence, but to a minimal number of other sequences.

"Targeting" or "targeted" means the process of design and selection of an antisense compound that will specifically hybridize to a target nucleic acid and induce a desired effect.

"Target nucleic acid," "target RNA," and "target RNA transcript" and "nucleic acid target" all mean a nucleic acid capable of being targeted by antisense compounds. In certain embodiments, the target nucleic acid is a MECP2 nucleic acid.

"Target region" means a portion of a target nucleic acid to which one or more antisense compounds is targeted.

"Target segment" means the sequence of nucleotides of a target nucleic acid to which an antisense compound is targeted. "5' target site" refers to the 5'-most nucleotide of a target segment. "3' target site" refers to the 3'-most nucleotide of a target segment.

"Therapeutically effective amount" means an amount of a pharmaceutical agent that provides a therapeutic benefit to an individual.

"Treat" or "treating" or "treatment" refers administering a composition to effect an alteration or improvement of the disorder or syndrome.

"Unmodified nucleobases" mean the purine bases adenine (A) and guanine (G), and the pyrimidine bases thymine (T), cytosine (C) and uracil (U).

"Unmodified nucleotide" means a nucleotide composed of naturally occuring nucleobases, sugar moieties, and internucleoside linkages. In certain embodiments, an unmodified nucleotide is an RNA nucleotide (*i.e.* β-D-ribonucleosides) or a DNA nucleotide (*i*.*e*. β-D-deoxyribonucleoside).

"Wing segment" means a plurality of nucleosides modified to impart to an oligonucleotide properties such as enhanced inhibitory activity, increased binding affinity for a target nucleic acid, or resistance to degradation by in vivo nucleases.

### Certain Embodiments

Certain embodiments provide methods for inhibiting MECP2 mRNA and protein expression. Certain embodiments provide methods, compounds, and compositions for decreasing MECP2 mRNA and protein levels.

Certain embodiments provide antisense compounds targeted to a MECP2 nucleic acid. In certain embodiments, the MECP2 nucleic acid is the sequence set forth in GENBANK Accession No. NM_004992.3 (incorporated herein as SEQ ID NO: 1) and the complement of GENBANK Accession No. NT_167198.1 truncated from nucleotides 4203000 to 4283000 (incorporated herein as SEQ ID NO: 2).

Certain embodiments provide methods for the treatment, prevention, or amelioration of disorders and syndromes associated with MECP2 in an individual in need thereof. Also contemplated are methods for the preparation of a medicament for the treatment, prevention, or amelioration of a disorder or syndrome associated with MECP2. MECP2 associated disorders and syndromes include neurological disorders. In certain embodiments, MECP2 associated disorders include MECP2 duplication syndrome.

The present disclosure provides the following non-limiting numbered embodiments:
Embodiment 1. A method comprising administering a MECP2 antisense compound to an animal for treating a MECP2 associated disorder.
Embodiment 2. A method comprising:
   identifying an animal having a MECP2 associated disorder; and
   administering a MECP2 antisense compound.
Embodiment 3. The method of any preceding embodiment, wherein MECP2 associated disorder is a neurological disorder.
Embodiment 4. The method of any preceding embodiment, wherein the MECP2 associated disorder is MECP2 duplication syndrome.
Embodiment 5. The method of any preceding embodiment, wherein the animal is a human.
Embodiment 6. The method of any preceding embodiment, wherein the administering is parenteral administration.
Embodiment 7. The method of embodiment 6, wherein the parenteral administration is any of intracerebroventricular administration or intrathecal administration.
Embodiment 8. The method of any preceding embodiment, wherein the administering reduces MECP2 mRNA and or protein levels.
Embodiment 9. The method of embodiment 8, wherein the administering reduces MECP2 mRNA and or protein levels by 15, 20, 25, 30, 35, 40, 45, 50, 55, 60, or 65 percent.
Embodiment 10. The method of any preceding embodiment, wherein the administering improves motor function.
Embodiment 11. The method of embodiment 10, wherein motor function is improved by 10, 15, 20, 25, 30, or 35 percent.
Embodiment 12. The method of any preceding embodiment, wherein the administering improves anxiety.
Embodiment 13. The method of embodiment 12, wherein the administering improves anxiety by 5, 10, 15, 20, 25, 30, 35, 40, 45, or 50 percent.
Embodiment 14. The method of any preceding embodiment, wherein the administering improves social interaction.
Embodiment 15. The method of embodiment 14, wherein the administering improves social interaction by 5, 10, 15, 20, 25, 30, 35, 40, 45, or 50 percent.
Embodiment 16. The method of any preceding embodiment, wherein the administering improves activity.
Embodiment 17. The method of embodiment 16, wherein the administering improves activity by 5, 10, 15, 20, 25, 30, 35, 40, 45, 50, 55, 60, 65, 70, 75, or 80 percent.
Embodiment 18. The method of any preceding claim, wherein the administering reduces seizures.
Embodiment 19. The method of any preceding claim, wherein the administering normalizes EEG discharges.
Embodiment 20. The method of any preceding embodiment, wherein at least one symptom of a MECP2 associated disorder is ameliorated, treated, prevented, or slowed.
Embodiment 21. The method of any preceding embodiment, wherein the antisense compound is a modified antisense oligonucleotide.
Embodiment 22. The method of embodiment 21, wherein the modified antisense oligonucleotide has the nucleobase sequence of SEQ ID NO: 12-16.

### Antisense Compounds

Oligomeric compounds include, but are not limited to, oligonucleotides, oligonucleosides, oligonucleotide analogs, oligonucleotide mimetics, antisense compounds, antisense oligonucleotides, and siRNAs. An oligomeric compound may be "antisense" to a target nucleic acid, meaning that is is capable of undergoing hybridization to a target nucleic acid through hydrogen bonding.

In certain embodiments, an antisense compound has a nucleobase sequence that, when written in the 5' to 3' direction, comprises the reverse complement of the target segment of a target nucleic acid to which it is targeted. In certain such embodiments, an antisense oligonucleotide has a nucleobase sequence that, when written in the 5' to 3' direction, comprises the reverse complement of the target segment of a target nucleic acid to which it is targeted.

In certain embodiments, an antisense compound targeted to a target nucleic acid is 12 to 30 subunits in length. In certain embodiments, an antisense compound targeted to a target nucleic acid is 12 to 25 subunits in length. In certain embodiments, an antisense compound targeted to a target nucleic acid is 12 to 22 subunits in length. In certain embodiments, an antisense compound targeted to a target nucleic acid is 14 to 20 subunits in length. In certain embodiments, an antisense compound targeted to a target nucleic acid is 15 to 25 subunits in length. In certain embodiments, an antisense compound targeted to a target nucleic acid is 18 to 22 subunits in length. In certain embodiments, an antisense compound targeted to a target nucleic acid is 19 to 21 subunits in length. In certain embodiments, the antisense compound is 8 to 80, 12 to 50, 13 to 30, 13 to 50, 14 to 30, 14 to 50, 15 to 30, 15 to 50, 16 to 30, 16 to 50, 17 to 30, 17 to 50, 18 to 30, 18 to 50, 19 to 30, 19 to 50, or 20 to 30 linked subunits in length.

In certain embodiments, an antisense compound targeted to a target nucleic acid is 12 subunits in length. In certain embodiments, an antisense compound targeted to a target nucleic acid is 13 subunits in length. In certain embodiments, an antisense compound targeted to a target nucleic acid is 14 subunits in length. In certain embodiments, an antisense compound targeted to a target nucleic acid is 15 subunits in length. In certain embodiments, an antisense compound targeted to a target nucleic acid is 16 subunits in length. In certain embodiments, an antisense compound targeted to a target nucleic acid is 17 subunits in length. In certain embodiments, an antisense compound targeted to a target nucleic acid is 18 subunits in length. In certain embodiments, an antisense compound targeted to a target nucleic acid is 19 subunits in length. In certain embodiments, an antisense compound targeted to a target nucleic acid is 20 subunits in length. In certain embodiments, an antisense compound targeted to a target nucleic acid is 21 subunits in length. In certain embodiments, an antisense compound targeted to a target nucleic acid is 22 subunits in length. In certain embodiments, an antisense compound targeted to a target nucleic acid is 23 subunits in length. In certain embodiments, an antisense compound targeted to a target nucleic acid is 24 subunits in length. In certain embodiments, an antisense compound targeted to a target nucleic acid is 25 subunits in length. In certain embodiments, an antisense compound targeted to a target nucleic acid is 26 subunits in length. In certain embodiments, an antisense compound targeted to a target nucleic acid is 27 subunits in length. In certain embodiments, an antisense compound targeted to a target nucleic acid is 28 subunits in length. In certain embodiments, an antisense compound targeted to a target nucleic acid is 29 subunits in length. In certain embodiments, an antisense compound targeted to a target nucleic acid is 30 subunits in length. In certain embodiments, the antisense compound targeted to a target nucleic acid is 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 linked subunits in length, or a range defined by any two of the above values. In certain embodiments the antisense compound is an antisense oligonucleotide, and the linked subunits are nucleosides.

In certain embodiments antisense oligonucleotides targeted to a target nucleic acid may be shortened or truncated. For example, a single subunit may be deleted from the 5' end (5' truncation), or alternatively from the 3' end (3' truncation). A shortened or truncated antisense compound targeted to a target nucleic acid may have two subunits deleted from the 5' end, or alternatively may have two subunits deleted from the 3' end, of the antisense compound. Alternatively, the deleted nucleosides may be dispersed throughout the antisense compound, for example, in an antisense compound having one nucleoside deleted from the 5' end and one nucleoside deleted from the 3' end.

When a single additional subunit is present in a lengthened antisense compound, the additional subunit may be located at the 5' or 3' end of the antisense compound. When two or more additional subunits are present, the added subunits may be adjacent to each other, for example, in an antisense compound having two subunits added to the 5' end (5' addition), or alternatively to the 3' end (3' addition), of the antisense compound. Alternatively, the added subunits may be dispersed throughout the antisense compound, for example, in an antisense compound having one subunit added to the 5' end and one subunit added to the 3' end.

It is possible to increase or decrease the length of an antisense compound, such as an antisense oligonucleotide, and/or introduce mismatch bases without eliminating activity. For example, in Woolf et al. (Proc. Natl. Acad. Sci. USA 89:7305-7309, 1992), a series of antisense oligonucleotides 13-25 nucleobases in length were tested for their ability to induce cleavage of a target RNA in an oocyte injection model. Antisense oligonucleotides 25 nucleobases in length with 8 or 11 mismatch bases near the ends of the antisense oligonucleotides were able to direct specific cleavage of the target mRNA, albeit to a lesser extent than the antisense oligonucleotides that contained no mismatches. Similarly, target specific cleavage was achieved using 13 nucleobase antisense oligonucleotides, including those with 1 or 3 mismatches.

Gautschi et al (J. Natl. Cancer Inst. 93:463-471, March 2001) demonstrated the ability of an oligonucleotide having 100% complementarity to the bcl-2 mRNA and having 3 mismatches to the bcl-xL mRNA to reduce the expression of both bcl-2 and bcl-xL *in vitro* and *in vivo.* Furthermore, this oligonucleotide demonstrated potent anti-tumor activity *in vivo.*

Maher and Dolnick (Nuc. Acid. Res. 16:3341-3358,1988) tested a series of tandem 14 nucleobase antisense oligonucleotides, and a 28 and 42 nucleobase antisense oligonucleotides comprised of the sequence of two or three of the tandem antisense oligonucleotides, respectively, for their ability to arrest translation of human DHFR in a rabbit reticulocyte assay. Each of the three 14 nucleobase antisense oligonucleotides alone was able to inhibit translation, albeit at a more modest level than the 28 or 42 nucleobase antisense oligonucleotides.

### Antisense Compound Motifs

In certain embodiments, antisense compounds targeted to a target nucleic acid have chemically modified subunits arranged in patterns, or motifs, to confer to the antisense compounds properties such as enhanced inhibitory activity, increased binding affinity for a target nucleic acid, or resistance to degradation by *in vivo* nucleases.

Chimeric antisense compounds typically contain at least one region modified so as to confer increased resistance to nuclease degradation, increased cellular uptake, increased binding affinity for the target nucleic acid, and/or increased inhibitory activity. A second region of a chimeric antisense compound may optionally serve as a substrate for the cellular endonuclease RNase H, which cleaves the RNA strand of an RNA:DNA duplex.

Antisense compounds having a gapmer motif are considered chimeric antisense compounds. In a gapmer an internal region having a plurality of nucleotides that supports RNaseH cleavage is positioned between external regions having a plurality of nucleotides that are chemically distinct from the nucleosides of the internal region. In the case of an antisense oligonucleotide having a gapmer motif, the gap segment generally serves as the substrate for endonuclease cleavage, while the wing segments comprise modified nucleosides. In certain embodiments, the regions of a gapmer are differentiated by the types of sugar moieties comprising each distinct region. The types of sugar moieties that are used to differentiate the regions of a gapmer may in some embodiments include β-D-ribonucleosides, β-D-deoxyribonucleosides, 2'-modified nucleosides (such 2'-modified nucleosides may include 2'-MOE, and 2'-O-CH₃, among others), and bicyclic sugar modified nucleosides (such bicyclic sugar modified nucleosides may include those having a 4'-(CH₂)n-O-2' bridge, where n=1 or n=2 and 4'-CH₂-O-CH₂-2'). In certain embodiments, wings may include several modified sugar moieties, including, for example 2'-MOE. In certain embodiments, wings may include several modified and unmodified sugar moieties. In certain embodiments, wings may include various combinations of 2'-MOE nucleosides and 2'-deoxynucleosides.

Each distinct region may comprise uniform sugar moieties, variant, or alternating sugar moieties. The wing-gap-wing motif is frequently described as "X-Y-Z", where "X" represents the length of the 5' wing, "Y" represents the length of the gap, and "Z" represents the length of the 3' wing. "X" and "Z" may comprise uniform, variant, or alternating sugar moieties. In certain embodiments, "X" and "Y" may include one or more 2'-deoxynucleosides. "Y" may comprise 2'-deoxynucleosides. As used herein, a gapmer described as "X-Y-Z" has a configuration such that the gap is positioned immediately adjacent to each of the 5' wing and the 3' wing. Thus, no intervening nucleotides exist between the 5' wing and gap, or the gap and the 3' wing. Any of the antisense compounds described herein can have a gapmer motif. In certain embodiments, "X" and "Z" are the same; in other embodiments they are different.

In certain embodiments, gapmers provided herein include, for example 20-mers having a motif of 5-10-5. In certain embodiments, gapmers provided herein include, for example 19-mers having a motif of 5-9-5. In certain embodiments, gapmers provided herein include, for example 18-mers having a motif of 5-8-5. In certain embodiments, gapmers provided herein include, for example 18-mers having a motif of 4-8-6. In certain embodiments, gapmers provided herein include, for example 18-mers having a motif of 6-8-4. In certain embodiments, gapmers provided herein include, for example 18-mers having a motif of 5-7-6.

### Target Nucleic Acids, Target Regions and Nucleotide Sequences

Nucleotide sequences that encode MECP2 include, without limitation, the following: GENBANK Accession No. NM_004992.3 (incorporated herein as SEQ ID NO: 1) and the complement of GENBANK Accession No. NT_167198.1 truncated from nucleotides 4203000 to 4283000 (incorporated herein as SEQ ID NO: 2).

It is understood that the sequence set forth in each SEQ ID NO in the Examples contained herein is independent of any modification to a sugar moiety, an internucleoside linkage, or a nucleobase. As such, antisense compounds defined by a SEQ ID NO may comprise, independently, one or more modifications to a sugar moiety, an internucleoside linkage, or a nucleobase. Antisense compounds described by Isis Number (Isis No) indicate a combination of nucleobase sequence and motif.

In certain embodiments, a target region is a structurally defined region of the target nucleic acid. For example, a target region may encompass a 3' UTR, a 5' UTR, an exon, an intron, an exon/intron junction, a coding region, a translation initiation region, translation termination region, or other defined nucleic acid region. The structurally defined regions for MECP2 can be obtained by accession number from sequence databases such as NCBI and such information is incorporated herein by reference. In certain embodiments, a target region may encompass the sequence from a 5' target site of one target segment within the target region to a 3' target site of another target segment within the same target region.

Targeting includes determination of at least one target segment to which an antisense compound hybridizes, such that a desired effect occurs. In certain embodiments, the desired effect is a reduction in mRNA target nucleic acid levels. In certain embodiments, the desired effect is reduction of levels of protein encoded by the target nucleic acid or a phenotypic change associated with the target nucleic acid.

A target region may contain one or more target segments. Multiple target segments within a target region may be overlapping. Alternatively, they may be non-overlapping. In certain embodiments, target segments within a target region are separated by no more than about 300 nucleotides. In certain emodiments, target segments within a target region are separated by a number of nucleotides that is, is about, is no more than, is no more than about, 250, 200, 150, 100, 90, 80, 70, 60, 50, 40, 30, 20, or 10 nucleotides on the target nucleic acid, or is a range defined by any two of the preceeding values. In certain embodiments, target segments within a target region are separated by no more than, or no more than about, 5 nucleotides on the target nucleic acid. In certain embodiments, target segments are contiguous. Contemplated are target regions defined by a range having a starting nucleic acid that is any of the 5' target sites or 3' target sites listed herein.

Suitable target segments may be found within a 5' UTR, a coding region, a 3' UTR, an intron, an exon, or an exon/intron junction. Target segments containing a start codon or a stop codon are also suitable target segments. A suitable target segment may specifcally exclude a certain structurally defined region such as the start codon or stop codon.

The determination of suitable target segments may include a comparison of the sequence of a target nucleic acid to other sequences throughout the genome. For example, the BLAST algorithm may be used to identify regions of similarity amongst different nucleic acids. This comparison can prevent the selection of antisense compound sequences that may hybridize in a non-specific manner to sequences other than a selected target nucleic acid (i.e., non-target or off-target sequences).

There may be variation in activity (e.g., as defined by percent reduction of target nucleic acid levels) of the antisense compounds within an active target region. In certain embodiments, reductions in MECP2 mRNA levels are indicative of inhibition of MECP2 expression. Reductions in levels of an MECP2 protein are also indicative of inhibition of target mRNA expression. Phenotypic changes are indicative of inhibition of MECP2 expression. Improvement in neurological function is indicative of inhibition of MECP2 expression. Improved motor function, activity, social behavior, and memory are indicative of inhibition of MECP2 expression. Reduction of anxiety-like behaviors is indicative of inhibition of MECP2 expression.

### Hybridization

In some embodiments, hybridization occurs between an antisense compound disclosed herein and an MECP2 nucleic acid. The most common mechanism of hybridization involves hydrogen bonding (e.g., Watson-Crick, Hoogsteen or reversed Hoogsteen hydrogen bonding) between complementary nucleobases of the nucleic acid molecules.

Hybridization can occur under varying conditions. Stringent conditions are sequence-dependent and are determined by the nature and composition of the nucleic acid molecules to be hybridized.

Methods of determining whether a sequence is specifically hybridizable to a target nucleic acid are well known in the art. In certain embodiments, the antisense compounds provided herein are specifically hybridizable with a MECP2 nucleic acid.

### Complementarity

An antisense compound and a target nucleic acid are complementary to each other when a sufficient number of nucleobases of the antisense compound can hydrogen bond with the corresponding nucleobases of the target nucleic acid, such that a desired effect will occur (e.g., antisense inhibition of a target nucleic acid, such as a MECP2 nucleic acid).

Non-complementary nucleobases between an antisense compound and a target nucleic acid may be tolerated provided that the antisense compound remains able to specifically hybridize to a target nucleic acid. Moreover, an antisense compound may hybridize over one or more segments of a target nucleic acid such that intervening or adjacent segments are not involved in the hybridization event (e.g., a loop structure, mismatch or hairpin structure).

In certain embodiments, the antisense compounds provided herein, or a specified portion thereof, are, or are at least, 70%, 80%, 85%, 86%, 87%, 88%, 89%, 90%, 91%, 92%, 93%, 94%, 95%, 96%, 97%, 98%, 99%, or 100% complementary to a MECP2 nucleic acid, a target region, target segment, or specified portion thereof. Percent complementarity of an antisense compound with a target nucleic acid can be determined using routine methods.

For example, an antisense compound in which 18 of 20 nucleobases of the antisense compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an antisense compound which is 18 nucleobases in length having 4 (four) noncomplementary nucleobases which are flanked by two regions of complete complementarity with the target nucleic acid would have 77.8% overall complementarity with the target nucleic acid and would thus fall within the scope of the present invention. Percent complementarity of an antisense compound with a region of a target nucleic acid can be determined routinely using BLAST programs (basic local alignment search tools) and PowerBLAST programs known in the art (Altschul et al., J. Mol. Biol., 1990, 215, 403 410; Zhang and Madden, Genome Res., 1997, 7, 649 656). Percent homology, sequence identity or complementarity, can be determined by, for example, the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482 489).

In certain embodiments, the antisense compounds provided herein, or specified portions thereof, are fully complementary (*i.e.,* 100% complementary) to a target nucleic acid, or specified portion thereof. For example, an antisense compound may be fully complementary to a MECP2 nucleic acid, or a target region, or a target segment or target sequence thereof. As used herein, "fully complementary" means each nucleobase of an antisense compound is capable of precise base pairing with the corresponding nucleobases of a target nucleic acid. For example, a 20 nucleobase antisense compound is fully complementary to a target sequence that is 400 nucleobases long, so long as there is a corresponding 20 nucleobase portion of the target nucleic acid that is fully complementary to the antisense compound. Fully complementary can also be used in reference to a specified portion of the first and /or the second nucleic acid. For example, a 20 nucleobase portion of a 30 nucleobase antisense compound can be "fully complementary" to a target sequence that is 400 nucleobases long. The 20 nucleobase portion of the 30 nucleobase oligonucleotide is fully complementary to the target sequence if the target sequence has a corresponding 20 nucleobase portion wherein each nucleobase is complementary to the 20 nucleobase portion of the antisense compound. At the same time, the entire 30 nucleobase antisense compound may or may not be fully complementary to the target sequence, depending on whether the remaining 10 nucleobases of the antisense compound are also complementary to the target sequence.

The location of a non-complementary nucleobase may be at the 5' end or 3' end of the antisense compound. Alternatively, the non-complementary nucleobase or nucleobases may be at an internal position of the antisense compound. When two or more non-complementary nucleobases are present, they may be contiguous (i.e., linked) or non-contiguous. In one embodiment, a non-complementary nucleobase is located in the wing segment of a gapmer antisense oligonucleotide.

In certain embodiments, antisense compounds that are, or are up to 11, 12, 13, 14, 15, 16, 17, 18, 19, or 20 nucleobases in length comprise no more than 4, no more than 3, no more than 2, or no more than 1 non-complementary nucleobase(s) relative to a target nucleic acid, or specified portion thereof.

In certain embodiments, antisense compounds that are, or are up to 11, 12, 13, 14, 15, 16, 17, 18, 19, 20, 21, 22, 23, 24, 25, 26, 27, 28, 29, or 30 nucleobases in length comprise no more than 6, no more than 5, no more than 4, no more than 3, no more than 2, or no more than 1 non-complementary nucleobase(s) relative to a target nucleic acid, or specified portion thereof.

The antisense compounds provided herein also include those which are complementary to a portion of a target nucleic acid. As used herein, "portion" refers to a defined number of contiguous (i.e. linked) nucleobases within a region or segment of a target nucleic acid. A "portion" can also refer to a defined number of contiguous nucleobases of an antisense compound. In certain embodiments, the antisense compounds, are complementary to at least an 8 nucleobase portion of a target segment. In certain embodiments, the antisense compounds are complementary to at least a 9 nucleobase portion of a target segment. In certain embodiments, the antisense compounds are complementary to at least a 10 nucleobase portion of a target segment. In certain embodiments, the antisense compounds, are complementary to at least an 11 nucleobase portion of a target segment. In certain embodiments, the antisense compounds, are complementary to at least a 12 nucleobase portion of a target segment. In certain embodiments, the antisense compounds, are complementary to at least a 13 nucleobase portion of a target segment. In certain embodiments, the antisense compounds, are complementary to at least a 14 nucleobase portion of a target segment. In certain embodiments, the antisense compounds, are complementary to at least a 15 nucleobase portion of a target segment. Also contemplated are antisense compounds that are complementary to at least a 9, 10, 11, 12, 13, 14, 15, 16, 17, 18, 19, 20, or more nucleobase portion of a target segment, or a range defined by any two of these values.

### Identity

The antisense compounds provided herein may also have a defined percent identity to a particular nucleotide sequence, SEQ ID NO, or compound represented by a specific Isis number, or portion thereof. As used herein, an antisense compound is identical to the sequence disclosed herein if it has the same nucleobase pairing ability. For example, a RNA which contains uracil in place of thymidine in a disclosed DNA sequence would be considered identical to the DNA sequence since both uracil and thymidine pair with adenine. Shortened and lengthened versions of the antisense compounds described herein as well as compounds having non-identical bases relative to the antisense compounds provided herein also are contemplated. The non-identical bases may be adjacent to each other or dispersed throughout the antisense compound. Percent identity of an antisense compound is calculated according to the number of bases that have identical base pairing relative to the sequence to which it is being compared.

In certain embodiments, the antisense compounds, or portions thereof, are at least 70%, 75%, 80%, 85%, 90%, 95%, 96%, 97%, 98%, 99% or 100% identical to one or more of the antisense compounds or SEQ ID NOs, or a portion thereof, disclosed herein.

In certain embodiments, a portion of the antisense compound is compared to an equal length portion of the target nucleic acid. In certain embodiments, an 8, 9, 10, 11, 12, 13, 14, 15, 16, 17, 18, 19, 20, 21, 22, 23, 24, or 25 nucleobase portion is compared to an equal length portion of the target nucleic acid.

In certain embodiments, a portion of the antisense oligonucleotide is compared to an equal length portion of the target nucleic acid. In certain embodiments, an 8, 9, 10, 11, 12, 13, 14, 15, 16, 17, 18, 19, 20, 21, 22, 23, 24, or 25 nucleobase portion is compared to an equal length portion of the target nucleic acid.

### Modifications

A nucleoside is a base-sugar combination. The nucleobase (also known as base) portion of the nucleoside is normally a heterocyclic base moiety. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. Oligonucleotides are formed through the covalent linkage of adjacent nucleosides to one another, to form a linear polymeric oligonucleotide. Within the oligonucleotide structure, the phosphate groups are commonly referred to as forming the internucleoside linkages of the oligonucleotide.

Modifications to antisense compounds encompass substitutions or changes to internucleoside linkages, sugar moieties, or nucleobases. Modified antisense compounds are often preferred over native forms because of desirable properties such as, for example, enhanced cellular uptake, enhanced affinity for nucleic acid target, increased stability in the presence of nucleases, or increased inhibitory activity.

Chemically modified nucleosides may also be employed to increase the binding affinity of a shortened or truncated antisense oligonucleotide for its target nucleic acid. Consequently, comparable results can often be obtained with shorter antisense compounds that have such chemically modified nucleosides.

### Modified Internucleoside Linkages

The naturally occuring internucleoside linkage of RNA and DNA is a 3' to 5' phosphodiester linkage. Antisense compounds having one or more modified, i.e. non-naturally occurring, internucleoside linkages are often selected over antisense compounds having naturally occurring internucleoside linkages because of desirable properties such as, for example, enhanced cellular uptake, enhanced affinity for target nucleic acids, and increased stability in the presence of nucleases.

Oligonucleotides having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates, phosphoramidate, and phosphorothioates. Methods of preparation of phosphorous-containing and non-phosphorous-containing linkages are well known.

In certain embodiments, antisense compounds targeted to a MECP2 nucleic acid comprise one or more modified internucleoside linkages. In certain embodiments, the modified internucleoside linkages are interspersed throughout the antisense compound. In certain embodiments, the modified internucleoside linkages are phosphorothioate linkages. In certain embodiments, each internucleoside linkage of an antisense compound is a phosphorothioate internucleoside linkage.

### Modified Sugar Moieties

Antisense compounds can optionally contain one or more nucleosides wherein the sugar group has been modified. Such sugar modified nucleosides may impart enhanced nuclease stability, increased binding affinity, or some other beneficial biological property to the antisense compounds. In certain embodiments, nucleosides comprise chemically modified ribofuranose ring moieties. Examples of chemically modified ribofuranose rings include without limitation, addition of substitutent groups (including 5' and 2' substituent groups, bridging of non-geminal ring atoms to form bicyclic nucleic acids (BNA), replacement of the ribosyl ring oxygen atom with S, N(R), or C(R₁)(R₂) (R, R₁ and R₂ are each independently H, C₁-C₁₂ alkyl or a protecting group) and combinations thereof. Examples of chemically modified sugars include 2'-F-5'-methyl substituted nucleoside (see PCT International Application WO 2008/101157 Published on 8/21/08 for other disclosed 5',2'-bis substituted nucleosides) or replacement of the ribosyl ring oxygen atom with S with further substitution at the 2'-position (see published U.S. Patent Application US2005-0130923, published on June 16, 2005) or alternatively 5'-substitution of a BNA (see PCT International Application WO 2007/134181 Published on 11/22/07 wherein LNA is substituted with for example a 5'-methyl or a 5'-vinyl group).

Examples of nucleosides having modified sugar moieties include without limitation nucleosides comprising 5'-vinyl, 5'-methyl (R or S), 4'-S, 2'-F, 2'-OCH₃, 2'-OCH₂CH₃, 2'-OCH₂CH₂F and 2'-O(CH₂)₂OCH₃ substituent groups. The substituent at the 2' position can also be selected from allyl, amino, azido, thio, O-allyl, O-C₁-C₁₀ alkyl, OCF₃, OCH₂F, O(CH₂)₂SCH₃, O(CH₂)₂-O-N(Rₘ)(Rₙ), O-CH₂-C(=O)-N(Rₘ)(Rₙ), and O-CH₂-C(=O)-N(R₁)-(CH₂)₂-N(Rₘ)(Rₙ), where each Rₗ, Rₘ and Rₙ is, independently, H or substituted or unsubstituted C₁-C₁₀ alkyl.

As used herein, "bicyclic nucleosides" refer to modified nucleosides comprising a bicyclic sugar moiety. Examples of bicyclic nucleosides include without limitation nucleosides comprising a bridge between the 4' and the 2' ribosyl ring atoms. In certain embodiments, antisense compounds provided herein include one or more bicyclic nucleosides comprising a 4' to 2' bridge. Examples of such 4' to 2' bridged bicyclic nucleosides, include but are not limited to one of the formulae: 4'-(CH₂)-O-2' (LNA); 4'-(CH₂)-S-2'; 4'-(CH₂)₂-O-2' (ENA); 4'-CH(CH₃)-O-2' and 4'-CH(CH₂OCH₃)-O-2' (and analogs thereof see U.S. Patent 7,399,845, issued on July 15, 2008); 4'-C(CH₃)(CH₃)-O-2' (and analogs thereof see published International Application WO/2009/006478, published January 8, 2009); 4'-CH₂-N(OCH₃)-2' (and analogs thereof see published International Application WO/2008/150729, published December 11, 2008); 4'-CH₂-O-N(CH₃)-2' (see published U.S. Patent Application US2004-0171570, published September 2, 2004 ); 4'-CH₂-N(R)-O-2', wherein R is H, C₁-C₁₂ alkyl, or a protecting group (see U.S. Patent 7,427,672, issued on September 23, 2008); 4'-CH₂-C(H)(CH₃)-2' (see Chattopadhyaya et al., J. Org. Chem., 2009, 74, 118-134); and 4'-CH₂-C-(=CH₂)-2' (and analogs thereof see published International Application WO 2008/154401, published on December 8, 2008).

Further reports related to bicyclic nucleosides can also be found in published literature (see for example: Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 2007, 129(26) 8362-8379; Elayadi et al., Curr. Opinion Invest. Drugs, 2001, 2, 558-561; Braasch et al., Chem. Biol., 2001, 8, 1-7; and Orum et al., Curr. Opinion Mol. Ther., 2001, 3, 239-243; U.S. Patent Nos. 6,268,490; 6,525,191; 6,670,461; 6,770,748; 6,794,499; 7,034,133; 7,053,207; 7,399,845; 7,547,684; and 7,696,345; U.S. Patent Publication No. US2008-0039618; US2009-0012281; U.S. Patent Serial Nos. 60/989,574; 61/026,995; 61/026,998; 61/056,564; 61/086,231; 61/097,787; and 61/099,844; Published PCT International applications WO 1994/014226; WO 2004/106356; WO 2005/021570; WO 2007/134181; WO 2008/150729; WO 2008/154401; and WO 2009/006478. Each of the foregoing bicyclic nucleosides can be prepared having one or more stereochemical sugar configurations including for example α-L-ribofuranose and β-D-ribofuranose (see PCT international application PCT/DK98/00393, published on March 25, 1999 as WO 99/14226).

In certain embodiments, bicyclic sugar moieties of BNA nucleosides include, but are not limited to, compounds having at least one bridge between the 4' and the 2' position of the pentofuranosyl sugar moiety wherein such bridges independently comprises 1 or from 2 to 4 linked groups independently selected from - [C(Rₐ)(R_{b})]ₙ-, -C(Rₐ)=C(R_{b})-, -C(Rₐ)=N-, -C(=O)-, -C(=NRₐ)-, -C(=S)-, -O-, -Si(Rₐ)₂-, -S(=O)ₓ-, and -N(Rₐ)-;
wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In certain embodiments, the bridge of a bicyclic sugar moiety is -[C(Rₐ)(R_{b})]ₙ-, -[C(Rₐ)(R_{b})]ₙ-O-, -C(RₐR_{b})-N(R)-O- or -C(RₐR_{b})-O-N(R)-. In certain embodiments, the bridge is 4'-CH₂-2', 4'-(CH₂)₂-2', 4'-(CH₂)₃-2', 4'-CH₂-O-2', 4'-(CH₂)₂-O-2', 4'-CH₂-O-N(R)-2' and 4'-CH₂-N(R)-O-2'- wherein each Ris, independently, H, a protecting group or C₁-C₁₂ alkyl.

In certain embodiments, bicyclic nucleosides are further defined by isomeric configuration. For example, a nucleoside comprising a 4'-2' methylene-oxy bridge, may be in the α-L configuration or in the β-D configuration. Previously, α-L-methyleneoxy (4'-CH₂-O-2') BNA's have been incorporated into antisense oligonucleotides that showed antisense activity (Frieden et al., Nucleic Acids Research, 2003, 21, 6365-6372).

In certain embodiments, bicyclic nucleosides include, but are not limited to, (A) α-L-methyleneoxy (4'-CH₂-O-2') BNA , (B) β-D-methyleneoxy (4'-CH₂-O-2') BNA , (C) ethyleneoxy (4'-(CH₂)₂-O-2') BNA , (D) aminooxy (4'-CH₂-O-N(R)-2') BNA, (E) oxyamino (4'-CH₂-N(R)-O-2') BNA, and (F) methyl(methyleneoxy) (4'-CH(CH₃)-O-2') BNA, (G) methylene-thio (4'-CH₂-S-2') BNA, (H) methylene-amino (4'-CH₂-N(R)-2') BNA, (I) methyl carbocyclic (4'-CH₂-CH(CH₃)-2') BNA, and (J) propylene carbocyclic (4'-(CH₂)₃-2') BNA as depicted below. 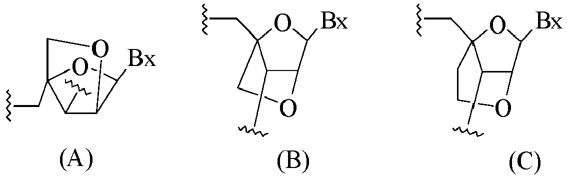 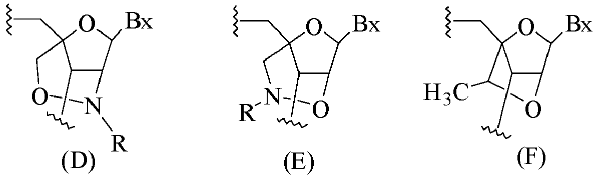 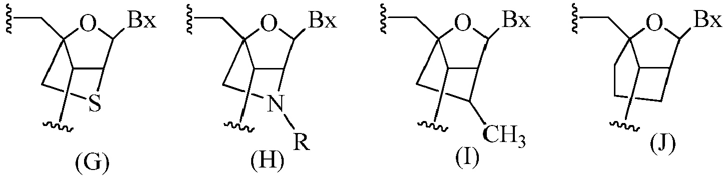 wherein Bx is the base moiety and R is independently H, a protecting group or C₁-C₁₂ alkyl.

In certain embodiments, bicyclic nucleosides are provided having Formula I: 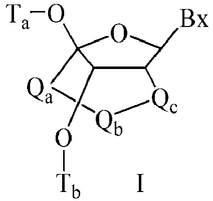 wherein:
Bx is a heterocyclic base moiety;
-Qₐ-Q_{b}-Q_{c}- is -CH₂-N(R_{c})-CH₂-, -C(=O)-N(R_{c})-CH₂-, -CH₂-O-N(R_{c})-, -CH₂-N(R_{c})-O- or -N(R_{c})-O-CH₂;
R_{c} is C₁-C₁₂ alkyl or an amino protecting group; and
Tₐ and T_{b} are each, independently H, a hydroxyl protecting group, a conjugate group, a reactive phosphorus group, a phosphorus moiety or a covalent attachment to a support medium.

In certain embodiments, bicyclic nucleosides are provided having Formula II: 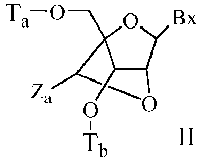 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Zₐ is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, substituted amide, thiol or substituted thio.

In one embodiment, each of the substituted groups is, independently, mono or poly substituted with substituent groups independently selected from halogen, oxo, hydroxyl, OJ_{c}, NJ_{c}J_{d}, SJ_{c}, N₃, OC(=X)J_{c}, and NJₑC(=X)NJ_{c}J_{d}, wherein each J_{c}, J_{d} and Jₑ is, independently, H, C₁-C₆ alkyl, or substituted C₁-C₆ alkyl and X is O or NJ_{c}.

In certain embodiments, bicyclic nucleosides are provided having Formula III: 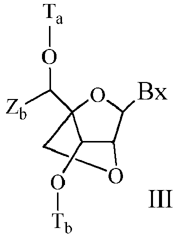 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Z_{b} is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl or substituted acyl (C(=O)-).

In certain embodiments, bicyclic nucleosides are provided having Formula IV: wherein: 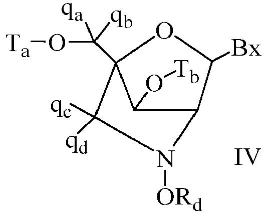
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
R_{d} is C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl;
each qₐ, q_{b}, q_{c} and q_{d} is, independently, H, halogen, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl, C₁-C₆ alkoxyl, substituted C₁-C₆ alkoxyl, acyl, substituted acyl, C₁-C₆ aminoalkyl or substituted C₁-C₆ aminoalkyl;

In certain embodiments, bicyclic nucleosides are provided having Formula V: 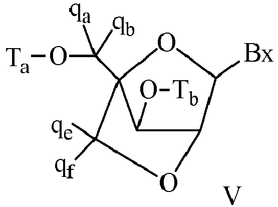 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
qₐ, q_{b}, qₑ and q_{f} are each, independently, hydrogen, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ;
or qₑ and q_{f} together are =C(q_{g})(qₕ);
q_{g} and qₕ are each, independently, H, halogen, C₁-C₁₂ alkyl or substituted C₁-C₁₂ alkyl.

The synthesis and preparation of the methyleneoxy (4'-CH₂-O-2') BNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). BNAs and preparation thereof are also described in WO 98/39352 and WO 99/14226.

Analogs of methyleneoxy (4'-CH₂-O-2') BNA and 2'-thio-BNAs, have also been prepared (Kumar et al., Bioorg. Med. Chem. Lett., 1998, 8, 2219-2222). Preparation of locked nucleoside analogs comprising oligodeoxyribonucleotide duplexes as substrates for nucleic acid polymerases has also been described (Wengel et al., WO 99/14226 ). Furthermore, synthesis of 2'-amino-BNA, a novel comformationally restricted high-affinity oligonucleotide analog has been described in the art (Singh et al., J. Org. Chem., 1998, 63, 10035-10039). In addition, 2'-amino- and 2'-methylamino-BNA's have been prepared and the thermal stability of their duplexes with complementary RNA and DNA strands has been previously reported.

In certain embodiments, bicyclic nucleosides are provided having Formula VI: 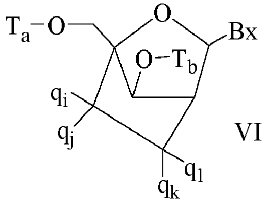 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
each qᵢ, qⱼ, qₖ and qₗ is, independently, H, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxyl, substituted C₁-C₁₂ alkoxyl, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖorN(H)C(=S)NJⱼJₖ; and
qᵢ and qⱼ or qₗ and qₖ together are =C(q_{g})(qₕ), wherein q_{g} and qₕ are each, independently, H, halogen, C₁-C₁₂ alkyl or substituted C₁-C₁₂ alkyl.

One carbocyclic bicyclic nucleoside having a 4'-(CH₂)₃-2' bridge and the alkenyl analog bridge 4'-CH=CH-CH₂-2' have been described (Freier et al., Nucleic Acids Research, 1997, 25(22), 4429-4443 and Albaek et al., J. Org. Chem., 2006, 71, 7731-7740). The synthesis and preparation of carbocyclic bicyclic nucleosides along with their oligomerization and biochemical studies have also been described (Srivastava et al., J. Am. Chem. Soc., 2007,129(26), 8362-8379).

As used herein, "4'-2' bicyclic nucleoside" or "4' to 2' bicyclic nucleoside" refers to a bicyclic nucleoside comprising a furanose ring comprising a bridge connecting two carbon atoms of the furanose ring connects the 2' carbon atom and the 4' carbon atom of the sugar ring.

As used herein, "monocylic nucleosides" refer to nucleosides comprising modified sugar moieties that are not bicyclic sugar moieties. In certain embodiments, the sugar moiety, or sugar moiety analogue, of a nucleoside may be modified or substituted at any position.

As used herein, "2'-modified sugar" means a furanosyl sugar modified at the 2' position. In certain embodiments, such modifications include substituents selected from: a halide, including, but not limited to substituted and unsubstituted alkoxy, substituted and unsubstituted thioalkyl, substituted and unsubstituted amino alkyl, substituted and unsubstituted alkyl, substituted and unsubstituted allyl, and substituted and unsubstituted alkynyl. In certain embodiments, 2' modifications are selected from substituents including, but not limited to: O[(CH₂)ₕO]ₘCH₃, O(CH₂)ₙNH₂, O(CH₂)ₙCH₃, O(CH₂)ₙF, O(CH₂)ₕONH₂, OCH₂C(=O)N(H)CH, and O(CH₂)ₙON[(CH₂)ₕCH₃]₂, where n and m are from 1 to about 10. Other 2'-substituent groups can also be selected from: C₁-C₁₂ alkyl, substituted alkyl, alkenyl, alkynyl, alkaryl, aralkyl, O-alkaryl or O-aralkyl, SH, SCH₃, OCN, Cl, Br, CN, F, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving pharmacokinetic properties, or a group for improving the pharmacodynamic properties of an antisense compound, and other substituents having similar properties. In certain embodiments, modifed nucleosides comprise a 2'-MOE side chain (Baker et al., J. Biol. Chem., 1997, 272, 11944-12000). Such 2'-MOE substitution have been described as having improved binding affinity compared to unmodified nucleosides and to other modified nucleosides, such as 2'- *O-*methyl, O-propyl, and O-aminopropyl. Oligonucleotides having the 2'-MOE substituent also have been shown to be antisense inhibitors of gene expression with promising features for *in vivo* use (Martin, Helv. Chim. Acta, 1995, 78, 486-504; Altmann et al., Chimia, 1996, 50, 168-176; Altmann et al., Biochem. Soc. Trans., 1996, 24, 630-637; and Altmann et al., Nucleosides Nucleotides, 1997, 16, 917-926).

As used herein, a "modified tetrahydropyran nucleoside" or "modified THP nucleoside" means a nucleoside having a six-membered tetrahydropyran "sugar" substituted in for the pentofuranosyl residue in normal nucleosides (a sugar surrogate). Modified THP nucleosides include, but are not limited to, what is referred to in the art as hexitol nucleic acid (HNA), anitol nucleic acid (ANA), manitol nucleic acid (MNA) (see Leumann, Bioorg. Med. Chem., 2002, 10, 841-854), fluoro HNA (F-HNA) or those compounds having Formula VII: 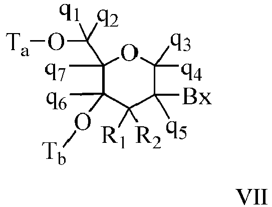 wherein independently for each of said at least one tetrahydropyran nucleoside analog of Formula VII:
Bx is a heterocyclic base moiety;
Tₐ and T_{b} are each, independently, an internucleoside linking group linking the tetrahydropyran nucleoside analog to the antisense compound or one of Tₐ and T_{b} is an internucleoside linking group linking the tetrahydropyran nucleoside analog to the antisense compound and the other of Tₐ and T_{b} is H, a hydroxyl protecting group, a linked conjugate group or a 5' or 3'-terminal group;
q₁, q₂, q₃, q₄, q₅, q₆ and q₇ are each independently, H, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl; and each of R₁ and R₂ is selected from hydrogen, hydroxyl, halogen, substituted or unsubstituted alkoxy, NJ₁J₂, SJ₁, N₃, OC(=X)J₁, OC(=X)NJ₁J₂, NJ₃C(=X)NJ₁J₂ and CN, wherein X is O, S or NJ₁ and each J₁, J₂ and J₃ is, independently, H or C₁-C₆ alkyl.
In certain embodiments, the modified THP nucleosides of Formula VII are provided wherein q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, THP nucleosides of Formula VII are provided wherein one of R₁ and R₂ is fluoro. In certain embodiments, R₁ is fluoro and R₂ is H; R₁ is methoxy and R₂ is H, and R₁ is H and R₂ is methoxyethoxy.

As used herein, "2'-modified" or "2'-substituted" refers to a nucleoside comprising a sugar comprising a substituent at the 2' position other than H or OH. 2'-modified nucleosides, include, but are not limited to, bicyclic nucleosides wherein the bridge connecting two carbon atoms of the sugar ring connects the 2' carbon and another carbon of the sugar ring; and nucleosides with non-bridging 2'substituents, such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-ON(Rₘ)(Rₙ), or O-CH₂-C(=O)-N(Rₘ)(Rₙ), where each Rₘ and Rₙ is, independently, H or substituted or unsubstituted C₁-C₁₀ alkyl. 2'-modifed nucleosides may further comprise other modifications, for example at other positions of the sugar and/or at the nucleobase.

As used herein, "2'-F" refers to a nucleoside comprising a sugar comprising a fluoro group at the 2' position.

As used herein, "2'-OMe" or "2'-OCH₃" or "2'-O-methyl" each refers to a nucleoside comprising a sugar comprising an -OCH₃ group at the 2' position of the sugar ring.

As used herein, "MOE" or "2'-MOE" or "2'-OCH₂CH₂OCH₃" or "2'-O-methoxyethyl" each refers to a nucleoside comprising a sugar comprising a -OCH₂CH₂OCH₃ group at the 2' position of the sugar ring.

As used herein, "oligonucleotide" refers to a compound comprising a plurality of linked nucleosides. In certain embodiments, one or more of the plurality of nucleosides is modified. In certain embodiments, an oligonucleotide comprises one or more ribonucleosides (RNA) and/or deoxyribonucleosides (DNA).

Many other bicyclo and tricyclo sugar surrogate ring systems are also known in the art that can be used to modify nucleosides for incorporation into antisense compounds (see for example review article: Leumann, Bioorg. Med. Chem., 2002, 10, 841-854).
Such ring systems can undergo various additional substitutions to enhance activity.

Methods for the preparations of modified sugars are well known to those skilled in the art.

In nucleotides having modified sugar moieties, the nucleobase moieties (natural, modified or a combination thereof) are maintained for hybridization with an appropriate nucleic acid target.

In certain embodiments, antisense compounds comprise one or more nucleosides having modified sugar moieties. In certain embodiments, the modified sugar moiety is 2'-MOE. In certain embodiments, the 2'-MOE modified nucleosides are arranged in a gapmer motif. In certain embodiments, the modified sugar moiety is a bicyclic nucleoside having a (4'-CH(CH₃)-O-2') bridging group. In certain embodiments, the (4'-CH(CH₃)-O-2') modified nucleosides are arranged throughout the wings of a gapmer motif.

### Compositions and Methods for Formulating Pharmaceutical Compositions

Antisense oligonucleotides may be admixed with pharmaceutically acceptable active or inert substances for the preparation of pharmaceutical compositions or formulations. Compositions and methods for the formulation of pharmaceutical compositions are dependent upon a number of criteria, including, but not limited to, route of administration, extent of disorder, or dose to be administered.

An antisense compound targeted to a MECP2 nucleic acid can be utilized in pharmaceutical compositions by combining the antisense compound with a suitable pharmaceutically acceptable diluent or carrier. A pharmaceutically acceptable diluent includes phosphate-buffered saline (PBS). PBS is a diluent suitable for use in compositions to be delivered parenterally. Accordingly, in one embodiment, employed in the methods described herein is a pharmaceutical composition comprising an antisense compound targeted to a MECP2 nucleic acid and a pharmaceutically acceptable diluent. In certain embodiments, the pharmaceutically acceptable diluent is PBS. In certain embodiments, the antisense compound is an antisense oligonucleotide.

Pharmaceutical compositions comprising antisense compounds encompass any pharmaceutically acceptable salts, esters, or salts of such esters, or any other oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

A prodrug can include the incorporation of additional nucleosides at one or both ends of an antisense compound which are cleaved by endogenous nucleases within the body, to form the active antisense compound.

### Conjugated Antisense Compounds

Antisense compounds may be covalently linked to one or more moieties or conjugates which enhance the activity, cellular distribution or cellular uptake of the resulting antisense oligonucleotides. Typical conjugate groups include cholesterol moieties and lipid moieties. Additional conjugate groups include carbohydrates, phospholipids, biotin, phenazine, folate, phenanthridine, anthraquinone, acridine, fluoresceins, rhodamines, coumarins, and dyes.

Antisense compounds can also be modified to have one or more stabilizing groups that are generally attached to one or both termini of antisense compounds to enhance properties such as, for example, nuclease stability. Included in stabilizing groups are cap structures. These terminal modifications protect the antisense compound having terminal nucleic acid from exonuclease degradation, and can help in delivery and/or localization within a cell. The cap can be present at the 5'-terminus (5'-cap), or at the 3'-terminus (3'-cap), or can be present on both termini. Cap structures are well known in the art and include, for example, inverted deoxy abasic caps. Further 3' and 5'-stabilizing groups that can be used to cap one or both ends of an antisense compound to impart nuclease stability include those disclosed in WO 03/004602 published on January 16, 2003.

### Cell culture and antisense compounds treatment

The effects of antisense compounds on the level, activity or expression of MECP2 nucleic acids can be tested *in vitro* in a variety of cell types. Cell types used for such analyses are available from commerical vendors (e.g. American Type Culture Collection, Manassus, VA; Zen-Bio, Inc., Research Triangle Park, NC; Clonetics Corporation, Walkersville, MD) and are cultured according to the vendor's instructions using commercially available reagents (e.g. Invitrogen Life Technologies, Carlsbad, CA). Illustrative cell types include, but are not limited to, HepG2 cells, Hep3B cells, and primary hepatocytes. In certain embodiments, cells are patient cells, such as B-lymphoblast cells.

### In vitro testing of antisense oligonucleotides

Described herein are methods for treatment of cells with antisense oligonucleotides, which can be modified appropriately for treatment with other antisense compounds.

Cells may be treated with antisense oligonucleotides when the cells reach approximately 60-80% confluency in culture.

One reagent commonly used to introduce antisense oligonucleotides into cultured cells includes the cationic lipid transfection reagent LIPOFECTIN (Invitrogen, Carlsbad, CA). Antisense oligonucleotides may be mixed with LIPOFECTIN in OPTI-MEM 1 (Invitrogen, Carlsbad, CA) to achieve the desired final concentration of antisense oligonucleotide and a LIPOFECTIN concentration that may range from 2 to 12 ug/mL per 100 nM antisense oligonucleotide.

Another reagent used to introduce antisense oligonucleotides into cultured cells includes LIPOFECTAMINE (Invitrogen, Carlsbad, CA). Antisense oligonucleotide is mixed with LIPOFECTAMINE in OPTI-MEM 1 reduced serum medium (Invitrogen, Carlsbad, CA) to achieve the desired concentration of antisense oligonucleotide and a LIPOFECTAMINE concentration that may range from 2 to 12 ug/mL per 100 nM antisense oligonucleotide.

Another reagent used to introduce antisense oligonucleotides into cultured cells includes TURBOFECT (Thermo Scientific, Carlsbad, CA).

Another technique used to introduce antisense oligonucleotides into cultured cells includes electroporation.

Cells are treated with antisense oligonucleotides by routine methods. Cells may be harvested 16-24 hours after antisense oligonucleotide treatment, at which time RNA or protein levels of target nucleic acids are measured by methods known in the art and described herein. In general, when treatments are performed in multiple replicates, the data are presented as the average of the replicate treatments.

The concentration of antisense oligonucleotide used varies from cell line to cell line. Methods to determine the optimal antisense oligonucleotide concentration for a particular cell line are well known in the art. Antisense oligonucleotides are typically used at concentrations ranging from 1 nM to 300 nM when transfected with LIPOFECTAMINE. Antisense oligonucleotides are used at higher concentrations ranging from 625 to 20,000 nM when transfected using electroporation.

### RNA Isolation

RNA analysis can be performed on total cellular RNA or poly(A)+ mRNA. Methods of RNA isolation are well known in the art. RNA is prepared using methods well known in the art, for example, using the TRIZOL Reagent (Invitrogen, Carlsbad, CA) according to the manufacturer's recommended protocols.

### Analysis of inhibition of target levels or expression

Inhibition of levels or expression of a MECP2 nucleic acid can be assayed in a variety of ways known in the art. For example, target nucleic acid levels can be quantitated by, e.g., Northern blot analysis, competitive polymerase chain reaction (PCR), or quantitaive real-time PCR. RNA analysis can be performed on total cellular RNA or poly(A)+ mRNA. Methods of RNA isolation are well known in the art. Northern blot analysis is also routine in the art. Quantitative real-time PCR can be conveniently accomplished using the commercially available ABI PRISM 7600, 7700, or 7900 Sequence Detection System, available from PE-Applied Biosystems, Foster City, CA and used according to manufacturer's instructions.

### Quantitative Real-Time PCR Analysis of Target RNA Levels

Quantitation of target RNA levels may be accomplished by quantitative real-time PCR using the ABI PRISM 7600, 7700, or 7900 Sequence Detection System (PE-Applied Biosystems, Foster City, CA) according to manufacturer's instructions. Methods of quantitative real-time PCR are well known in the art.

Prior to real-time PCR, the isolated RNA is subjected to a reverse transcriptase (RT) reaction, which produces complementary DNA (cDNA) that is then used as the substrate for the real-time PCR amplification. The RT and real-time PCR reactions are performed sequentially in the same sample well. RT and real-time PCR reagents may be obtained from Invitrogen (Carlsbad, CA). RT real-time-PCR reactions are carried out by methods well known to those skilled in the art.

Gene (or RNA) target quantities obtained by real time PCR are normalized using either the expression level of a gene whose expression is constant, such as cyclophilin A, or by quantifying total RNA using RIBOGREEN (Invitrogen, Inc. Carlsbad, CA). Cyclophilin A expression is quantified by real time PCR, by being run simultaneously with the target, multiplexing, or separately. Total RNA is quantified using RIBOGREEN RNA quantification reagent (Invetrogen, Inc. Eugene, OR). Methods of RNA quantification by RIBOGREEN are taught in Jones, L.J., et al, (Analytical Biochemistry, 1998, 265, 368-374). A CYTOFLUOR 4000 instrument (PE Applied Biosystems) is used to measure RIBOGREEN fluorescence.

Probes and primers are designed to hybridize to a MECP2 nucleic acid. Methods for designing real-time PCR probes and primers are well known in the art, and may include the use of software such as PRIMER EXPRESS Software (Applied Biosystems, Foster City, CA).

### Analysis of Protein Levels

Antisense inhibition of MECP2 nucleic acids can be assessed by measuring MECP2 protein levels. Protein levels of MECP2 can be evaluated or quantitated in a variety of ways well known in the art, such as immunoprecipitation, Western blot analysis (immunoblotting), enzyme-linked immunosorbent assay (ELISA), quantitative protein assays, protein activity assays (for example, caspase activity assays), immunohistochemistry, immunocytochemistry or fluorescence-activated cell sorting (FACS). Antibodies directed to a target can be identified and obtained from a variety of sources, such as the MSRS catalog of antibodies (Aerie Corporation, Birmingham, MI), or can be prepared via conventional monoclonal or polyclonal antibody generation methods well known in the art.

### In vivo testing of antisense compounds

Antisense compounds, for example, antisense oligonucleotides, are tested in animals to assess their ability to inhibit expression of MECP2 and produce phenotypic changes, such as, improved behavior, motor function, and cognition. In certain embodiments, motor function is measured by walking initiation analysis, rotarod, grip strength, pole climb, open field performance, balance beam, hindpaw footprint testing in the animal. In certain embodiments, behavior is measured by elevated plus maze and three-chamber social interaction. Testing may be performed in normal animals, or in experimental models. For administration to animals, antisense oligonucleotides are formulated in a pharmaceutically acceptable diluent, such as phosphate-buffered saline. Administration includes parenteral routes of administration, such as intraperitoneal, intravenous, and subcutaneous. Calculation of antisense oligonucleotide dosage and dosing frequency is within the abilities of those skilled in the art, and depends upon factors such as route of administration and animal body weight. Following a period of treatment with antisense oligonucleotides, RNA is isolated from CNS tissue or CSF and changes in MECP2 nucleic acid expression are measured.

### Certain Indications

In certain embodiments, provided herein are methods of treating an individual comprising administering one or more pharmaceutical compositions described herein. In certain embodiments, the individual has a neurological disorder. In certain embodiments, the individual is at risk for developing a neurological disorder, including, but not limited to, MECP2 duplication syndrome. In certain embodiments, the individual has been identified as having a MECP2 associated disorder. In certain embodiments, provided herein are methods for prophylactically reducing MECP2 expression in an individual. Certain embodiments include treating an individual in need thereof by administering to an individual a therapeutically effective amount of an antisense compound targeted to a MECP2 nucleic acid.

In one embodiment, administration of a therapeutically effective amount of an antisense compound targeted to a MECP2 nucleic acid is accompanied by monitoring of MECP2 levels in an individual, to determine an individual's response to administration of the antisense compound. An individual's response to administration of the antisense compound may be used by a physician to determine the amount and duration of therapeutic intervention.

In certain embodiments, administration of an antisense compound targeted to a MECP2 nucleic acid results in reduction of MECP2 mRNA and or protein expression by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in improved motor function in an animal. In certain embodiments, administration of a MECP2 antisense compound improves motor function by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in improved anxiety in an animal. In certain embodiments, administration of a MECP2 antisense compound improves anxiety by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in improved social interaction in an animal. In certain embodiments, administration of a MECP2 antisense compound improves social interaction by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in improved activity in an animal. In certain embodiments, administration of a MECP2 antisense compound improves activity by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in reduction of seizures. In certain embodiments, administration of a MECP2 antisense compound reduces seizures by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in normalized EEG discharges.

In certain embodiments, pharmaceutical compositions comprising an antisense compound targeted to MECP2 are used for the preparation of a medicament for treating a patient suffering or susceptible to a neurological disorder including MECP2 duplication syndrome.

### EXAMPLES

### Non-limiting disclosure and incorporation by reference

While certain methods described herein have been described with specificity in accordance with certain embodiments, the following examples serve only to illustrate the compounds described herein and are not intended to limit the same. Each of the references recited in the present application is incorporated herein by reference in its entirety.

### Example 1: Effect of antisense oligonucleotides targeting MECP2 in vivo

Antisense oligonucleotides (ASOs) that target human Methyl CpG Binding Protein 2 (MECP2), the complement of GENBANK accession number NT_167198.1 truncated from 4203000 to 4283000, SEQ ID NO: 2, were synthesized using standard solid phase oligonucleotide synthetic methods. They are chimeric oligonucleotides ("gapmers"), composed of a central "gap" region consisting of 2'-deoxynucleotides, which is flanked on both sides (5' and 3') by "wings" that are composed of modified nucleotides. The internucleoside (backbone) linkages are phosphorothioate or phosphodiester throughout the oligonucleotides. The sequences and structures of the antisense oligonucleotides and their start and stop sites along SEQ ID NO: 2 are shown in the table below.

**Table 1: Antisense oligonucleotides targeted to human MECP2**

| Isis No. | Sequence (5' to 3') | Start site | Stop site | SEQ ID NO. |
|---|---|---|---|---|
| 628724 | A_es A_eo ^mC_eo T_eo ^mC_eo T_ds ^mC_ds T_ds ^mC_ds G_ds G_ds T_ds ^mC_ds A_ds ^mC_ds G_eo G_eo G_es ^mC_es G_e | 69220 | 69239 | 12 |
| 628749 | mCes Aeo mCeo Aeo mCeo Tds Gds Ads mCds mCds Tds Tds Tds mCds Ads Geo Geo Ges mCes Te | 7615 | 7634 | 13 |
| 628772 | Gₑₛ Aₑₒ Tₑₒ ^{m}Cₑₒ Aₑₒ ^{m}C_{ds} T_{ds} G_{ds} G_{ds} A_{ds} A_{ds} ^{m}C_{ds} A_{ds} ^{m}C_{ds} A_{ds} Aₑₒ Tₑₒ Gₑₛ Gₑₛ Tₑ | 22839 | 22858 | 14 |
| 628775 | mCes Geo Teo Geo mCeo mCds Ads Tds Gds Gds Ads Ads Gds Tds mCds mCeo Teo Tes mCes mCe | 24936 | 24955 | 15 |
| 628785 | Gₑₛ Gₑₒ Tₑₒ Tₑₒ Tₑₒ T_{ds} T_{ds} ^{m}C_{ds} T_{ds} ^{m}C_{ds} ^{m}C_{ds} T_{ds} T_{ds} T_{ds} A_{ds} Tₑₒ Tₑₒ Aₑₛ Tₑₛ ^{m}Cₑ | 30744 | 30763 | 16 |

Superscript "m" indicates 5-methylcytosine. Subscripts: "o" indicates a phosphodiester internucleoside linkage, "s" indicates a phosphorothioate internucleoside linkage, "e" indicates a 2'-methoxyethyl modified nucleoside, and "d" indicates a 2'-deoxynucleoside.

The antisense oligonucleotides were analyzed for their effects on MECP2 mRNA and protein levels in transgenic MECP2 duplication mice that overexpress wild type human MECP2 (F1 hybrid MECP2-TG1 mice(FVB/N x 129)(Samaco et al., Nat Genet, 2012). At 8 weeks of age, FVB/N x 129 mice display hypoactivity in the open field test, increased anxiety in the open field and elevated plus maze tests, abnormal social behavior in the 3-chamber test, and increased motor coordination in the rotarod test. Seven week old *MECP2-TG* mice were given stereotactic intracerebral injection of 500 µg of an antisense oligonucleotide listed in Table 1 or saline into the right ventricle of the brain. Wild type mice were given stereotactic intracerebral injection of saline into the right ventricle of the brain as a control. Each group consisted of two or three mice. Two weeks following the injection, the mice were sacrificed, and cortical brain samples were collected for analysis of MECP2 mRNA and protein levels. MECP2 and GAPDH protein levels were analyzed by western blot performed on the cortical sample lysates. Rabbit antiserum raised against the N-terminus of MECP2 and mouse anti-GAPDH 6C5 (Advanced Immunochemicals, Long Beach, CA) were used as the primary antibodies. Western blot images were quanitifed using Image J software, and the MECP2 protein levels normalized to GAPDH levels are shown in Table 2 below.

Total MECP2 mRNA, human MECP2 mRNA (both the e1 and e2 isoforms), and mouse MECP2 mRNA (both the e1 and e2 isoforms) were separeately analyzed by RT-qPCR. The primers common to human and mouse used for total MECP2 mRNA were: 5'-TATTTGATCAATCCCCAGGG-3', SEQ ID NO: 3 , and 5'-CTCCCTCTCCCAGTTACCGT-3', SEQ ID NO: 4. The human specific primers used for MECP2-e1 were 5'-AGGAGAGACTGGAAGAAAAGTC-3', SEQ ID NO: 5, and 5'-CTTGAGGGGTTTGTCCTTGA-3', SEQ ID NO: 6. The human specific primers used for MECP2-e2 were 5'-CTCACCAGTTCCTGCTTTGATGT-3', SEQ ID NO: 7, and 5'-CTTGAGGGGTTTGTCCTTGA-3', SEQ ID NO: 6. The mouse specific primers used for MECP2-e1 were 5'-AGGAGAGACTGGAGGAAAAGTC-3', SEQ ID NO: 8, and 5'-CTTAAACTTCAGTGGCTTGTCTCTG-3', SEQ ID NO: 9. The mouse specific primers used for MECP2-e2 were 5'-CTCACCAGTTCCTGCTTTGATGT-3', SEQ ID NO: 7, and 5'-CTTAAACTTCAGTGGCTTGTCTCTG-3', SEQ ID NO: 9. MECP2 mRNA levels were normalized to Hprt mRNA levels, which were analyzed using primer 5'-CGGGGGACATAAAAGTTATTG-3', SEQ ID NO: 10, and 5'-TGCATTGTTTTACCAGTGTCAA-3', SEQ ID NO: 11. Results are presented in Table 2 below as average normlized MECP2 mRNA levels relative to saline treated wild type (WT) mice. The results show that all of the antisense oligonucleotides tested inhibited MECP2 mRNA and protein levels in the transgenic mice, and human MECP2 mRNA levels were specifically inhibited, whereas mouse MECP2 mRNA levels were not inhibited. Isis Number 628785 was the most potent in the first experiments and was carried forward. Entries listed as "n/a" indicate that the corresponding experiment was not performed.

**Table 2: MECP2 mRNA and protein levels in transgenic mice following ASO administration**

| Mouse/Isis No. | MECP2 protein level | Total MECP2 mRNA | Human mRNA | | Mouse mRNA | | SEQ ID NO. |
|---|---|---|---|---|---|---|---|
| | | | MECP2-e1 isoform | MECP2-e2 isoform | MECP2-e1 isoform | MECP2-e2 isoform | |
| WT/PBS | 1.0 | 1.0 | 0.0 | 0.0 | 1.0 | 1.3 | |
| TG/PBS | 2.0 | 3.3 | 0.9 | 8.3 | 1.2 | 1.4 | |
| TG/628724 | 1.5 | 2.0 | n/a | n/a | n/a | n/a | 12 |
| TG/628749 | 1.6 | 2.5 | n/a | n/a | n/a | n/a | 13 |
| TG/628772 | 1.7 | 2.7 | n/a | n/a | n/a | n/a | 14 |
| TG/628775 | 1.4 | 2.1 | n/a | n/a | n/a | n/a | 15 |
| TG/628785 | 1.3 | 1.6 | 0.3 | 2.3 | 1.0 | 1.3 | 16 |

### Example 2: Effect of gradual infusion of antisense oligonucleotide targeting MECP2 in vivo

In order to gradually infuse antisense oligonucleotide into the right ventricle of the brain, micro-osmotic pumps (Alzet model 1004, Durect, Cupertino, CA) were filled with 500µg of Isis No. 628785 or a control oligonucleotide that is not targeted to MECP2, dissolved in 100µl saline. The pump was then connected through a plastic catheter to a cannula (Alzet Brain Infusion Kit 3, Durect, Cupertino, CA). The pump was designed to deliver the drug at a rate of 0.11µl per hour for 28 days. The cannula and pump assembly was primed in sterile saline for two days at 37°C. Mice were anesthetized with isoflurane and placed on a computer-guided stereotaxic instrument (Angle Two Stereotaxic Instrument, Leica Microsystems, Bannockburn, IL). Anesthesia (isoflurane 3%) was continuously delivered via a small face mask. Ketoprofen 5mg/kg was administered subcutaneously at the initiation of the surgery. After sterilizing the surgical site with betadine and 70% alcohol, a midline incision was made over the skull and a subcutaneous pocket was generated on the back of the animal. Next, the pump was inserted into the pocket and the cannula was stereotactically implanted to deliver the drug in the right ventricle using the following coordinates: AP = -0.2 mm, ML = 1 mm, DV = -3 mm. The incision was sutured shut. Carprofen-containing food pellets were provided for 5 days after the surgery. 28 days after the initiation of the treatment the pump was disconnected from the cannula and removed. Two additional weeks were given to the animals to recover.

Isis No. 628785 was gradually infused into the right ventricles of the brains of 7-week old WT or TG mice using the micro-osmotic pumps. Each treatment group consisted of 4 or 5 animals. At the end of the four-week treatment period, western blot was performed as described in Example 1 to analyze MECP2 protein levels at 4, 8, and 12 weeks following the initiation of antisense oligonucleotide treatment. The results are shown in Table 3 below.

**Table 3: MECP2 protein levels following antisense oligonucleotide infusion**

| Mouse/Isis No. | MECP2 protein level (relative to WT/Control) | | |
|---|---|---|---|
| | 4 weeks | 8 weeks | 12 weeks |
| WT/Control | 1.0 | 1.0 | 1.0 |
| TG/Control | 2.9 | 2.7 | 2.3 |
| TG/628785 | 1.6 | 1.8 | 2.2 |

### Example 3: Behavioral effects of antisense oligonucleotide targeting human MECP2 in vivo

Following infusion of antisense oligonucleotide as described in Example 2, a battery of behavioral assays were performed to assess phenotypic effects of oligonucleotide treatment in TG mice treated with Isis No. 628785 or a control oligonucleotide and WT mice treated with a control oligonucleotide. Each treatment group contained at least 15 animals.

An open field test was performed two weeks and six weeks after the completion of the 4 week infusion by placing mice into the center of an open arena after habituation in the test room (40 x 40 x 30 cm). Their behavior was tracked by laser photobeam breaks for 30 min. Horizontal locomotor activity, rearing activity, time spent in the center of the arena, and entries to the center were analyzed using AccuScan Fusion software (Omnitech, Columbus, OH). The results are reported in table 4 below. The results show that the TG mice displayed hypoactivity in the open field test relative to WT mice at both time points, and treatment of TG mice with Isis No. 628785 restored activity close to WT levels.

**Table 4: Open field test**

| Mouse/Isis No. | Horizontal activity (activity counts) | | Rearing episodes | | Time in center (s) | | Entries to center | |
|---|---|---|---|---|---|---|---|---|
| | 2 weeks | 6 weeks | 2 weeks | 6 weeks | 2 weeks | 6 weeks | 2 weeks | 6 weeks |
| WT/Control | 7134 | 5632 | 277 | 236 | 179 | n/a | 147 | 103 |
| TG/Control | 4116 | 3493 | 156 | 106 | 105 | n/a | 65 | 45 |
| TG/628785 | 5550 | 6114 | 170 | 205 | 93 | n/a | 75 | 99 |

Mice were tested in an elevated plus maze two weeks and six weeks after the completion of the 4 week infusion. After habituation in the test room, mice were placed in the center part of the maze facing one of the two open arms. Mouse behavior was video-tracked for 10 minutes, and the time the mice spent in the open arms and the entries to the open arms were recorded and analyzed using ANY-maze system (Stoelting, Wood Dale, IL). The results are shown in Table 5 below. The results show that the TG mice displayed increased anxiety in the elevated plus maze test relative to WT mice at both time points, and treatment of TG mice with Isis No. 628785 restored anxiety levels close to WT levels.

**Table 5: Elevated plus maze**

| Mouse/Isis No. | Time in open arms (s) | | Entries into open arms | |
|---|---|---|---|---|
| | 2 weeks | 6 weeks | 2 weeks | 6 weeks |
| WT/Control | 139 | 81 | 21 | 12 |
| TG/Control | 76 | 13 | 12 | 2 |
| TG/628785 | 91 | 55 | 11 | 7 |

Mice were assessed in a three-chamber social interaction test three weeks and seven weeks after the completion of the 4 week infusion. The apparatus comprised a clear Plexiglas box with removable partitions that separated the box into three chambers: left, central, and right. In the left and right chambers a cylindrical wire cup was placed with the open side down. Age and gender-matched mice were used as novel partners. Two days before the test, the novel partner mice were habituated to the wire cups (3 inches diameter by 4 inches in height) for 1 hour per day. After habituation in the test room, each mouse was placed in the central chamber and allowed to explore the three chambers for 10 minutes (habituation phase). The time spent in each chamber during the habituation phase was recorded automatically and analyzed using ANY-maze system (Stoelting, Wood Dale, IL). Next, a novel partner mouse was placed under a wire cup in either the left or the right chamber. An inanimate object was placed as a control under the wire cup of the opposite chamber. The location of the novel mouse was randomized between the left and right chambers for each test mouse to control for side preference. The mouse tested was allowed to explore again for an additional 10 minutes. The time spent investigating the novel partner (defined by rearing, sniffing or pawing at the wire cup) and the time spent investigating the inanimate object were measured manually. The results are shown in Table 6 below. The results show that the TG mice displayed hypoactivity and decreased social interaction in the three-chamber social interaction test relative to WT mice at both time points, and treatment of TG mice with Isis No. 628785 restored social interaction with a novel partner to WT levels at the 6 week time point.

**Table 6: Three-chamber social interaction test**

| Mouse/Isis No. | Time spent investigating chambers during habituation phase (s) | | | | Time spent investigating novel partner or inanimate object (s) | | | |
|---|---|---|---|---|---|---|---|---|
| | Left | | Right | | Novel partner | | Inanimate object | |
| | 2 weeks | 6 weeks | 2 weeks | 6 weeks | 2 weeks | 6 weeks | 2 weeks | 6 weeks |
| WT/Control | 52.4 | n/a | 44.1 | n/a | 141 | 107 | 38 | 37 |
| TG/Control | 35.5 | n/a | 29.9 | n/a | 106 | 59 | 33 | 28 |
| TG/628785 | 37.7 | n/a | 23.0 | n/a | 94 | 106 | 27 | 28 |

Mice were assessed in an accelerating rotarod test three weeks after the completion of the 4 week infusion. After habituation in the test room, motor coordination was measured using an accelerating rotarod apparatus (Ugo Basile, Varese, Italy). Mice were tested 2 consecutive days, 4 trials each, with an interval of 60 minutes between trials to rest. Each trial lasted for a maximum of 10 minutes; mice that never fell were given a measurement of 600 seconds. The rod accelerated from 4 to 40 r.p.m. in the first 5 minutes. The time that it took for each mouse to fall from the rod (latency to fall) was recorded. Results are shown in Table 7 below. The results show that the TG mice displayed increased performance in the rotarod test relative to WT mice, and treatment of TG mice with Isis No. 628785 restored performance to WT levels.

**Table 7: Accelerating rotarod test**

| Mouse/Isis No. | Latency to fall (s) | |
|---|---|---|
| | Day 1 | Day 2 |
| WT/Control | 174 | 300 |
| TG/Control | 275 | 400 |
| TG/628785 | 183 | 282 |

The results in tables 4-7 above show that treatment with Isis No. 628785 targeting MECP2 reversed behavioral phenotypes of the TG mice. The TG mice treated with Isis No. 628785 performed similarly to WT mice in the rotarod test 3-4 weeks after completion of the infusion. By 6-7 weeks after completion of the infusion, the hypoactivity, anxiety-like behaviors and social behavior of the TG mice were reversed, as evidenced by the open field, elevated plus maze and three-chamber tests, respectively.

### Example 4: Dose response of antisense oligonucleotide targeting human MECP2 in patient cells

In order to test for a dose dependent effect of Isis No. 628785 on human cells, B-lymphoblast cells from two individuals affected with MECP2-duplication symdrome and age-matched control cells were cultured in suspension in RPMI 1640 medium with L-glutamine, penicillin-streptomycin, and 10% (v/v) fetal bovice serum. A day before transfection, cells were seeded in triplicate for each treatment in 6-well plates at 10⁶ cells per well in a total volume of 2 mL medium. Cells were transfected with Isis No. 628785 or control oligonucleotide at a concentration listed in Table 8 below with TurboFect transfection reagent (Thermo Scientific, Carlsbad, CA). Cells were harvested and RNA was extracted 48 hours after transfection, and MECP2 mRNA levels were analyzed as described in Example 1. Results are presented in Table 8 below as average normlized MECP2 mRNA levels for both patients' cells relative to untreated control cells. The results show that Isis No. 628785 inhibited MECP2 expression in human MECP2 duplication patient cells.

**Table 8: Antisense oligonucleotide treatment of patient lymphoblasts**

| Cell type/Isis No. | Concentration (nM) | Total relative MECP2 mRNA |
|---|---|---|
| Control/Control | 600 | 1.0 |
| Patient/Control | 600 | 3.1 |
| Patient/628785 | 150 | 2.2 |
| Patient/628785 | 300 | 1.6 |
| Patient/628785 | 600 | 1.3 |

### Example 5: Reduction of seizure activity with an antisense oligonucleotide targeting human MECP2 in vivo

Without treatment, seizures and accompanying abnormal electrographic discharges occur in MECP2-TG1 mice as they age. In order to test the effect of antisense oligonucleotide treatment on seizure activity in MECP2-TG1 mice, electrocephalography recordings were performed and behavioral seizure activity was observed.

25-35 week old MECP2-TG1 mice that had been treated as described in Example 2 were anaesthetized with isoflurane and mounted in a stereotaxic frame for the surgical implantation of three recording electrodes (Teflon-coated silver wire, 125 µm in diameter) in the subdural space of the left frontal cortex, the left parietal cortex, and the right parietal cortex, with a reference electrode placed in the occipital region of the skull. After 3-5 days of surgical recovery, cortical EEG activity and behavior were recorded for 2h per day over 3-5 days. Strong electrographic seizure events were typically accompanied by behavioral seizures. Figure 1 displays EEG traces for WT mice, MECP2-TG1 mice without Isis No. 628785 treatment, and MECP2-TG1 mice that received treatment with Isis No. 628785. Treatment of MECP2-TG1 mice with Isis No. 628785 eliminated both behavioral seizures and abnormal EEG discharges.

The invention also provides the following numbered embodiments:
1. A method comprising administering an antisense compound complementary to MECP2 to an animal for treating a MECP2 associated disorder.
2. A method comprising:
   identifying an animal having a MECP2 associated disorder; and
   administering a MECP2 antisense compound.
3. The method of any preceding embodiment, wherein MECP2 associated disorder is a neurological disorder.
4. The method of any preceding embodiment, wherein the MECP2 associated disorder is MECP2 duplication syndrome.
5. The method of any preceding embodiment, wherein the animal is a human.
6. The method of any preceding embodiment, wherein the administering is parenteral administration.
7. The method of embodiment 6, wherein the parenteral administration is any of intracerebroventricular administration or intrathecal administration.
8. The method of any preceding embodiment, wherein the administering reduces MECP2 mRNA and or protein levels.
9. The method of embodiment 8, wherein the administering reduces MECP2 mRNA and or protein levels by 15, 20, 25, 30, 35, 40, 45, 50, 55, 60, or 65 percent.
10. The method of any preceding embodiment, wherein the administering improves motor function.
11. The method of embodiment 10, wherein motor function is improved by 10, 15, 20, 25, 30, or 35 percent.
12. The method of any preceding embodiment, wherein the administering improves anxiety.
13. The method of embodiment 12, wherein the administering improves anxiety by 5, 10, 15, 20, 25, 30, 35, 40, 45, or 50 percent.
14. The method of any preceding embodiment, wherein the administering improves social interaction.
15. The method of embodiment 14, wherein the administering improves social interaction by 5, 10, 15, 20, 25, 30, 35, 40, 45, or 50 percent.
16. The method of any preceding embodiment, wherein the administering improves activity.
17. The method of embodiment 16, wherein the administering improves activity by 5, 10, 15, 20, 25, 30, 35, 40, 45, 50, 55, 60, 65, 70, 75, or 80 percent.
18. The method of any preceding embodiment, wherein the administering reduces seizures.
19. The method of any preceding embodiment, wherein the administering normalizes EEG discharges.
20. The method of any preceding embodiment, wherein at least one symptom of a MECP2 associated disorder is ameliorated, treated, prevented, or slowed.
21. The method of any preceding embodiment, wherein the antisense compound is a modified antisense oligonucleotide.
22. The method of embodiment 21, wherein the modified antisense oligonucleotide has the nucleobase sequence of SEQ ID NO: 12-16.

## Claims

1. An antisense compound complementary to MECP2 for use in treating a MECP2 associated disorder.

2. A MECP2 antisense compound for use in treating a MECP2 associated disorder in an animal identified as having a MECP2 associated disorder.

3. The antisense compound for use according to any preceding claim, wherein the MECP2 associated disorder is a neurological disorder.

4. The antisense compound for use according to any preceding claim, wherein the MECP2 associated disorder is MECP2 duplication syndrome.

5. The antisense compound for use according to any preceding claim, wherein the animal is a human.

6. The antisense compound for use according to any preceding claim, wherein the administering is parenteral administration, wherein optionally the parenteral administration is any of intracerebroventricular administration or intrathecal administration.

7. The antisense compound for use according to any preceding claim, wherein the administering reduces MECP2 mRNA and or protein levels, wherein optionally the administering reduces MECP2 mRNA and or protein levels by 15, 20, 25, 30, 35, 40, 45, 50, 55, 60, or 65 percent.

8. The antisense compound for use according to any preceding claim, wherein the administering improves motor function, wherein optionally motor function is improved by 10, 15, 20, 25, 30, or 35 percent.

9. The antisense compound for use according to any preceding claim, wherein the administering improves anxiety, wherein optionally the administering improves anxiety by 5, 10, 15, 20, 25, 30, 35, 40, 45, or 50 percent.

10. The antisense compound for use according to any preceding claim, wherein the administering improves social interaction, wherein optionally the administering improves social interaction by 5, 10, 15, 20, 25, 30, 35, 40, 45, or 50 percent.

11. The antisense compound for use according to any preceding claim, wherein the administering improves activity, wherein optionally the administering improves activity by 5, 10, 15, 20, 25, 30, 35, 40, 45, 50, 55, 60, 65, 70, 75, or 80 percent.

12. The antisense compound for use according to any preceding claim, wherein the administering reduces seizures.

13. The antisense compound for use according to any preceding claim, wherein the administering normalizes EEG discharges.

14. The antisense compound for use according to any preceding claim, wherein at least one symptom of a MECP2 associated disorder is ameliorated, treated, prevented, or slowed.

15. The antisense compound for use according to any preceding claim, wherein the antisense compound is a modified antisense oligonucleotide, wherein optionally the modified antisense oligonucleotide has the nucleobase sequence of SEQ ID NO: 12-16.
